# EUROPEAN PATENT APPLICATION

(11) **EP 4 595 753 A1**
(43) Date of publication of application: **06.08.2025**
(21) Application number: 23872614.5
(22) Date of filing: 29.09.2023
(51) Int. Cl.: A01N 43/56, A01C 1/08, A01N 43/78, A01N 43/832, A01P 3/00, C07D 401/12

(54) **PHENYLPYRAZOLE COMPOUND, AND PLANT DISEASE CONTROL METHOD**

(30) Priority: 30.09.2022 JP 2022158301
(71) Applicant: Sumitomo Chemical Company, Limited, Tokyo 103-6020 (JP)
(72) Inventor: NOKURA, Yoshihiko, Takarazuka-shi, Hyogo 665-8555 (JP); MAEHATA, Nao, Takarazuka-shi, Hyogo 665-8555 (JP); ARAI, Keisuke, Takarazuka-shi, Hyogo 665-8555 (JP)
(74) Representative: Vossius & Partner Patentanwälte Rechtsanwälte mbB
(86) International application number: PCT/JP2023/035684
(87) International publication number: WO 2024/071395

(57) **Abstract**

The present invention provides an excellent control method against plant diseases. A compound represented by formula (I) 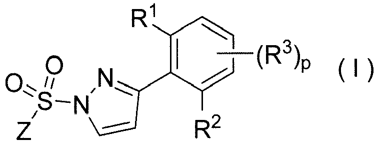 [wherein:
Z represents a C6-C10 aryl group or the like;
R¹ and R² are identical to or different from each other, and each represent a C1-C6 chain hydrocarbon group or the like;
R³ represents a C1-C6 chain hydrocarbon group or the like;
adjacent R¹ and R³ may be optionally combined with the carbon atoms to which they are attached to form a C4-C7 alicyclic hydrocarbon or the like;
adjacent two R³ and R³ may be optionally combined with the carbon atoms to which they are attached to form a C4-C7 alicyclic hydrocarbon or the like;
p represents 0, 1, 2, or 3; and
when p represents 2 or 3, then two or three R³ are identical to or different from each other]
or an N-oxide thereof, or a salt thereof can be used for controlling plant diseases.

## Description

### TECHNICAL FIELD

This application claims the priority to and the benefit of Japanese Patent Application No. 2022-158301 filed on September 30, 2022, the entire contents of which are incorporated herein by reference.

The present invention relates to phenylpyrazole compounds and methods for controlling plant diseases.

### BACKGROUND ART

PATENT DOCUMENTs 1 and 2 disclose phenylpyrazole compounds.

### CITATION LIST

### PATENT DOCUMENT

PATENT DOCUMENT 1: WO 96/02138 pamphlet
PATENT DOCUMENT 2: EP 538156 A1

### SUMMARY OF THE INVENTION

### PROBLEMS TO BE SOLVED BY INVENTION

An object of the present invention is to provide excellent control methods against plant diseases.

### MEANS TO SOLVE PROBLEMS

The present inventors have studied to find out an excellent control method against plant diseases. As a result, they have found that a compound represented by the following formula (I) has excellent control efficacy against plant diseases.

Namely, the present invention provides the followings.

[1] A method for controlling a plant disease which comprises applying a compound represented by formula (I) 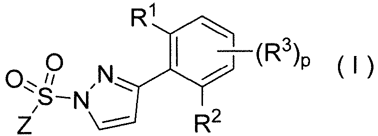 [wherein:
   Z represents a C6-C10 aryl group or a 5-10 membered aromatic heterocyclic group {wherein said C6-C10 aryl group and said 5-10 membered aromatic heterocyclic group may be optionally substituted with one or more substituent(s) selected from Group D};
   R¹ and R² are identical to or different from each other, and each represent a C1-C6 chain hydrocarbon group optionally substituted with one or more substituent(s) selected from Group A, a C3-C8 alicyclic hydrocarbon group, a 3-8 membered nonaromatic heterocyclic group, a C6-C10 aryl group, a 5-10 membered aromatic heterocyclic group {wherein said C3-C8 alicyclic hydrocarbon group, said 3-8 membered nonaromatic heterocyclic group, said C6-C10 aryl group, and said 5-10 membered aromatic heterocyclic group may be optionally substituted with one or more substituent(s) selected from Group C}, a hydrogen atom, a halogen atom, a nitro group, a cyano group, NR⁸R⁹, OR¹⁰, S(O)ₖR¹¹, C(O)R¹², C(O)OR¹³, C(O)NR⁴R⁵, or CR⁶=N-O-R⁷;
   R³ represents a C1-C6 chain hydrocarbon group optionally substituted with one or more substituent(s) selected from Group A, a C3-C8 alicyclic hydrocarbon group, a 3-8 membered nonaromatic heterocyclic group, a C6-C10 aryl group, a 5-10 membered aromatic heterocyclic group {wherein said C3-C8 alicyclic hydrocarbon group, said 3-8 membered nonaromatic heterocyclic group, said C6-C10 aryl group, and said 5-10 membered aromatic heterocyclic group may be optionally substituted with one or more substituent(s) selected from Group C}, a halogen atom, a nitro group, a cyano group, NR⁸R⁹, OR¹⁰, S(O)ₖR¹¹, C(O)R¹², C(O)OR¹³, C(O)NR⁴R⁵, or CR⁶=N-O-R⁷;
   adjacent R¹ and R³ may be optionally combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B}, a benzene ring, or a 5-7 membered aromatic heterocyclic ring {wherein said benzene ring and said 5-7 membered aromatic heterocyclic ring may be optionally substituted with one or more substituent(s) selected from Group E};
   adjacent two R³ and R³ may be optionally combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B}, a benzene ring, or a 5-7 membered aromatic heterocyclic ring {wherein said benzene ring and said 5-7 membered aromatic heterocyclic ring may be optionally substituted with one or more substituent(s) selected from Group E};
   R⁴, R⁵, R⁶, R⁷, R⁸, R⁹, R¹⁰, R¹¹, R¹², and R¹³ are identical to or different from each other, and each represent a C1-C6 chain hydrocarbon group optionally substituted with one or more substituent(s) selected from Group A, a C3-C8 alicyclic hydrocarbon group, a 3-8 membered nonaromatic heterocyclic group, a C6-C10 aryl group, a 5-10 membered aromatic heterocyclic group {wherein said C3-C8 alicyclic hydrocarbon group, said 3-8 membered nonaromatic heterocyclic group, said C6-C10 aryl group, and said 5-10 membered aromatic heterocyclic group may be optionally substituted with one or more substituent(s) selected from Group C}, or a hydrogen atom;
   p represents 0, 1, 2, or 3;
   when p represents 2 or 3, then two or three R³ are identical to or different from each other; and
   k represents 0, 1, or 2;
   Group A: a group consisting of a C1-C6 alkoxy group, a C1-C6 alkylsulfanyl group, a C1-C6 alkylsulfinyl group, a C1-C6 alkylsulfonyl group {wherein said C1-C6 alkoxy group, said C1-C6 alkylsulfanyl group, said C1-C6 alkylsulfinyl group, and said C1-C6 alkylsulfonyl group may be optionally substituted with one or more halogen atom(s)}, a C3-C8 alicyclic hydrocarbon group, a 3-8 membered nonaromatic heterocyclic group, a C6-C10 aryl group, a 5-10 membered aromatic heterocyclic group {wherein said C3-C8 alicyclic hydrocarbon group, said C6-C10 aryl group, and said 5-10 membered aromatic heterocyclic group may be optionally substituted with one or more substituent(s) selected from Group C}, a halogen atom, and a cyano group;
   Group B: a group consisting of an oxo group, a thioxo group, a C1-C6 chain hydrocarbon group, a C3-C8 alicyclic hydrocarbon group, a C1-C6 alkoxy group {wherein said C1-C6 chain hydrocarbon group, said C3-C8 alicyclic hydrocarbon group, and said C1-C6 alkoxy group may be optionally substituted with one or more halogen atom(s)}, a halogen atom, a nitro group, and a cyano group;
   Group C: a group consisting of a C1-C6 chain hydrocarbon group, a C3-C8 alicyclic hydrocarbon group, a C1-C6 alkoxy group, a C1-C6 alkylsulfanyl group, a C1-C6 alkylsulfinyl group, a C1-C6 alkylsulfonyl group {wherein said C1-C6 chain hydrocarbon group, said C3-C8 alicyclic hydrocarbon group, said C1-C6 alkoxy group, said C1-C6 alkylsulfanyl group, said C1-C6 alkylsulfinyl group, and said C1-C6 alkylsulfonyl group may be optionally substituted with one or more halogen atom(s)}, a halogen atom, a nitro group, and a cyano group;
   Group D: a group consisting of a C1-C6 chain hydrocarbon group, a C3-C8 alicyclic hydrocarbon group, a C1-C6 alkoxy group {wherein said C1-C6 chain hydrocarbon group, said C3-C8 alicyclic hydrocarbon group, and said C1-C6 alkoxy group may be optionally substituted with one or more halogen atom(s)}, a halogen atom, a nitro group, and a cyano group;
   Group E: a group consisting of a C1-C6 chain hydrocarbon group, a C3-C8 alicyclic hydrocarbon group, a C1-C6 alkoxy group {wherein said C1-C6 chain hydrocarbon group, said C3-C8 alicyclic hydrocarbon group, and said C1-C6 alkoxy group may be optionally substituted with one or more halogen atom(s)}, a halogen atom, a nitro group, and a cyano group]
   (hereinafter referred to as "Present compound N") or an N-oxide thereof, or a salt thereof (hereinafter the compound represented by formula (I) or an N-oxide thereof, or a salt thereof is referred to as "Present compound"), to a plant or soil for cultivating a plant.
[2] The method for controlling a plant disease according to [1], wherein the compound represented by formula (I) or an N-oxide thereof, or a salt thereof in [1] is a compound or an N-oxide thereof, or a salt thereof wherein
   Z represents a phenyl group or a 5-6 membered aromatic heterocyclic group {wherein said phenyl group and said 5-6 membered aromatic heterocyclic group may be optionally substituted with one or more substituent(s) selected from Group D};
   R¹ and R² each represent a hydrogen atom;
   R³ represents a C1-C6 chain hydrocarbon group optionally substituted with one or more halogen atom(s), a C3-C8 alicyclic hydrocarbon group, a halogen atom, a nitro group, NR⁸R⁹, OR¹⁰, SR¹¹, C(O)OR¹³, or C(O)NR⁴R⁵;
   adjacent two R³ and R³ may be optionally combined with the carbon atoms to which they are attached to form a C4-C7 alicyclic hydrocarbon, a 5-7 membered nonaromatic heterocyclic ring, or a 5-7 membered aromatic heterocyclic ring {wherein said C4-C7 alicyclic hydrocarbon, said 5-7 membered nonaromatic heterocyclic ring, and said 5-7 membered aromatic heterocyclic ring may be optionally substituted with one or more halogen atom(s)}; and
   R⁴, R⁵, R⁸, R⁹, R¹⁰, R¹¹, and R¹³ are identical to or different from each other, and each represent a C1-C6 chain hydrocarbon group, a C3-C8 alicyclic hydrocarbon group, a phenyl group, or a hydrogen atom.
[3] The method for controlling a plant disease according to [1] or [2], wherein the compound represented by formula (I) or an N-oxide thereof, or a salt thereof in [1] or [2] is a compound or an N-oxide thereof, or a salt thereof wherein
   Z represents a phenyl group or a 5-6 membered aromatic heterocyclic group {wherein said phenyl group and said 5-6 membered aromatic heterocyclic group may be optionally substituted with one or more substituent(s) selected from Group F}; and
   R¹ and R² each represent a hydrogen atom;
   Group F: a group consisting of a C1-C6 chain hydrocarbon group and a halogen atom.
[4] A compound represented by formula (II) 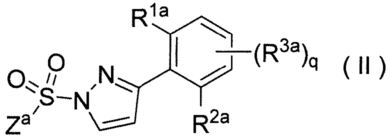 [wherein:
   Z^{a} represents a 5-10 membered aromatic heterocyclic group optionally substituted with one or more substituent(s) selected from Group D^{a};
   R^{1a} and R^{2a} are identical to or different from each other, and each represent a C1-C6 chain hydrocarbon group optionally substituted with one or more substituent(s) selected from Group A^{a}, a C3-C8 alicyclic hydrocarbon group, a 3-8 membered nonaromatic heterocyclic group, a C6-C10 aryl group, a 5-10 membered aromatic heterocyclic group {wherein said C3-C8 alicyclic hydrocarbon group, said 3-8 membered nonaromatic heterocyclic group, said C6-C10 aryl group, and said 5-10 membered aromatic heterocyclic group may be optionally substituted with one or more substituent(s) selected from Group C^{a}}, a hydrogen atom, a halogen atom, a nitro group, a cyano group, NR^{8a}R^{9a}, OR^{10a}, S(O)ₘR^{11a}, C(O)R^{12a}, C(O)OR^{13a}, C(O)NR^{4a}R^{5a}, or CR^{6a}=N-O-R^{7a};
   R^{3a} represents a C1-C6 chain hydrocarbon group optionally substituted with one or more substituent(s) selected from Group A^{a}, a C3-C8 alicyclic hydrocarbon group, a 3-8 membered nonaromatic heterocyclic group, a C6-C10 aryl group, a 5-10 membered aromatic heterocyclic group {wherein said C3-C8 alicyclic hydrocarbon group, said 3-8 membered nonaromatic heterocyclic group, said C6-C10 aryl group, and said 5-10 membered aromatic heterocyclic group may be optionally substituted with one or more substituent(s) selected from Group C^{a}}, a halogen atom, a nitro group, a cyano group, NR^{8a}R^{9a}, OR^{10a}, S(O)ₘR^{11a}, C(O)R^{12a}, C(O)OR^{13a}, C(O)NR^{4a}R^{5a}, or CR^{6a}=N-O-R^{7a};
   adjacent R^{1a} and R^{3a} may be optionally combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B^{a}}, a benzene ring, or a 5-7 membered aromatic heterocyclic ring {wherein said benzene ring and said 5-7 membered aromatic heterocyclic ring may be optionally substituted with one or more substituent(s) selected from Group E^{a}};
   adjacent two R^{3a} and R^{3a} may be optionally combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B^{a}}, a benzene ring, or a 5-7 membered aromatic heterocyclic ring {wherein said benzene ring and said 5-7 membered aromatic heterocyclic ring may be optionally substituted with one or more substituent(s) selected from Group E^{a}};
   R^{4a} , R^{5a}, R^{6a}, R^{7a}, R^{8a}, R^{9a}, R^{10a}, R^{11a}, R^{12a}, and R^{13a} are identical to or different from each other, and each represent a C1-C6 chain hydrocarbon group optionally substituted with one or more substituent(s) selected from Group A^{a}, a C3-C8 alicyclic hydrocarbon group, a 3-8 membered nonaromatic heterocyclic group, a C6-C10 aryl group, a 5-10 membered aromatic heterocyclic group {wherein said C3-C8 alicyclic hydrocarbon group, said 3-8 membered nonaromatic heterocyclic group, said C6-C10 aryl group, and said 5-10 membered aromatic heterocyclic group may be optionally substituted with one or more substituent(s) selected from Group C^{a}}, or a hydrogen atom;
   q represents 1, 2, or 3;
   when q represents 2 or 3, then two or three R^{3a} are identical to or different from each other; and
   m represents 0, 1, or 2;
   Group A^{a}: a group consisting of a C1-C6 alkoxy group, a C1-C6 alkylsulfanyl group, a C1-C6 alkylsulfinyl group, a C1-C6 alkylsulfonyl group {wherein said C1-C6 alkoxy group, said C1-C6 alkylsulfanyl group, said C1-C6 alkylsulfinyl group, and said C1-C6 alkylsulfonyl group may be optionally substituted with one or more halogen atom(s)}, a C3-C8 alicyclic hydrocarbon group, a 3-8 membered nonaromatic heterocyclic group, a C6-C10 aryl group, a 5-10 membered aromatic heterocyclic group {wherein said C3-C8 alicyclic hydrocarbon group, said 3-8 membered nonaromatic heterocyclic group, said C6-C10 aryl group, and said 5-10 membered aromatic heterocyclic group may be optionally substituted with one or more substituent(s) selected from Group C^{a}}, a halogen atom, and a cyano group;
   Group B^{a}: a group consisting of an oxo group, a thioxo group, a C1-C6 chain hydrocarbon group, a C3-C8 alicyclic hydrocarbon group, a C1-C6 alkoxy group {wherein said C1-C6 chain hydrocarbon group, said C3-C8 alicyclic hydrocarbon group, and said C1-C6 alkoxy group may be optionally substituted with one or more halogen atom(s)}, a halogen atom, a nitro group, and a cyano group;
   Group C^{a}: a group consisting of a C1-C6 chain hydrocarbon group, a C3-C8 alicyclic hydrocarbon group, a C1-C6 alkoxy group, a C1-C6 alkylsulfanyl group, a C1-C6 alkylsulfinyl group, a C1-C6 alkylsulfonyl group {wherein said C1-C6 chain hydrocarbon group, said C3-C8 alicyclic hydrocarbon group, said C1-C6 alkoxy group, said C1-C6 alkylsulfanyl group, said C1-C6 alkylsulfinyl group, and said C1-C6 alkylsulfonyl group may be optionally substituted with one or more halogen atom(s)}, a halogen atom, a nitro group, and a cyano group;
   Group D^{a}: a group consisting of a C1-C6 chain hydrocarbon group, a C3-C8 alicyclic hydrocarbon group, a C1-C6 alkoxy group {wherein said C1-C6 chain hydrocarbon group, said C3-C8 alicyclic hydrocarbon group, and said C1-C6 alkoxy group may be optionally substituted with one or more halogen atom(s)}, a halogen atom, a nitro group, and a cyano group;
   Group E^{a}: a group consisting of a C1-C6 chain hydrocarbon group, a C3-C8 alicyclic hydrocarbon group, a C1-C6 alkoxy group {wherein said C1-C6 chain hydrocarbon group, said C3-C8 alicyclic hydrocarbon group, and said C1-C6 alkoxy group may be optionally substituted with one or more halogen atom(s)}, a halogen atom, a nitro group, and a cyano group]

   (provided that 4-{[3-(4-fluorophenyl)-1H-pyrazol-1-yl]sulfonyl}-1,3-dimethyl-1H-pyrazole and 3-(2,3-dihydrobenzofuran-5-yl)-1-(thiophen-2-ylsulfonyl)-1H-pyrazole are excluded)
   (hereinafter referred to as "Compound N of the present invention") or an N-oxide thereof, or a salt thereof (hereinafter the compound represented by formula (II) or an N-oxide thereof, or a salt thereof is referred to as "Compound of the present invention").
[5] The compound or an N-oxide thereof, or a salt thereof according to [4], wherein
   Z^{a} represents a 5-6 membered aromatic heterocyclic group optionally substituted with one or more substituent(s) selected from Group F^{a}; and
   R^{1a} and R^{2a} each represent a hydrogen atom;
   Group F^{a}: a group consisting of a C1-C6 chain hydrocarbon group and a halogen atom.
[6] The compound or an N-oxide thereof, or a salt thereof according to [4] or [5], wherein
   R^{3a} represents a C1-C6 chain hydrocarbon group optionally substituted with one or more halogen atom(s), a C3-C8 alicyclic hydrocarbon group, a halogen atom, a nitro group, NR^{8a}R^{9a} , OR^{10a}, or SR^{11a};
   adjacent two R^{3a} and R^{3a} may be optionally combined with the carbon atoms to which they are attached to form a C4-C7 alicyclic hydrocarbon, a 5-7 membered nonaromatic heterocyclic ring, or a 5-7 membered aromatic heterocyclic ring {wherein said C4-C7 alicyclic hydrocarbon, said 5-7 membered nonaromatic heterocyclic ring, and said 5-7 membered aromatic heterocyclic ring may be optionally substituted with one or more halogen atom(s)}; and
   R^{8a}, R^{9a}, R^{10a}, and R^{11a} are identical to or different from each other, and each represent a C1-C6 chain hydrocarbon group.
[7] A composition comprising the compound or an N-oxide thereof, or a salt thereof according to any one of [4] to [6], and an inert carrier.
[8] A composition comprising one or more ingredient(s) selected from the group consisting of Group (a), Group (b), Group (c), and Group (d), and the compound or an N-oxide thereof, or a salt thereof according to any one of [4] to [6] :
   Group (a): a group consisting of insecticidal active ingredients, miticidal active ingredients, and nematicidal active ingredients;
   Group (b): fungicidal active ingredients;
   Group (c): plant growth regulatory ingredients;
   Group (d): repellent ingredients.
[9] A method for controlling a plant disease which comprises applying an effective amount of the compound or an N-oxide thereof, or a salt thereof according to any one of [4] to [6], or an effective amount of the composition according to [8], to a plant or soil for cultivating a plant.
[10] Use of the compound or an N-oxide thereof, or a salt thereof according to any one of [4] to [6], or the composition according to [8], for controlling a plant disease.
[11] A seed or a vegetative reproductive organ holding an effective amount of the compound or an N-oxide thereof, or a salt thereof according to any one of [4] to [6], or an effective amount of the composition according to [8].
[12] A compound represented by formula (III) 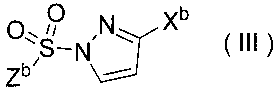 [wherein:
   Z^{b} represents a 5-10 membered aromatic heterocyclic group optionally substituted with one or more substituent(s) selected from Group D^{b}; and
   X^{b} represents a chlorine atom, a bromine atom, an iodine atom, B(OH)₂, or a 4,4,5,5-tetramethyl-1,3,2-dioxaborolan-2-yl group;
   Group D^{b}: a group consisting of a C1-C6 chain hydrocarbon group, a C3-C8 alicyclic hydrocarbon group, a C1-C6 alkoxy group {wherein said C1-C6 chain hydrocarbon group, said C3-C8 alicyclic hydrocarbon group, and said C1-C6 alkoxy group may be optionally substituted with one or more halogen atom(s)}, a halogen atom, a nitro group, and a cyano group]
   (hereinafter referred to as "Intermediate A").

### EFFECT OF INVENTION

According to the present invention, plant diseases can be controlled.

### MODE FOR CARRYING OUT THE INVENTION

The substituents in the present invention are explained as follows.

The term of "halogen atom" represents a fluorine atom, a chlorine atom, a bromine atom, or an iodine atom.

When a substituent is substituted with two or more halogen atoms or substituents, these halogen atoms or substituents may be identical to or different from each other.

Regarding the expression of "optionally substituted with one or more substituent(s) selected from Group X" (wherein X represents any one of A, B, C, D, E, F, A^{a}, B^{a}, C^{a}, D^{a}, E^{a}, F^{a}, D^{b}, F^{b}, B2^{a}, D2^{a}, D3^{a}, B2, and D2) as described herein, when two or more substituents selected from Group X are present, these substituents may be identical to or different from each other.

The expression of "CX-CY" as described herein means that the number of carbon atom is X to Y. For example, the expression of "C1-C6" means that the number of carbon atom is 1 to 6.

The term of "chain hydrocarbon group" represents an alkyl group, an alkenyl group, or an alkynyl group.

Examples of the term of "alkyl group" include a methyl group, an ethyl group, a propyl group, an isopropyl group, a 1,1-dimethylpropyl group, a 1,2-dimethylpropyl group, a butyl group, a sec-butyl group, a tert-butyl group, a pentyl group, and a hexyl group.

Examples of the term of "alkenyl group" include a vinyl group, a 1-propenyl group, a 2-propenyl group, a 1-methyl-1-propenyl group, a 1-methyl-2-propenyl group, a 1,2-dimethyl-1-propenyl group, a 3-butenyl group, a 4-pentenyl group, and a 5-hexenyl group.

Examples of the term of "alkynyl group" include an ethynyl group, a 1-propynyl group, a 2-propynyl group, a 1-methyl-2-propynyl group, a 1,1-dimethyl-2-propynyl group, a 2-butynyl group, a 4-pentynyl group, and a 5-hexynyl group.

Examples of the term of "alkoxy group" include a methoxy group, an ethoxy group, a propoxy group, an isopropoxy group, a butoxy group, a tert-butoxy group, a pentyloxy group, and a hexyloxy group.

Examples of the term of "alkylsulfanyl group" include a methylsulfanyl group, an ethylsulfanyl group, an isopropylsulfanyl group, and a hexylsulfanyl group.

Examples of the term of "alkylsulfinyl group" include a methylsulfinyl group, an ethylsulfinyl group, an isopropylsulfinyl group, and a hexylsulfinyl group.

Examples of the term of "alkylsulfonyl group" include a methanesulfonyl group, an ethanesulfonyl group, an isopropanesulfonyl group, and a hexanesulfonyl group.

Examples of the term of "alkylcarbonyl group" include an acetyl group and a propionyl group.

Examples of the term of "alkoxycarbonyl group" include a methoxycarbonyl group, an isopropoxycarbonyl group, and a hexyloxycarbonyl group.

Examples of the term of "alicyclic hydrocarbon" include a cyclobutene ring, a cyclopentene ring, a cyclopentadiene ring, a cyclohexene ring, a cyclohexadiene ring, and a cycloheptene ring.

Examples of the term of "alicyclic hydrocarbon group" include a cycloalkyl group and a cycloalkenyl group.

Examples of the term of "cycloalkyl group" include a cyclopropyl group, a cyclobutyl group, a cyclopentyl group, and a cyclohexyl group.

Examples of the term of "cycloalkenyl group" include a cyclopentenyl group and a cyclohexenyl group.

Examples of the term of "aryl group" include a phenyl group and a naphthyl group.

Examples of the term of "nonaromatic heterocyclic ring" include a dihydrofuran ring, a dihydrothiophene ring, a dihydropyrrole ring, a 1,3-dioxole ring, a pyran ring, and a dihydropyran ring.

Examples of the term of "aromatic heterocyclic ring" include a furan ring, a thiophene ring, a pyrrole ring, a pyrazole ring, an imidazole ring, an oxazole ring, an isoxazole ring, a thiazole ring, an isothiazole ring, a triazole ring, an oxadiazole ring, a thiadiazole ring, a pyridine ring, a pyridazine ring, a pyrimidine ring, and a pyrazine ring.

Examples of the term of "aromatic heterocyclic group" include 5 membered aromatic heterocyclic groups such as a pyrrolyl group, a furanyl group, a thienyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, and a thiadiazolyl group; 6 membered aromatic heterocyclic groups such as a pyridyl group, a pyridazinyl group, a pyrimidinyl group, a pyrazinyl group, a triazinyl group, and a tetrazinyl group; and bicyclic aromatic heterocyclic groups such as a benzofuranyl group.

Examples of the term of "nonaromatic heterocyclic group" include an aziridinyl group, an oxiranyl group, a thiiranyl group, an azetidinyl group, an oxetanyl group, a thietanyl group, a pyrrolidinyl group, a tetrahydrofuranyl group, a tetrahydrothienyl group, a pyrazolinyl group, a pyrazolidinyl group, an imidazolinyl group, an imidazolidinyl group, an oxazolinyl group, a thiazolinyl group, an oxazolidinyl group, a thiazolidinyl group, an isoxazolinyl group, an isoxazolidinyl group, an isothiazolinyl group, an isothiazolidinyl group, a dioxolanyl group, a dioxanyl group, a piperidyl group, a piperazinyl group, a morpholinyl group, a thiomorpholinyl group, a pyranyl group, a dihydropyranyl group, a tetrahydropyranyl group, a tetrahydrothiopyranyl group, an azepanyl group, an oxepanyl group, and a thiepanyl group.

4-{[3-(4-fluorophenyl)-1H-pyrazol-1-yl]sulfonyl}-1,3-dimethyl-1H-pyrazole is a compound represented by the following formula.

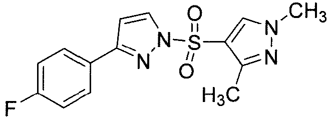

3-(2,3-dihydrobenzofuran-5-yl)-1-(thiophen-2-ylsulfonyl)-1H-pyrazole is a compound represented by the following formula.

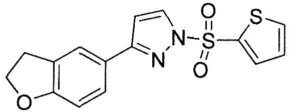

An N-oxide of the compound represented by formula (I) or formula (II) means a structure in which at least one nitrogen atom contained in the compound represented by formula (I) or formula (II) is substituted with an oxo group.

The Present compound or the Compound of the present invention may optionally have one or more stereoisomer(s). Examples of the stereoisomer(s) include enantiomers, diastereomers, atropisomers, and geometric isomers. The present invention encompasses each stereoisomer and mixtures of stereoisomers at any ratio.

The compound represented by formula (I) or formula (II), or an N-oxide thereof may optionally be mixed with an acid such as hydrochloric acid, sulfuric acid, nitric acid, phosphoric acid, acetic acid, and benzoic acid to form an acid addition salt such as hydrochloride, sulfate, nitrate, phosphate, acetate, and benzoate.

In the Present compound, a compound wherein adjacent R¹ and R³ are combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B}, a benzene ring, or a 5-7 membered aromatic heterocyclic ring {wherein said benzene ring and said 5-7 membered aromatic heterocyclic ring may be optionally substituted with one or more substituent(s) selected from Group E} specifically represents a compound represented by formula (IA-1) 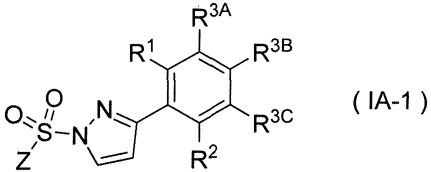 wherein
R² and Z represent the same meanings as defined in [1] ;
R^{3B} and R^{3C} are identical to or different from each other, and represent the same meanings as defined in R³ in [1] or a hydrogen atom; and
R¹ and R^{3A} are combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B}, a benzene ring, or a 5-7 membered aromatic heterocyclic ring {wherein said benzene ring and said 5-7 membered aromatic heterocyclic ring may be optionally substituted with one or more substituent(s) selected from Group E}.

In the Present compound, a compound wherein adjacent two R³ and R³ are combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B}, a benzene ring, or a 5-7 membered aromatic heterocyclic ring {wherein said benzene ring and said 5-7 membered aromatic heterocyclic ring may be optionally substituted with one or more substituent(s) selected from Group E} specifically represents:
1) a compound represented by formula (IA-1), wherein
   R¹, R², and Z represent the same meanings as defined in [1];
   R^{3C} represents the same meaning as defined in R³ in [1] or a hydrogen atom; and
   R^{3A} and R^{3B} are combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B}, a benzene ring, or a 5-7 membered aromatic heterocyclic ring {wherein said benzene ring and said 5-7 membered aromatic heterocyclic ring may be optionally substituted with one or more substituent(s) selected from Group E}; or 2) a compound represented by formula (IA-1), wherein
   R¹, R², and Z represent the same meanings as defined in [1] ;
   R^{3A} represents the same meaning as defined in R³ in [1] or a hydrogen atom; and
   R^{3B} and R^{3C} are combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B}, a benzene ring, or a 5-7 membered aromatic heterocyclic ring {wherein said benzene ring and said 5-7 membered aromatic heterocyclic ring may be optionally substituted with one or more substituent(s) selected from Group E}.

In the Compound of the present invention, a compound wherein adjacent R^{1a} and R^{3a} are combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B^{a}}, a benzene ring, or a 5-7 membered aromatic heterocyclic ring {wherein said benzene ring and said 5-7 membered aromatic heterocyclic ring may be optionally substituted with one or more substituent(s) selected from Group E^{a}} specifically represents a compound represented by formula (IIA-1) 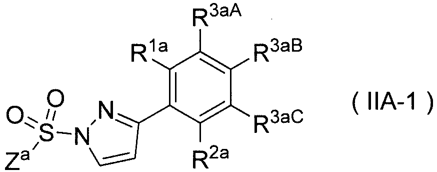 wherein
R^{2a} and Z^{a} represent the same meanings as defined in [4] ;
R^{3aB} and R^{3aC} are identical to or different from each other, and represent the same meanings as defined in R^{3a} in [4] or a hydrogen atom; and
R^{1a} and R^{3aA} are combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B^{a}}, a benzene ring, or a 5-7 membered aromatic heterocyclic ring {wherein said benzene ring and said 5-7 membered aromatic heterocyclic ring may be optionally substituted with one or more substituent(s) selected from Group E^{a}}.

In the Compound of the present invention, a compound wherein adjacent two R^{3a} and R^{3a} are combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B^{a}}, a benzene ring, or a 5-7 membered aromatic heterocyclic ring {wherein said benzene ring and said 5-7 membered aromatic heterocyclic ring may be optionally substituted with one or more substituent(s) selected from Group E^{a}} specifically represents:
1) a compound represented by formula (IIA-1), wherein
   R^{1a}, R^{2a}, and Z^{a} represent the same meanings as defined in [4];
   R^{3aC} represents the same meaning as defined in R^{3a} in [4] or a hydrogen atom; and
   R^{3aA} and R^{3aB} are combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B^{a}}, a benzene ring, or a 5-7 membered aromatic heterocyclic ring {wherein said benzene ring and said 5-7 membered aromatic heterocyclic ring may be optionally substituted with one or more substituent(s) selected from Group E^{a}}; or
2) a compound represented by formula (IIA-1), wherein
   R^{1a}, R^{2a}, and Z^{a} represent the same meanings as defined in [4];
   R^{3aA} represents the same meaning as defined in R^{3a} in [4] or a hydrogen atom; and
   R^{3aB} and R^{3aC} are combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B^{a}}, a benzene ring, or a 5-7 membered aromatic heterocyclic ring {wherein said benzene ring and said 5-7 membered aromatic heterocyclic ring may be optionally substituted with one or more substituent(s) selected from Group E^{a}}.

Aspects of the Present compound N include the following compounds.
[Aspect 1] The Present compound N, wherein R³ represents a C1-C6 chain hydrocarbon group optionally substituted with one or more substituent(s) selected from Group A or a C3-C8 alicyclic hydrocarbon group optionally substituted with one or more halogen atom(s).
[Aspect 2] The Present compound N, wherein R³ represents a halogen atom.
[Aspect 3] The Present compound N, wherein
   R³ represents a nitro group, NR⁸R⁹, or C(O)NR⁴R⁵; and
   R⁴, R⁵, R⁸, and R⁹ are identical to or different from each other, and each represent a C1-C3 alkyl group or a hydrogen atom.
[Aspect 4] The Present compound N, wherein
   R³ represents a C1-C6 chain hydrocarbon group optionally substituted with one or more substituent(s) selected from Group A, a C3-C8 alicyclic hydrocarbon group optionally substituted with one or more halogen atom(s), a halogen atom, a nitro group, NR⁸R⁹, or C(O)NR⁴R⁵; and
   R⁴, R⁵, R⁸, and R⁹ are identical to or different from each other, and each represent a C1-C3 alkyl group or a hydrogen atom.
[Aspect 5] The Present compound N, wherein adjacent R¹ and R³ are combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B}, a benzene ring, or a 5-7 membered aromatic heterocyclic ring {wherein said benzene ring and said 5-7 membered aromatic heterocyclic ring may be optionally substituted with one or more substituent(s) selected from Group E}.
[Aspect 6] The Present compound N, wherein adjacent R¹ and R³ are combined with the carbon atoms to which they are attached to form a C5-C6 alicyclic hydrocarbon or a 5-6 membered nonaromatic heterocyclic ring {wherein said C5-C6 alicyclic hydrocarbon and said 5-6 membered nonaromatic heterocyclic ring may be optionally substituted with one or more substituent(s) selected from Group B2};
   Group B2: a group consisting of a C1-C6 chain hydrocarbon group optionally substituted with one or more halogen atom(s), and a halogen atom.
[Aspect 7] The Present compound N, wherein adjacent R¹ and R³ are combined with the carbon atoms to which they are attached to form a benzene ring or a 5-6 membered aromatic heterocyclic ring {wherein said benzene ring and said 5-6 membered aromatic heterocyclic ring may be optionally substituted with one or more substituent(s) selected from Group B2}.
[Aspect 8] The Present compound N, wherein adjacent two R³ and R³ are combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B}, a benzene ring, or a 5-7 membered aromatic heterocyclic ring {wherein said benzene ring and said 5-7 membered aromatic heterocyclic ring may be optionally substituted with one or more substituent(s) selected from Group E}.
[Aspect 9] The Present compound N, wherein adjacent two R³ and R³ are combined with the carbon atoms to which they are attached to form a C5-C6 alicyclic hydrocarbon or a 5-6 membered nonaromatic heterocyclic ring {wherein said C5-C6 alicyclic hydrocarbon and said 5-6 membered nonaromatic heterocyclic ring may be optionally substituted with one or more substituent(s) selected from Group B2}.
[Aspect 10] The Present compound N, wherein adjacent two R³ and R³ are combined with the carbon atoms to which they are attached to form a benzene ring or a 5-6 membered aromatic heterocyclic ring {wherein said benzene ring and said 5-6 membered aromatic heterocyclic ring may be optionally substituted with one or more substituent(s) selected from Group B2}.
[Aspect 11] The Present compound N, wherein adjacent two R³ and R³ are combined with the carbon atoms to which they are attached to form a C5-C6 alicyclic hydrocarbon, a 5-6 membered nonaromatic heterocyclic ring, a benzene ring, or a 5-6 membered aromatic heterocyclic ring {wherein said C5-C6 alicyclic hydrocarbon, said 5-6 membered nonaromatic heterocyclic ring, said benzene ring, and said 5-6 membered aromatic heterocyclic ring may be optionally substituted with one or more substituent(s) selected from Group B2}.
[Aspect 12] The Present compound N, wherein
   R³ represents a C1-C6 chain hydrocarbon group optionally substituted with one or more substituent(s) selected from Group A, a C1-C3 alkoxy group, a C3-C8 alicyclic hydrocarbon group {wherein said C1-C3 alkoxy group and said C3-C8 alicyclic hydrocarbon group may be optionally substituted with one or more halogen atom(s)}, a halogen atom, a nitro group, NR⁸R⁹, or C(O)NR⁴R⁵; and
   R⁴, R⁵, R⁸, and R⁹ are identical to or different from each other, and each represent a C1-C3 alkyl group, a cyclopropyl group, or a hydrogen atom.
[Aspect 13] The Present compound N, wherein R³ represents a C1-C3 chain hydrocarbon group, a C1-C3 alkoxy group {wherein said C1-C3 chain hydrocarbon group and said C1-C3 alkoxy group may be optionally substituted with one or more halogen atom(s)}, a cyclopropyl group, a halogen atom, a nitro group, NR⁸R⁹, or C(O)NR⁴R⁵; and
   R⁴, R⁵, R⁸, and R⁹ are identical to or different from each other, and each represent a C1-C3 alkyl group, a cyclopropyl group, or a hydrogen atom.
[Aspect 14] The Present compound N, wherein
   R³ represents a C1-C6 chain hydrocarbon group optionally substituted with one or more substituent(s) selected from Group A, a C1-C3 alkoxy group, a C3-C8 alicyclic hydrocarbon group {wherein said C1-C3 alkoxy group and said C3-C8 alicyclic hydrocarbon group may be optionally substituted with one or more halogen atom(s)}, a halogen atom, a nitro group, NR⁸R⁹, or C(O)NR⁴R⁵; and
   R⁴, R⁵, R⁸, and R⁹ are identical to or different from each other, and each represent a C1-C3 alkyl group, a cyclopropyl group, or a hydrogen atom;
   or adjacent two R³ and R³ may be optionally combined with the carbon atoms to which they are attached to form a C5-C6 alicyclic hydrocarbon, a 5-6 membered nonaromatic heterocyclic ring, a benzene ring, or a 5-6 membered aromatic heterocyclic ring {wherein said C5-C6 alicyclic hydrocarbon, said 5-6 membered nonaromatic heterocyclic ring, said benzene ring, and said 5-6 membered aromatic heterocyclic ring may be optionally substituted with one or more substituent(s) selected from Group B2}.
[Aspect 15] The Present compound N, wherein
   R³ represents a C1-C3 chain hydrocarbon group, a C1-C3 alkoxy group {wherein said C1-C3 chain hydrocarbon group and said C1-C3 alkoxy group may be optionally substituted with one or more halogen atom(s)}, a cyclopropyl group, a halogen atom, a nitro group, NR⁸R⁹, or C(O)NR⁴R⁵; and
   R⁴, R⁵, R⁸, and R⁹ are identical to or different from each other, and each represent a hydrogen atom, a cyclopropyl group, or a C1-C3 alkyl group;
   or adjacent two R³ and R³ may be optionally combined with the carbon atoms to which they are attached to form a C5-C6 alicyclic hydrocarbon, a 5-6 membered nonaromatic heterocyclic ring, or a 5-6 membered aromatic heterocyclic ring {wherein said C5-C6 alicyclic hydrocarbon, said 5-6 membered nonaromatic heterocyclic ring, and said 5-6 membered aromatic heterocyclic ring may be optionally substituted with one or more substituent(s) selected from Group B2}.
[Aspect 16] The Present compound N, wherein p represents 0.
[Aspect 17] The Present compound N, wherein p represents 1.
[Aspect 18] The Present compound N, wherein p represents 2.
[Aspect 19] The Present compound N, wherein p represents 3.
[Aspect 20] The Present compound N, wherein p represents 1, 2, or 3.
[Aspect 21] The compound according to the Aspect 1, wherein p represents 1.
[Aspect 22] The compound according to the Aspect 1, wherein p represents 2.
[Aspect 23] The compound according to the Aspect 1, wherein p represents 3.
[Aspect 24] The compound according to the Aspect 1, wherein p represents 1, 2, or 3.
[Aspect 25] The compound according to the Aspect 2, wherein p represents 1.
[Aspect 26] The compound according to the Aspect 2, wherein p represents 2.
[Aspect 27] The compound according to the Aspect 2, wherein p represents 3.
[Aspect 28] The compound according to the Aspect 2, wherein p represents 1, 2, or 3.
[Aspect 29] The compound according to the Aspect 3, wherein p represents 1.
[Aspect 30] The compound according to the Aspect 3, wherein p represents 2.
[Aspect 31] The compound according to the Aspect 3, wherein p represents 3.
[Aspect 32] The compound according to the Aspect 3, wherein p represents 1, 2, or 3.
[Aspect 33] The compound according to the Aspect 4, wherein p represents 1.
[Aspect 34] The compound according to the Aspect 4, wherein p represents 2.
[Aspect 35] The compound according to the Aspect 4, wherein p represents 3.
[Aspect 36] The compound according to the Aspect 4, wherein p represents 1, 2, or 3.
[Aspect 37] The compound according to the Aspect 5, wherein p represents 1.
[Aspect 38] The compound according to the Aspect 5, wherein p represents 2.
[Aspect 39] The compound according to the Aspect 5, wherein p represents 3.
[Aspect 40] The compound according to the Aspect 5, wherein p represents 1, 2 or 3.
[Aspect 41] The compound according to the Aspect 6, wherein p represents 1.
[Aspect 42] The compound according to the Aspect 6, wherein p represents 2.
[Aspect 43] The compound according to the Aspect 6, wherein p represents 3.
[Aspect 44] The compound according to the Aspect 6, wherein p represents 1, 2, or 3.
[Aspect 45] The compound according to the Aspect 7, wherein p represents 1.
[Aspect 46] The compound according to the Aspect 7, wherein p represents 2.
[Aspect 47] The compound according to the Aspect 7, wherein p represents 3.
[Aspect 48] The compound according to the Aspect 7, wherein p represents 1, 2, or 3.
[Aspect 49] The compound according to the Aspect 8, wherein p represents 2.
[Aspect 50] The compound according to the Aspect 8, wherein p represents 3.
[Aspect 51] The compound according to the Aspect 8, wherein p represents 2 or 3.
[Aspect 52] The compound according to the Aspect 9, wherein p represents 2.
[Aspect 53] The compound according to the Aspect 9, wherein p represents 3.
[Aspect 54] The compound according to the Aspect 9, wherein p represents 2 or 3.
[Aspect 55] The compound according to the Aspect 10, wherein p represents 2.
[Aspect 56] The compound according to the Aspect 10, wherein p represents 3.
[Aspect 57] The compound according to the Aspect 10, wherein p represents 2 or 3.
[Aspect 58] The compound according to the Aspect 11, wherein p represents 2.
[Aspect 59] The compound according to the Aspect 11, wherein p represents 3.
[Aspect 60] The compound according to the Aspect 11, wherein p represents 2 or 3.
[Aspect 61] The compound according to the Aspect 12, wherein p represents 1.
[Aspect 62] The compound according to the Aspect 12, wherein p represents 2.
[Aspect 63] The compound according to the Aspect 12, wherein p represents 3.
[Aspect 64] The compound according to the Aspect 12, wherein p represents 1, 2, or 3.
[Aspect 65] The compound according to the Aspect 13, wherein p represents 1.
[Aspect 66] The compound according to the Aspect 13, wherein p represents 2.
[Aspect 67] The compound according to the Aspect 13, wherein p represents 3.
[Aspect 68] The compound according to the Aspect 13, wherein p represents 1, 2, or 3.
[Aspect 69] The compound according to the Aspect 14, wherein p represents 1.
[Aspect 70] The compound according to the Aspect 14, wherein p represents 2.
[Aspect 71] The compound according to the Aspect 14, wherein p represents 3.
[Aspect 72] The compound according to the Aspect 14, wherein p represents 1, 2, or 3.
[Aspect 73] The compound according to the Aspect 15, wherein p represents 1.
[Aspect 74] The compound according to the Aspect 15, wherein p represents 2.
[Aspect 75] The compound according to the Aspect 15, wherein p represents 3.
[Aspect 76] The compound according to the Aspect 15, wherein p represents 1, 2, or 3.
[Aspect 77] The Present compound N, wherein R¹ and R² are identical to or different from each other, and each represent a C1-C3 alkyl group or a hydrogen atom.
[Aspect 78] The compound according to the Aspect 1, wherein R¹ and R² are identical to or different from each other, and each represent a C1-C3 alkyl group or a hydrogen atom.
[Aspect 79] The compound according to the Aspect 2, wherein R¹ and R² are identical to or different from each other, and each represent a C1-C3 alkyl group or a hydrogen atom.
[Aspect 80] The compound according to the Aspect 3, wherein R¹ and R² are identical to or different from each other, and each represent a C1-C3 alkyl group or a hydrogen atom.
[Aspect 81] The compound according to the Aspect 4, wherein R¹ and R² are identical to or different from each other, and each represent a C1-C3 alkyl group or a hydrogen atom.
[Aspect 82] The compound according to the Aspect 5, wherein R¹ and R² are identical to or different from each other, and each represent a C1-C3 alkyl group or a hydrogen atom.
[Aspect 83] The compound according to the Aspect 6, wherein R¹ and R² are identical to or different from each other, and each represent a C1-C3 alkyl group or a hydrogen atom.
[Aspect 84] The compound according to the Aspect 7, wherein R¹ and R² are identical to or different from each other, and each represent a C1-C3 alkyl group or a hydrogen atom.
[Aspect 85] The compound according to the Aspect 8, wherein R¹ and R² are identical to or different from each other, and each represent a C1-C3 alkyl group or a hydrogen atom.
[Aspect 86] The compound according to the Aspect 9, wherein R¹ and R² are identical to or different from each other, and each represent a C1-C3 alkyl group or a hydrogen atom.
[Aspect 87] The compound according to the Aspect 10, wherein R¹ and R² are identical to or different from each other, and each represent a C1-C3 alkyl group or a hydrogen atom.
[Aspect 88] The compound according to the Aspect 11, wherein R¹ and R² are identical to or different from each other, and each represent a C1-C3 alkyl group or a hydrogen atom.
[Aspect 89] The compound according to the Aspect 12, wherein R¹ and R² are identical to or different from each other, and each represent a C1-C3 alkyl group or a hydrogen atom.
[Aspect 90] The compound according to the Aspect 13, wherein R¹ and R² are identical to or different from each other, and each represent a C1-C3 alkyl group or a hydrogen atom.
[Aspect 91] The compound according to the Aspect 14, wherein R¹ and R² are identical to or different from each other, and each represent a C1-C3 alkyl group or a hydrogen atom.
[Aspect 92] The compound according to the Aspect 15, wherein R¹ and R² are identical to or different from each other, and each represent a C1-C3 alkyl group or a hydrogen atom.
[Aspect 93] The compound according to the Aspect 16, wherein R¹ and R² are identical to or different from each other, and each represent a C1-C3 alkyl group or a hydrogen atom.
[Aspect 94] The compound according to the Aspect 17, wherein R¹ and R² are identical to or different from each other, and each represent a C1-C3 alkyl group or a hydrogen atom.
[Aspect 95] The compound according to the Aspect 18, wherein R¹ and R² are identical to or different from each other, and each represent a C1-C3 alkyl group or a hydrogen atom.
[Aspect 96] The compound according to the Aspect 19, wherein R¹ and R² are identical to or different from each other, and each represent a C1-C3 alkyl group or a hydrogen atom.
[Aspect 97] The compound according to the Aspect 20, wherein R¹ and R² are identical to or different from each other, and each represent a C1-C3 alkyl group or a hydrogen atom.
[Aspect 98] The compound according to the Aspect 21, wherein R¹ and R² are identical to or different from each other, and each represent a C1-C3 alkyl group or a hydrogen atom.
[Aspect 99] The compound according to the Aspect 22, wherein R¹ and R² are identical to or different from each other, and each represent a C1-C3 alkyl group or a hydrogen atom.
[Aspect 100] The compound according to the Aspect 23, wherein R¹ and R² are identical to or different from each other, and each represent a C1-C3 alkyl group or a hydrogen atom.
[Aspect 101] The compound according to the Aspect 24, wherein R¹ and R² are identical to or different from each other, and each represent a C1-C3 alkyl group or a hydrogen atom.
[Aspect 102] The compound according to the Aspect 25, wherein R¹ and R² are identical to or different from each other, and each represent a C1-C3 alkyl group or a hydrogen atom.
[Aspect 103] The compound according to the Aspect 26, wherein R¹ and R² are identical to or different from each other, and each represent a C1-C3 alkyl group or a hydrogen atom.
[Aspect 104] The compound according to the Aspect 27, wherein R¹ and R² are identical to or different from each other, and each represent a C1-C3 alkyl group or a hydrogen atom.
[Aspect 105] The compound according to the Aspect 28, wherein R¹ and R² are identical to or different from each other, and each represent a C1-C3 alkyl group or a hydrogen atom.
[Aspect 106] The compound according to the Aspect 29, wherein R¹ and R² are identical to or different from each other, and each represent a C1-C3 alkyl group or a hydrogen atom.
[Aspect 107] The compound according to the Aspect 30, wherein R¹ and R² are identical to or different from each other, and each represent a C1-C3 alkyl group or a hydrogen atom.
[Aspect 108] The compound according to the Aspect 31, wherein R¹ and R² are identical to or different from each other, and each represent a C1-C3 alkyl group or a hydrogen atom.
[Aspect 109] The compound according to the Aspect 32, wherein R¹ and R² are identical to or different from each other, and each represent a C1-C3 alkyl group or a hydrogen atom.
[Aspect 110] The compound according to the Aspect 33, wherein R¹ and R² are identical to or different from each other, and each represent a C1-C3 alkyl group or a hydrogen atom.
[Aspect 111] The compound according to the Aspect 34, wherein R¹ and R² are identical to or different from each other, and each represent a C1-C3 alkyl group or a hydrogen atom.
[Aspect 112] The compound according to the Aspect 35, wherein R¹ and R² are identical to or different from each other, and each represent a C1-C3 alkyl group or a hydrogen atom.
[Aspect 113] The compound according to the Aspect 36, wherein R¹ and R² are identical to or different from each other, and each represent a C1-C3 alkyl group or a hydrogen atom.
[Aspect 114] The compound according to the Aspect 37, wherein R¹ and R² are identical to or different from each other, and each represent a C1-C3 alkyl group or a hydrogen atom.
[Aspect 115] The compound according to the Aspect 38, wherein R¹ and R² are identical to or different from each other, and each represent a C1-C3 alkyl group or a hydrogen atom.
[Aspect 116] The compound according to the Aspect 39, wherein R¹ and R² are identical to or different from each other, and each represent a C1-C3 alkyl group or a hydrogen atom.
[Aspect 117] The compound according to the Aspect 40, wherein R¹ and R² are identical to or different from each other, and each represent a C1-C3 alkyl group or a hydrogen atom.
[Aspect 118] The compound according to the Aspect 41, wherein R¹ and R² are identical to or different from each other, and each represent a C1-C3 alkyl group or a hydrogen atom.
[Aspect 119] The compound according to the Aspect 42, wherein R¹ and R² are identical to or different from each other, and each represent a C1-C3 alkyl group or a hydrogen atom.
[Aspect 120] The compound according to the Aspect 43, wherein R¹ and R² are identical to or different from each other, and each represent a C1-C3 alkyl group or a hydrogen atom.
[Aspect 121] The compound according to the Aspect 44, wherein R¹ and R² are identical to or different from each other, and each represent a C1-C3 alkyl group or a hydrogen atom.
[Aspect 122] The compound according to the Aspect 45, wherein R¹ and R² are identical to or different from each other, and each represent a C1-C3 alkyl group or a hydrogen atom.
[Aspect 123] The compound according to the Aspect 46, wherein R¹ and R² are identical to or different from each other, and each represent a C1-C3 alkyl group or a hydrogen atom.
[Aspect 124] The compound according to the Aspect 47, wherein R¹ and R² are identical to or different from each other, and each represent a C1-C3 alkyl group or a hydrogen atom.
[Aspect 125] The compound according to the Aspect 48, wherein R¹ and R² are identical to or different from each other, and each represent a C1-C3 alkyl group or a hydrogen atom.
[Aspect 126] The compound according to the Aspect 49, wherein R¹ and R² are identical to or different from each other, and each represent a C1-C3 alkyl group or a hydrogen atom.
[Aspect 127] The compound according to the Aspect 50, wherein R¹ and R² are identical to or different from each other, and each represent a C1-C3 alkyl group or a hydrogen atom.
[Aspect 128] The compound according to the Aspect 51, wherein R¹ and R² are identical to or different from each other, and each represent a C1-C3 alkyl group or a hydrogen atom.
[Aspect 129] The compound according to the Aspect 52, wherein R¹ and R² are identical to or different from each other, and each represent a C1-C3 alkyl group or a hydrogen atom.
[Aspect 130] The compound according to the Aspect 53, wherein R¹ and R² are identical to or different from each other, and each represent a C1-C3 alkyl group or a hydrogen atom.
[Aspect 131] The compound according to the Aspect 54, wherein R¹ and R² are identical to or different from each other, and each represent a C1-C3 alkyl group or a hydrogen atom.
[Aspect 132] The compound according to the Aspect 55, wherein R¹ and R² are identical to or different from each other, and each represent a C1-C3 alkyl group or a hydrogen atom.
[Aspect 133] The compound according to the Aspect 56, wherein R¹ and R² are identical to or different from each other, and each represent a C1-C3 alkyl group or a hydrogen atom.
[Aspect 134] The compound according to the Aspect 57, wherein R¹ and R² are identical to or different from each other, and each represent a C1-C3 alkyl group or a hydrogen atom.
[Aspect 135] The compound according to the Aspect 58, wherein R¹ and R² are identical to or different from each other, and each represent a C1-C3 alkyl group or a hydrogen atom.
[Aspect 136] The compound according to the Aspect 59, wherein R¹ and R² are identical to or different from each other, and each represent a C1-C3 alkyl group or a hydrogen atom.
[Aspect 137] The compound according to the Aspect 60, wherein R¹ and R² are identical to or different from each other, and each represent a C1-C3 alkyl group or a hydrogen atom.
[Aspect 138] The compound according to the Aspect 61, wherein R¹ and R² are identical to or different from each other, and each represent a C1-C3 alkyl group or a hydrogen atom.
[Aspect 139] The compound according to the Aspect 62, wherein R¹ and R² are identical to or different from each other, and each represent a C1-C3 alkyl group or a hydrogen atom.
[Aspect 140] The compound according to the Aspect 63, wherein R¹ and R² are identical to or different from each other, and each represent a C1-C3 alkyl group or a hydrogen atom.
[Aspect 141] The compound according to the Aspect 64, wherein R¹ and R² are identical to or different from each other, and each represent a C1-C3 alkyl group or a hydrogen atom.
[Aspect 142] The compound according to the Aspect 65, wherein R¹ and R² are identical to or different from each other, and each represent a C1-C3 alkyl group or a hydrogen atom.
[Aspect 143] The compound according to the Aspect 66, wherein R¹ and R² are identical to or different from each other, and each represent a C1-C3 alkyl group or a hydrogen atom.
[Aspect 144] The compound according to the Aspect 67, wherein R¹ and R² are identical to or different from each other, and each represent a C1-C3 alkyl group or a hydrogen atom.
[Aspect 145] The compound according to the Aspect 68, wherein R¹ and R² are identical to or different from each other, and each represent a C1-C3 alkyl group or a hydrogen atom.
[Aspect 146] The compound according to the Aspect 69, wherein R¹ and R² are identical to or different from each other, and each represent a C1-C3 alkyl group or a hydrogen atom.
[Aspect 147] The compound according to the Aspect 70, wherein R¹ and R² are identical to or different from each other, and each represent a C1-C3 alkyl group or a hydrogen atom.
[Aspect 148] The compound according to the Aspect 71, wherein R¹ and R² are identical to or different from each other, and each represent a C1-C3 alkyl group or a hydrogen atom.
[Aspect 149] The compound according to the Aspect 72, wherein R¹ and R² are identical to or different from each other, and each represent a C1-C3 alkyl group or a hydrogen atom.
[Aspect 150] The compound according to the Aspect 73, wherein R¹ and R² are identical to or different from each other, and each represent a C1-C3 alkyl group or a hydrogen atom.
[Aspect 151] The compound according to the Aspect 74, wherein R¹ and R² are identical to or different from each other, and each represent a C1-C3 alkyl group or a hydrogen atom.
[Aspect 152] The compound according to the Aspect 75, wherein R¹ and R² are identical to or different from each other, and each represent a C1-C3 alkyl group or a hydrogen atom.
[Aspect 153] The compound according to the Aspect 76, wherein R¹ and R² are identical to or different from each other, and each represent a C1-C3 alkyl group or a hydrogen atom.
[Aspect 154] The Present compound N, wherein R¹ and R² each represent a hydrogen atom.
[Aspect 155] The compound according to the Aspect 1, wherein R¹ and R² each represent a hydrogen atom.
[Aspect 156] The compound according to the Aspect 2, wherein R¹ and R² each represent a hydrogen atom.
[Aspect 157] The compound according to the Aspect 3, wherein R¹ and R² each represent a hydrogen atom.
[Aspect 158] The compound according to the Aspect 4, wherein R¹ and R² each represent a hydrogen atom.
[Aspect 159] The compound according to the Aspect 5, wherein R¹ and R² each represent a hydrogen atom.
[Aspect 160] The compound according to the Aspect 6, wherein R¹ and R² each represent a hydrogen atom.
[Aspect 161] The compound according to the Aspect 7, wherein R¹ and R² each represent a hydrogen atom.
[Aspect 162] The compound according to the Aspect 8, wherein R¹ and R² each represent a hydrogen atom.
[Aspect 163] The compound according to the Aspect 9, wherein R¹ and R² each represent a hydrogen atom.
[Aspect 164] The compound according to the Aspect 10, wherein R¹ and R² each represent a hydrogen atom.
[Aspect 165] The compound according to the Aspect 11, wherein R¹ and R² each represent a hydrogen atom.
[Aspect 166] The compound according to the Aspect 12, wherein R¹ and R² each represent a hydrogen atom.
[Aspect 167] The compound according to the Aspect 13, wherein R¹ and R² each represent a hydrogen atom.
[Aspect 168] The compound according to the Aspect 14, wherein R¹ and R² each represent a hydrogen atom.
[Aspect 169] The compound according to the Aspect 15, wherein R¹ and R² each represent a hydrogen atom.
[Aspect 170] The compound according to the Aspect 16, wherein R¹ and R² each represent a hydrogen atom.
[Aspect 171] The compound according to the Aspect 17, wherein R¹ and R² each represent a hydrogen atom.
[Aspect 172] The compound according to the Aspect 18, wherein R¹ and R² each represent a hydrogen atom.
[Aspect 173] The compound according to the Aspect 19, wherein R¹ and R² each represent a hydrogen atom.
[Aspect 174] The compound according to the Aspect 20, wherein R¹ and R² each represent a hydrogen atom.
[Aspect 175] The compound according to the Aspect 21, wherein R¹ and R² each represent a hydrogen atom.
[Aspect 176] The compound according to the Aspect 22, wherein R¹ and R² each represent a hydrogen atom.
[Aspect 177] The compound according to the Aspect 23, wherein R¹ and R² each represent a hydrogen atom.
[Aspect 178] The compound according to the Aspect 24, wherein R¹ and R² each represent a hydrogen atom.
[Aspect 179] The compound according to the Aspect 25, wherein R¹ and R² each represent a hydrogen atom.
[Aspect 180] The compound according to the Aspect 26, wherein R¹ and R² each represent a hydrogen atom.
[Aspect 181] The compound according to the Aspect 27, wherein R¹ and R² each represent a hydrogen atom.
[Aspect 182] The compound according to the Aspect 28, wherein R¹ and R² each represent a hydrogen atom.
[Aspect 183] The compound according to the Aspect 29, wherein R¹ and R² each represent a hydrogen atom.
[Aspect 184] The compound according to the Aspect 30, wherein R¹ and R² each represent a hydrogen atom.
[Aspect 185] The compound according to the Aspect 31, wherein R¹ and R² each represent a hydrogen atom.
[Aspect 186] The compound according to the Aspect 32, wherein R¹ and R² each represent a hydrogen atom.
[Aspect 187] The compound according to the Aspect 33, wherein R¹ and R² each represent a hydrogen atom.
[Aspect 188] The compound according to the Aspect 34, wherein R¹ and R² each represent a hydrogen atom.
[Aspect 189] The compound according to the Aspect 35, wherein R¹ and R² each represent a hydrogen atom.
[Aspect 190] The compound according to the Aspect 36, wherein R¹ and R² each represent a hydrogen atom.
[Aspect 191] The compound according to the Aspect 37, wherein R¹ and R² each represent a hydrogen atom.
[Aspect 192] The compound according to the Aspect 38, wherein R¹ and R² each represent a hydrogen atom.
[Aspect 193] The compound according to the Aspect 39, wherein R¹ and R² each represent a hydrogen atom.
[Aspect 194] The compound according to the Aspect 40, wherein R¹ and R² each represent a hydrogen atom.
[Aspect 195] The compound according to the Aspect 41, wherein R¹ and R² each represent a hydrogen atom.
[Aspect 196] The compound according to the Aspect 42, wherein R¹ and R² each represent a hydrogen atom.
[Aspect 197] The compound according to the Aspect 43, wherein R¹ and R² each represent a hydrogen atom.
[Aspect 198] The compound according to the Aspect 44, wherein R¹ and R² each represent a hydrogen atom.
[Aspect 199] The compound according to the Aspect 45, wherein R¹ and R² each represent a hydrogen atom.
[Aspect 200] The compound according to the Aspect 46, wherein R¹ and R² each represent a hydrogen atom.
[Aspect 201] The compound according to the Aspect 47, wherein R¹ and R² each represent a hydrogen atom.
[Aspect 202] The compound according to the Aspect 48, wherein R¹ and R² each represent a hydrogen atom.
[Aspect 203] The compound according to the Aspect 49, wherein R¹ and R² each represent a hydrogen atom.
[Aspect 204] The compound according to the Aspect 50, wherein R¹ and R² each represent a hydrogen atom.
[Aspect 205] The compound according to the Aspect 51, wherein R¹ and R² each represent a hydrogen atom.
[Aspect 206] The compound according to the Aspect 52, wherein R¹ and R² each represent a hydrogen atom.
[Aspect 207] The compound according to the Aspect 53, wherein R¹ and R² each represent a hydrogen atom.
[Aspect 208] The compound according to the Aspect 54, wherein R¹ and R² each represent a hydrogen atom.
[Aspect 209] The compound according to the Aspect 55, wherein R¹ and R² each represent a hydrogen atom.
[Aspect 210] The compound according to the Aspect 56, wherein R¹ and R² each represent a hydrogen atom.
[Aspect 211] The compound according to the Aspect 57, wherein R¹ and R² each represent a hydrogen atom.
[Aspect 212] The compound according to the Aspect 58, wherein R¹ and R² each represent a hydrogen atom.
[Aspect 213] The compound according to the Aspect 59, wherein R¹ and R² each represent a hydrogen atom.
[Aspect 214] The compound according to the Aspect 60, wherein R¹ and R² each represent a hydrogen atom.
[Aspect 215] The compound according to the Aspect 61, wherein R¹ and R² each represent a hydrogen atom.
[Aspect 216] The compound according to the Aspect 62, wherein R¹ and R² each represent a hydrogen atom.
[Aspect 217] The compound according to the Aspect 63, wherein R¹ and R² each represent a hydrogen atom.
[Aspect 218] The compound according to the Aspect 64, wherein R¹ and R² each represent a hydrogen atom.
[Aspect 219] The compound according to the Aspect 65, wherein R¹ and R² each represent a hydrogen atom.
[Aspect 220] The compound according to the Aspect 66, wherein R¹ and R² each represent a hydrogen atom.
[Aspect 221] The compound according to the Aspect 67, wherein R¹ and R² each represent a hydrogen atom.
[Aspect 222] The compound according to the Aspect 68, wherein R¹ and R² each represent a hydrogen atom.
[Aspect 223] The compound according to the Aspect 69, wherein R¹ and R² each represent a hydrogen atom.
[Aspect 224] The compound according to the Aspect 70, wherein R¹ and R² each represent a hydrogen atom.
[Aspect 225] The compound according to the Aspect 71, wherein R¹ and R² each represent a hydrogen atom.
[Aspect 226] The compound according to the Aspect 72, wherein R¹ and R² each represent a hydrogen atom.
[Aspect 227] The compound according to the Aspect 73, wherein R¹ and R² each represent a hydrogen atom.
[Aspect 228] The compound according to the Aspect 74, wherein R¹ and R² each represent a hydrogen atom.
[Aspect 229] The compound according to the Aspect 75, wherein R¹ and R² each represent a hydrogen atom.
[Aspect 230] The compound according to the Aspect 76, wherein R¹ and R² each represent a hydrogen atom.
[Aspect 231] The Present compound N, wherein R¹ and R² are identical to or different from each other, and each represent a hydrogen atom or a halogen atom.
[Aspect 232] The compound according to the Aspect 1, wherein R¹ and R² are identical to or different from each other, and each represent a hydrogen atom or a halogen atom.
[Aspect 233] The compound according to the Aspect 2, wherein R¹ and R² are identical to or different from each other, and each represent a hydrogen atom or a halogen atom.
[Aspect 234] The compound according to the Aspect 3, wherein R¹ and R² are identical to or different from each other, and each represent a hydrogen atom or a halogen atom.
[Aspect 235] The compound according to the Aspect 4, wherein R¹ and R² are identical to or different from each other, and each represent a hydrogen atom or a halogen atom.
[Aspect 236] The compound according to the Aspect 5, wherein R¹ and R² are identical to or different from each other, and each represent a hydrogen atom or a halogen atom.
[Aspect 237] The compound according to the Aspect 6, wherein R¹ and R² are identical to or different from each other, and each represent a hydrogen atom or a halogen atom.
[Aspect 238] The compound according to the Aspect 7, wherein R¹ and R² are identical to or different from each other, and each represent a hydrogen atom or a halogen atom.
[Aspect 239] The compound according to the Aspect 8, wherein R¹ and R² are identical to or different from each other, and each represent a hydrogen atom or a halogen atom.
[Aspect 240] The compound according to the Aspect 9, wherein R¹ and R² are identical to or different from each other, and each represent a hydrogen atom or a halogen atom.
[Aspect 241] The compound according to the Aspect 10, wherein R¹ and R² are identical to or different from each other, and each represent a hydrogen atom or a halogen atom.
[Aspect 242] The compound according to the Aspect 11, wherein R¹ and R² are identical to or different from each other, and each represent a hydrogen atom or a halogen atom.
[Aspect 243] The compound according to the Aspect 12, wherein R¹ and R² are identical to or different from each other, and each represent a hydrogen atom or a halogen atom.
[Aspect 244] The compound according to the Aspect 13, wherein R¹ and R² are identical to or different from each other, and each represent a hydrogen atom or a halogen atom.
[Aspect 245] The compound according to the Aspect 14, wherein R¹ and R² are identical to or different from each other, and each represent a hydrogen atom or a halogen atom.
[Aspect 246] The compound according to the Aspect 15, wherein R¹ and R² are identical to or different from each other, and each represent a hydrogen atom or a halogen atom.
[Aspect 247] The compound according to the Aspect 16, wherein R¹ and R² are identical to or different from each other, and each represent a hydrogen atom or a halogen atom.
[Aspect 248] The compound according to the Aspect 17, wherein R¹ and R² are identical to or different from each other, and each represent a hydrogen atom or a halogen atom.
[Aspect 249] The compound according to the Aspect 18, wherein R¹ and R² are identical to or different from each other, and each represent a hydrogen atom or a halogen atom.
[Aspect 250] The compound according to the Aspect 19, wherein R¹ and R² are identical to or different from each other, and each represent a hydrogen atom or a halogen atom.
[Aspect 251] The compound according to the Aspect 20, wherein R¹ and R² are identical to or different from each other, and each represent a hydrogen atom or a halogen atom.
[Aspect 252] The compound according to the Aspect 21, wherein R¹ and R² are identical to or different from each other, and each represent a hydrogen atom or a halogen atom.
[Aspect 253] The compound according to the Aspect 22, wherein R¹ and R² are identical to or different from each other, and each represent a hydrogen atom or a halogen atom.
[Aspect 254] The compound according to the Aspect 23, wherein R¹ and R² are identical to or different from each other, and each represent a hydrogen atom or a halogen atom.
[Aspect 255] The compound according to the Aspect 24, wherein R¹ and R² are identical to or different from each other, and each represent a hydrogen atom or a halogen atom.
[Aspect 256] The compound according to the Aspect 25, wherein R¹ and R² are identical to or different from each other, and each represent a hydrogen atom or a halogen atom.
[Aspect 257] The compound according to the Aspect 26, wherein R¹ and R² are identical to or different from each other, and each represent a hydrogen atom or a halogen atom.
[Aspect 258] The compound according to the Aspect 27, wherein R¹ and R² are identical to or different from each other, and each represent a hydrogen atom or a halogen atom.
[Aspect 259] The compound according to the Aspect 28, wherein R¹ and R² are identical to or different from each other, and each represent a hydrogen atom or a halogen atom.
[Aspect 260] The compound according to the Aspect 29, wherein R¹ and R² are identical to or different from each other, and each represent a hydrogen atom or a halogen atom.
[Aspect 261] The compound according to the Aspect 30, wherein R¹ and R² are identical to or different from each other, and each represent a hydrogen atom or a halogen atom.
[Aspect 262] The compound according to the Aspect 31, wherein R¹ and R² are identical to or different from each other, and each represent a hydrogen atom or a halogen atom.
[Aspect 263] The compound according to the Aspect 32, wherein R¹ and R² are identical to or different from each other, and each represent a hydrogen atom or a halogen atom.
[Aspect 264] The compound according to the Aspect 33, wherein R¹ and R² are identical to or different from each other, and each represent a hydrogen atom or a halogen atom.
[Aspect 265] The compound according to the Aspect 34, wherein R¹ and R² are identical to or different from each other, and each represent a hydrogen atom or a halogen atom.
[Aspect 266] The compound according to the Aspect 35, wherein R¹ and R² are identical to or different from each other, and each represent a hydrogen atom or a halogen atom.
[Aspect 267] The compound according to the Aspect 36, wherein R¹ and R² are identical to or different from each other, and each represent a hydrogen atom or a halogen atom.
[Aspect 268] The compound according to the Aspect 37, wherein R¹ and R² are identical to or different from each other, and each represent a hydrogen atom or a halogen atom.
[Aspect 269] The compound according to the Aspect 38, wherein R¹ and R² are identical to or different from each other, and each represent a hydrogen atom or a halogen atom.
[Aspect 270] The compound according to the Aspect 39, wherein R¹ and R² are identical to or different from each other, and each represent a hydrogen atom or a halogen atom.
[Aspect 271] The compound according to the Aspect 40, wherein R¹ and R² are identical to or different from each other, and each represent a hydrogen atom or a halogen atom.
[Aspect 272] The compound according to the Aspect 41, wherein R¹ and R² are identical to or different from each other, and each represent a hydrogen atom or a halogen atom.
[Aspect 273] The compound according to the Aspect 42, wherein R¹ and R² are identical to or different from each other, and each represent a hydrogen atom or a halogen atom.
[Aspect 274] The compound according to the Aspect 43, wherein R¹ and R² are identical to or different from each other, and each represent a hydrogen atom or a halogen atom.
[Aspect 275] The compound according to the Aspect 44, wherein R¹ and R² are identical to or different from each other, and each represent a hydrogen atom or a halogen atom.
[Aspect 276] The compound according to the Aspect 45, wherein R¹ and R² are identical to or different from each other, and each represent a hydrogen atom or a halogen atom.
[Aspect 277] The compound according to the Aspect 46, wherein R¹ and R² are identical to or different from each other, and each represent a hydrogen atom or a halogen atom.
[Aspect 278] The compound according to the Aspect 47, wherein R¹ and R² are identical to or different from each other, and each represent a hydrogen atom or a halogen atom.
[Aspect 279] The compound according to the Aspect 48, wherein R¹ and R² are identical to or different from each other, and each represent a hydrogen atom or a halogen atom.
[Aspect 280] The compound according to the Aspect 49, wherein R¹ and R² are identical to or different from each other, and each represent a hydrogen atom or a halogen atom.
[Aspect 281] The compound according to the Aspect 50, wherein R¹ and R² are identical to or different from each other, and each represent a hydrogen atom or a halogen atom.
[Aspect 282] The compound according to the Aspect 51, wherein R¹ and R² are identical to or different from each other, and each represent a hydrogen atom or a halogen atom.
[Aspect 283] The compound according to the Aspect 52, wherein R¹ and R² are identical to or different from each other, and each represent a hydrogen atom or a halogen atom.
[Aspect 284] The compound according to the Aspect 53, wherein R¹ and R² are identical to or different from each other, and each represent a hydrogen atom or a halogen atom.
[Aspect 285] The compound according to the Aspect 54, wherein R¹ and R² are identical to or different from each other, and each represent a hydrogen atom or a halogen atom.
[Aspect 286] The compound according to the Aspect 55, wherein R¹ and R² are identical to or different from each other, and each represent a hydrogen atom or a halogen atom.
[Aspect 287] The compound according to the Aspect 56, wherein R¹ and R² are identical to or different from each other, and each represent a hydrogen atom or a halogen atom.
[Aspect 288] The compound according to the Aspect 57, wherein R¹ and R² are identical to or different from each other, and each represent a hydrogen atom or a halogen atom.
[Aspect 289] The compound according to the Aspect 58, wherein R¹ and R² are identical to or different from each other, and each represent a hydrogen atom or a halogen atom.
[Aspect 290] The compound according to the Aspect 59, wherein R¹ and R² are identical to or different from each other, and each represent a hydrogen atom or a halogen atom.
[Aspect 291] The compound according to the Aspect 60, wherein R¹ and R² are identical to or different from each other, and each represent a hydrogen atom or a halogen atom.
[Aspect 292] The compound according to the Aspect 61, wherein R¹ and R² are identical to or different from each other, and each represent a hydrogen atom or a halogen atom.
[Aspect 293] The compound according to the Aspect 62, wherein R¹ and R² are identical to or different from each other, and each represent a hydrogen atom or a halogen atom.
[Aspect 294] The compound according to the Aspect 63, wherein R¹ and R² are identical to or different from each other, and each represent a hydrogen atom or a halogen atom.
[Aspect 295] The compound according to the Aspect 64, wherein R¹ and R² are identical to or different from each other, and each represent a hydrogen atom or a halogen atom.
[Aspect 296] The compound according to the Aspect 65, wherein R¹ and R² are identical to or different from each other, and each represent a hydrogen atom or a halogen atom.
[Aspect 297] The compound according to the Aspect 66, wherein R¹ and R² are identical to or different from each other, and each represent a hydrogen atom or a halogen atom.
[Aspect 298] The compound according to the Aspect 67, wherein R¹ and R² are identical to or different from each other, and each represent a hydrogen atom or a halogen atom.
[Aspect 299] The compound according to the Aspect 68, wherein R¹ and R² are identical to or different from each other, and each represent a hydrogen atom or a halogen atom.
[Aspect 300] The compound according to the Aspect 69, wherein R¹ and R² are identical to or different from each other, and each represent a hydrogen atom or a halogen atom.
[Aspect 301] The compound according to the Aspect 70, wherein R¹ and R² are identical to or different from each other, and each represent a hydrogen atom or a halogen atom.
[Aspect 302] The compound according to the Aspect 71, wherein R¹ and R² are identical to or different from each other, and each represent a hydrogen atom or a halogen atom.
[Aspect 303] The compound according to the Aspect 72, wherein R¹ and R² are identical to or different from each other, and each represent a hydrogen atom or a halogen atom.
[Aspect 304] The compound according to the Aspect 73, wherein R¹ and R² are identical to or different from each other, and each represent a hydrogen atom or a halogen atom.
[Aspect 305] The compound according to the Aspect 74, wherein R¹ and R² are identical to or different from each other, and each represent a hydrogen atom or a halogen atom.
[Aspect 306] The compound according to the Aspect 75, wherein R¹ and R² are identical to or different from each other, and each represent a hydrogen atom or a halogen atom.
[Aspect 307] The compound according to the Aspect 76, wherein R¹ and R² are identical to or different from each other, and each represent a hydrogen atom or a halogen atom.
[Aspect 308] The Present compound N or the compound according to any one of the Aspects 1 to 307, wherein Z represents a phenyl group optionally substituted with one or more substituent(s) selected from Group D.
[Aspect 309] The Present compound N or the compound according to any one of the Aspects 1 to 307, wherein Z represents a 5-6 membered aromatic heterocyclic group optionally substituted with one or more substituent(s) selected from Group D.
[Aspect 310] The Present compound N or the compound according to any one of the Aspects 1 to 307, wherein Z represents a 5 membered aromatic heterocyclic group optionally substituted with one or more substituent(s) selected from Group D.
[Aspect 311] The Present compound N or the compound according to any one of the Aspects 1 to 307, wherein Z represents a 6 membered aromatic heterocyclic group optionally substituted with one or more substituent(s) selected from Group D.
[Aspect 312] The Present compound N or the compound according to any one of the Aspects 1 to 307, wherein Z represents a phenyl group optionally substituted with one or more substituent(s) selected from Group D2;
   Group D2: a group consisting of a C1-C3 alkyl group, a halogen atom, and a cyano group.
[Aspect 313] The Present compound N or the compound according to any one of the Aspects 1 to 307, wherein Z represents a 5-6 membered aromatic heterocyclic group optionally substituted with one or more substituent(s) selected from Group D2.
[Aspect 314] The Present compound N or the compound according to any one of the Aspects 1 to 307, wherein Z represents a 5 membered aromatic heterocyclic group optionally substituted with one or more substituent(s) selected from Group D2.
[Aspect 315] The Present compound N or the compound according to any one of the Aspects 1 to 307, wherein Z represents a 6 membered aromatic heterocyclic group optionally substituted with one or more substituent(s) selected from Group D2.
[Aspect 316] The Present compound N or the compound according to any one of the Aspects 1 to 307, wherein Z represents a 5-6 membered aromatic heterocyclic group {provided that the atom in said 5-6 membered aromatic heterocyclic group attached to the sulfonyl group is a carbon atom. Also, said 5-6 membered aromatic heterocyclic group may be optionally substituted with one or more substituent(s) selected from Group D}.
[Aspect 317] The Present compound N or the compound according to any one of the Aspects 1 to 307, wherein Z represents a phenyl group or a 5-6 membered aromatic heterocyclic group {wherein said phenyl group and said 5-6 membered aromatic heterocyclic group may be optionally substituted with one or more substituent(s) selected from Group D}.
[Aspect 318] The Present compound N or the compound according to any one of the Aspects 1 to 307, wherein Z represents a phenyl group or a 5-6 membered aromatic heterocyclic group {wherein said phenyl group and said 5-6 membered aromatic heterocyclic group may be optionally substituted with one or more substituent(s) selected from Group D2}.
[Aspect 319] The Present compound N or the compound according to any one of the Aspects 1 to 307, wherein Z represents a phenyl group optionally substituted with one or more substituent(s) selected from Group D2 or a 5-6 membered aromatic heterocyclic group {provided that the atom in said 5-6 membered aromatic heterocyclic group attached to the sulfonyl group is a carbon atom. Also, said 5-6 membered aromatic heterocyclic group may be optionally substituted with one or more substituent(s) selected from Group D2}.
[Aspect 320] The Present compound N or the compound according to any one of the Aspects 1 to 307, wherein Z represents a 5 membered aromatic heterocyclic group {provided that the atom in said 5 membered aromatic heterocyclic group attached to the sulfonyl group is a carbon atom. Also, said 5 membered aromatic heterocyclic group may be optionally substituted with one or more substituent(s) selected from Group D}.
[Aspect 321] The Present compound N or the compound according to any one of the Aspects 1 to 307, wherein Z represents a 6 membered aromatic heterocyclic group {provided that the atom in said 6 membered aromatic heterocyclic group attached to the sulfonyl group is a carbon atom. Also, said 6 membered aromatic heterocyclic group may be optionally substituted with one or more substituent(s) selected from Group D}.
[Aspect 322] The Present compound N or the compound according to any one of the Aspects 1 to 307, wherein Z represents a 5 membered aromatic heterocyclic group {provided that the atom in said 5 membered aromatic heterocyclic group attached to the sulfonyl group is a carbon atom. Also, said 5 membered aromatic heterocyclic group may be optionally substituted with one or more substituent(s) selected from Group D2}.
[Aspect 323] The Present compound N or the compound according to any one of the Aspects 1 to 307, wherein Z represents a 6 membered aromatic heterocyclic group {provided that the atom in said 6 membered aromatic heterocyclic group attached to the sulfonyl group is a carbon atom. Also, said 6 membered aromatic heterocyclic group may be optionally substituted with one or more substituent(s) selected from Group D2}.
[Aspect 324] A seed or a vegetative reproductive organ holding an effective amount of the Present compound N or an N-oxide thereof, or a salt thereof.

Aspects of the Compound N of the present invention include the following compounds.
[Aspect C1] The Compound N of the present invention, wherein R^{3a} represents a C1-C6 chain hydrocarbon group optionally substituted with one or more substituent(s) selected from Group A^{a} or a C3-C8 alicyclic hydrocarbon group optionally substituted with one or more halogen atom(s).
[Aspect C2] The Compound N of the present invention, wherein R^{3a} represents a halogen atom.
[Aspect C3] The Compound N of the present invention, wherein
   R^{3a} represents a nitro group, NR^{8a}R^{9a}, or C(O)NR^{4a}R^{5a}; and
   R^{4a}, R^{5a}, R^{8a}, and R^{9a} are identical to or different from each other, and each represent a C1-C3 alkyl group or a hydrogen atom.
[Aspect C4] The Compound N of the present invention, wherein
   R^{3a} represents a C1-C6 chain hydrocarbon group optionally substituted with one or more substituent(s) selected from Group A^{a}, a C3-C8 alicyclic hydrocarbon group optionally substituted with one or more halogen atom(s), a halogen atom, a nitro group, NR^{8a}R^{9a}, or C(O)NR^{4a}R^{5a}; and
   R^{4a}, R^{5a}, R^{8a}, and R^{9a} are identical to or different from each other, and each represent a C1-C3 alkyl group or a hydrogen atom.
[Aspect C5] The Compound N of the present invention, wherein adjacent R^{1a} and R^{3a} are combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B^{a}}, a benzene ring, or a 5-7 membered aromatic heterocyclic ring {wherein said benzene ring and said 5-7 membered aromatic heterocyclic ring may be optionally substituted with one or more substituent(s) selected from Group E^{a}}.
[Aspect C6] The Compound N of the present invention, wherein adjacent R^{1a} and R^{3a} are combined with the carbon atoms to which they are attached to form a C5-C6 alicyclic hydrocarbon or a 5-6 membered nonaromatic heterocyclic ring {wherein said C5-C6 alicyclic hydrocarbon and said 5-6 membered nonaromatic heterocyclic ring may be optionally substituted with one or more substituent(s) selected from Group B2^{a}};
   Group B2^{a}: a group consisting of a C1-C6 chain hydrocarbon group optionally substituted with one or more halogen atom(s), and a halogen atom.
[Aspect C7] The Compound N of the present invention, wherein adjacent R^{1a} and R^{3a} are combined with the carbon atoms to which they are attached to form a benzene ring or a 5-6 membered aromatic heterocyclic ring {wherein said benzene ring and said 5-6 membered aromatic heterocyclic ring may be optionally substituted with one or more substituent(s) selected from Group B2^{a}}.
[Aspect C8] The Compound N of the present invention, wherein adjacent two R^{3a} and R^{3a} are combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B^{a}}, a benzene ring, or a 5-7 membered aromatic heterocyclic ring {wherein said benzene ring and said 5-7 membered aromatic heterocyclic ring may be optionally substituted with one or more substituent(s) selected from Group E^{a}}.
[Aspect C9] The Compound N of the present invention, wherein adjacent two R^{3a} and R^{3a} are combined with the carbon atoms to which they are attached to form a C5-C6 alicyclic hydrocarbon or a 5-6 membered nonaromatic heterocyclic ring {wherein said C5-C6 alicyclic hydrocarbon and said 5-6 membered nonaromatic heterocyclic ring may be optionally substituted with one or more substituent(s) selected from Group B2^{a}}.
[Aspect C10] The Compound N of the present invention, wherein adjacent two R^{3a} and R^{3a} are combined with the carbon atoms to which they are attached to form a benzene ring or a 5-6 membered aromatic heterocyclic ring {wherein said benzene ring and said 5-6 membered aromatic heterocyclic ring may be optionally substituted with one or more substituent(s) selected from Group B2^{a}}.
[Aspect C11] The Compound N of the present invention, wherein adjacent two R^{3a} and R^{3a} are combined with the carbon atoms to which they are attached to form a C5-C6 alicyclic hydrocarbon, a 5-6 membered nonaromatic heterocyclic ring, a benzene ring, or a 5-6 membered aromatic heterocyclic ring {wherein said C5-C6 alicyclic hydrocarbon, said 5-6 membered nonaromatic heterocyclic ring, said benzene ring, and said 5-6 membered aromatic heterocyclic ring may be optionally substituted with one or more substituent(s) selected from Group B2^{a}}.
[Aspect C12] The Compound N of the present invention, wherein
   R^{3a} represents a C1-C6 chain hydrocarbon group optionally substituted with one or more substituent(s) selected from Group A^{a}, a C1-C3 alkoxy group, a C3-C8 alicyclic hydrocarbon group {wherein said C1-C3 alkoxy group and said C3-C8 alicyclic hydrocarbon group may be optionally substituted with one or more halogen atom(s)}, a halogen atom, a nitro group, NR^{8a}R^{9a}, or C(O)NR^{4a}R^{5a}; and
   R^{4a}, R^{5a}, R^{8a}, and R^{9a} are identical to or different from each other, and each represent a C1-C3 alkyl group, a cyclopropyl group, or a hydrogen atom.
[Aspect C13] The Compound N of the present invention, wherein
   R^{3a} represents a C1-C3 chain hydrocarbon group, a C1-C3 alkoxy group {wherein said C1-C3 chain hydrocarbon group and said C1-C3 alkoxy group may be optionally substituted with one or more halogen atom(s)}, a cyclopropyl group, a halogen atom, a nitro group, NR^{8a}R^{9a}, or C(O)NR^{4a}R^{5a}; and
   R^{4a}, R^{5a}, R^{8a}, and R^{9a} are identical to or different from each other, and each represent a C1-C3 alkyl group, a cyclopropyl group, or a hydrogen atom.
[Aspect C14] The Compound N of the present invention, wherein
   R^{3a} represents a C1-C6 chain hydrocarbon group optionally substituted with one or more substituent(s) selected from Group A^{a}, a C1-C3 alkoxy group, a C3-C8 alicyclic hydrocarbon group {wherein said C1-C3 alkoxy group and said C3-C8 alicyclic hydrocarbon group may be optionally substituted with one or more halogen atom(s)}, a halogen atom, a nitro group, NR^{8a}R^{9a}, or C(O)NR^{4a}R^{5a}; and
   R^{4a}, R^{5a}, R^{8a}, and R^{9a} are identical to or different from each other, and each represent a hydrogen atom, a cyclopropyl group, or a C1-C3 alkyl group;
   or adjacent two R^{3a} and R^{3a} may be optionally combined with the carbon atoms to which they are attached to form a C5-C6 alicyclic hydrocarbon, a 5-6 membered nonaromatic heterocyclic ring, a benzene ring, or a 5-6 membered aromatic heterocyclic ring {wherein said C5-C6 alicyclic hydrocarbon, said 5-6 membered nonaromatic heterocyclic ring, said benzene ring, and said 5-6 membered aromatic heterocyclic ring may be optionally substituted with one or more substituent(s) selected from Group B2^{a}}.
[Aspect C15] The Compound N of the present invention, wherein
   R^{3a} represents a C1-C3 chain hydrocarbon group, a C1-C3 alkoxy group {wherein said C1-C3 chain hydrocarbon group and said C1-C3 alkoxy group may be optionally substituted with one or more halogen atom(s)}, a cyclopropyl group, a halogen atom, a nitro group, NR^{8a}R^{9a}, or C(O)NR^{4a}R^{5a}; and
   R^{4a}, R^{5a}, R^{8a}, and R^{9a} are identical to or different from each other, and each represent a C1-C3 alkyl group, a cyclopropyl group, or a hydrogen atom;
   or adjacent two R^{3a} and R^{3a} may be optionally combined with the carbon atoms to which they are attached to form a C5-C6 alicyclic hydrocarbon, a 5-6 membered nonaromatic heterocyclic ring, or a 5-6 membered aromatic heterocyclic ring {wherein said C5-C6 alicyclic hydrocarbon, said 5-6 membered nonaromatic heterocyclic ring, and said 5-6 membered aromatic heterocyclic ring may be optionally substituted with one or more substituent(s) selected from Group B2^{a}}.
[Aspect C16] The Compound N of the present invention, wherein q represents 1.
[Aspect C17] The Compound N of the present invention, wherein q represents 2.
[Aspect C18] The Compound N of the present invention, wherein q represents 3.
[Aspect C19] The Compound N of the present invention, wherein q represents 1 or 2.
[Aspect C20] The compound according to the Aspect C1, wherein q represents 1.
[Aspect C21] The compound according to the Aspect C1, wherein q represents 2.
[Aspect C22] The compound according to the Aspect C1, wherein q represents 3.
[Aspect C23] The compound according to the Aspect C1, wherein q represents 1 or 2.
[Aspect C24] The compound according to the Aspect C2, wherein q represents 1.
[Aspect C25] The compound according to the Aspect C2, wherein q represents 2.
[Aspect C26] The compound according to the Aspect C2, wherein q represents 3.
[Aspect C27] The compound according to the Aspect C2, wherein q represents 1 or 2.
[Aspect C28] The compound according to the Aspect C3, wherein q represents 1.
[Aspect C29] The compound according to the Aspect C3, wherein q represents 2.
[Aspect C30] The compound according to the Aspect C3, wherein q represents 3.
[Aspect C31] The compound according to the Aspect C3, wherein q represents 1 or 2.
[Aspect C32] The compound according to the Aspect C4, wherein q represents 1.
[Aspect C33] The compound according to the Aspect C4, wherein q represents 2.
[Aspect C34] The compound according to the Aspect C4, wherein q represents 3.
[Aspect C35] The compound according to the Aspect C4, wherein q represents 1 or 2.
[Aspect C36] The compound according to the Aspect C5, wherein q represents 1.
[Aspect C37] The compound according to the Aspect C5, wherein q represents 2.
[Aspect C38] The compound according to the Aspect C5, wherein q represents 3.
[Aspect C39] The compound according to the Aspect C5, wherein q represents 1 or 2.
[Aspect C40] The compound according to the Aspect C6, wherein q represents 1.
[Aspect C41] The compound according to the Aspect C6, wherein q represents 2.
[Aspect C42] The compound according to the Aspect C6, wherein q represents 3.
[Aspect C43] The compound according to the Aspect C6, wherein q represents 1 or 2.
[Aspect C44] The compound according to the Aspect C7, wherein q represents 1.
[Aspect C45] The compound according to the Aspect C7, wherein q represents 2.
[Aspect C46] The compound according to the Aspect C7, wherein q represents 3.
[Aspect C47] The compound according to the Aspect C7, wherein q represents 1 or 2.
[Aspect C48] The compound according to the Aspect C8, wherein q represents 2.
[Aspect C49] The compound according to the Aspect C8, wherein q represents 3.
[Aspect C50] The compound according to the Aspect C8, wherein q represents 2 or 3.
[Aspect C51] The compound according to the Aspect C9, wherein q represents 2.
[Aspect C52] The compound according to the Aspect C9, wherein q represents 3.
[Aspect C53] The compound according to the Aspect C9, wherein q represents 2 or 3.
[Aspect C54] The compound according to the Aspect C10, wherein q represents 2.
[Aspect C55] The compound according to the Aspect C10, wherein q represents 3.
[Aspect C56] The compound according to the Aspect C10, wherein q represents 2 or 3.
[Aspect C57] The compound according to the Aspect C11, wherein q represents 2.
[Aspect C58] The compound according to the Aspect C11, wherein q represents 3.
[Aspect C59] The compound according to the Aspect C11, wherein q represents 2 or 3.
[Aspect C60] The compound according to the Aspect C12, wherein q represents 1.
[Aspect C61] The compound according to the Aspect C12, wherein q represents 2.
[Aspect C62] The compound according to the Aspect C12, wherein q represents 3.
[Aspect C63] The compound according to the Aspect C12, wherein q represents 1 or 2.
[Aspect C64] The compound according to the Aspect C13, wherein q represents 1.
[Aspect C65] The compound according to the Aspect C13, wherein q represents 2.
[Aspect C66] The compound according to the Aspect C13, wherein q represents 3.
[Aspect C67] The compound according to the Aspect C13, wherein q represents 1 or 2.
[Aspect C68] The compound according to the Aspect C14, wherein q represents 1.
[Aspect C69] The compound according to the Aspect C14, wherein q represents 2.
[Aspect C70] The compound according to the Aspect C14, wherein q represents 3.
[Aspect C71] The compound according to the Aspect C14, wherein q represents 1 or 2.
[Aspect C72] The compound according to the Aspect C15, wherein q represents 1.
[Aspect C73] The compound according to the Aspect C15, wherein q represents 2.
[Aspect C74] The compound according to the Aspect C15, wherein q represents 3.
[Aspect C75] The compound according to the Aspect C15, wherein q represents 1 or 2.
[Aspect C76] The Compound N of the present invention, wherein R¹⁵ and R^{2a} are identical to or different from each other, and each represent a C1-C3 alkyl group or a hydrogen atom.
[Aspect C77] The compound according to the Aspect C1, wherein R^{1a} and R^{2a} are identical to or different from each other, and each represent a C1-C3 alkyl group or a hydrogen atom.
[Aspect C78] The compound according to the Aspect C2, wherein R^{1a} and R^{2a} are identical to or different from each other, and each represent a C1-C3 alkyl group or a hydrogen atom.
[Aspect C79] The compound according to the Aspect C3, wherein R^{1a} and R^{2a} are identical to or different from each other, and each represent a C1-C3 alkyl group or a hydrogen atom.
[Aspect C80] The compound according to the Aspect C4, wherein R^{1a} and R^{2a} are identical to or different from each other, and each represent a C1-C3 alkyl group or a hydrogen atom.
[Aspect C81] The compound according to the Aspect C5, wherein R^{1a} and R^{2a} are identical to or different from each other, and each represent a C1-C3 alkyl group or a hydrogen atom.
[Aspect C82] The compound according to the Aspect C6, wherein R^{1a} and R^{2a} are identical to or different from each other, and each represent a C1-C3 alkyl group or a hydrogen atom.
[Aspect C83] The compound according to the Aspect C7, wherein R^{1a} and R^{2a} are identical to or different from each other, and each represent a C1-C3 alkyl group or a hydrogen atom.
[Aspect C84] The compound according to the Aspect C8, wherein R^{1a} and R^{2a} are identical to or different from each other, and each represent a C1-C3 alkyl group or a hydrogen atom.
[Aspect C85] The compound according to the Aspect C9, wherein R^{1a} and R^{2a} are identical to or different from each other, and each represent a C1-C3 alkyl group or a hydrogen atom.
[Aspect C86] The compound according to the Aspect C10, wherein R^{1a} and R^{2a} are identical to or different from each other, and each represent a C1-C3 alkyl group or a hydrogen atom.
[Aspect C87] The compound according to the Aspect C11, wherein R^{1a} and R^{2a} are identical to or different from each other, and each represent a C1-C3 alkyl group or a hydrogen atom.
[Aspect C88] The compound according to the Aspect C12, wherein R^{1a} and R^{2a} are identical to or different from each other, and each represent a C1-C3 alkyl group or a hydrogen atom.
[Aspect C89] The compound according to the Aspect C13, wherein R^{1a} and R^{2a} are identical to or different from each other, and each represent a C1-C3 alkyl group or a hydrogen atom.
[Aspect C90] The compound according to the Aspect C14, wherein R^{1a} and R^{2a} are identical to or different from each other, and each represent a C1-C3 alkyl group or a hydrogen atom.
[Aspect C91] The compound according to the Aspect C15, wherein R^{1a} and R^{2a} are identical to or different from each other, and each represent a C1-C3 alkyl group or a hydrogen atom.
[Aspect C92] The compound according to the Aspect C16, wherein R^{1a} and R^{2a} are identical to or different from each other, and each represent a C1-C3 alkyl group or a hydrogen atom.
[Aspect C93] The compound according to the Aspect C17, wherein R^{1a} and R^{2a} are identical to or different from each other, and each represent a C1-C3 alkyl group or a hydrogen atom.
[Aspect C94] The compound according to the Aspect C18, wherein R^{1a} and R^{2a} are identical to or different from each other, and each represent a C1-C3 alkyl group or a hydrogen atom.
[Aspect C95] The compound according to the Aspect C19, wherein R^{1a} and R^{2a} are identical to or different from each other, and each represent a C1-C3 alkyl group or a hydrogen atom.
[Aspect C96] The compound according to the Aspect C20, wherein R^{1a} and R^{2a} are identical to or different from each other, and each represent a C1-C3 alkyl group or a hydrogen atom.
[Aspect C97] The compound according to the Aspect C21, wherein R^{1a} and R^{2a} are identical to or different from each other, and each represent a C1-C3 alkyl group or a hydrogen atom.
[Aspect C98] The compound according to the Aspect C22, wherein R^{1a} and R^{2a} are identical to or different from each other, and each represent a C1-C3 alkyl group or a hydrogen atom.
[Aspect C99] The compound according to the Aspect C23, wherein R^{1a} and R^{2a} are identical to or different from each other, and each represent a C1-C3 alkyl group or a hydrogen atom.
[Aspect C100] The compound according to the Aspect C24, wherein R^{1a} and R^{2a} are identical to or different from each other, and each represent a C1-C3 alkyl group or a hydrogen atom.
[Aspect C101] The compound according to the Aspect C25, wherein R^{1a} and R^{2a} are identical to or different from each other, and each represent a C1-C3 alkyl group or a hydrogen atom.
[Aspect C102] The compound according to the Aspect C26, wherein R^{1a} and R^{2a} are identical to or different from each other, and each represent a C1-C3 alkyl group or a hydrogen atom.
[Aspect C103] The compound according to the Aspect C27, wherein R^{1a} and R^{2a} are identical to or different from each other, and each represent a C1-C3 alkyl group or a hydrogen atom.
[Aspect C104] The compound according to the Aspect C28, wherein R^{1a} and R^{2a} are identical to or different from each other, and each represent a C1-C3 alkyl group or a hydrogen atom.
[Aspect C105] The compound according to the Aspect C29, wherein R^{1a} and R^{2a} are identical to or different from each other, and each represent a C1-C3 alkyl group or a hydrogen atom.
[Aspect C106] The compound according to the Aspect C30, wherein R^{1a} and R^{2a} are identical to or different from each other, and each represent a C1-C3 alkyl group or a hydrogen atom.
[Aspect C107] The compound according to the Aspect C31, wherein R^{1a} and R^{2a} are identical to or different from each other, and each represent a C1-C3 alkyl group or a hydrogen atom.
[Aspect C108] The compound according to the Aspect C32, wherein R^{1a} and R^{2a} are identical to or different from each other, and each represent a C1-C3 alkyl group or a hydrogen atom.
[Aspect C109] The compound according to the Aspect C33, wherein R^{1a} and R^{2a} are identical to or different from each other, and each represent a C1-C3 alkyl group or a hydrogen atom.
[Aspect C110] The compound according to the Aspect C34, wherein R^{1a} and R^{2a} are identical to or different from each other, and each represent a C1-C3 alkyl group or a hydrogen atom.
[Aspect C111] The compound according to the Aspect C35, wherein R^{1a} and R^{2a} are identical to or different from each other, and each represent a C1-C3 alkyl group or a hydrogen atom.
[Aspect C112] The compound according to the Aspect C36, wherein R^{1a} and R^{2a} are identical to or different from each other, and each represent a C1-C3 alkyl group or a hydrogen atom.
[Aspect C113] The compound according to the Aspect C37, wherein R^{1a} and R^{2a} are identical to or different from each other, and each represent a C1-C3 alkyl group or a hydrogen atom.
[Aspect C114] The compound according to the Aspect C38, wherein R^{1a} and R^{2a} are identical to or different from each other, and each represent a C1-C3 alkyl group or a hydrogen atom.
[Aspect C115] The compound according to the Aspect C39, wherein R^{1a} and R^{2a} are identical to or different from each other, and each represent a C1-C3 alkyl group or a hydrogen atom.
[Aspect C116] The compound according to the Aspect C40, wherein R^{1a} and R^{2a} are identical to or different from each other, and each represent a C1-C3 alkyl group or a hydrogen atom.
[Aspect C117] The compound according to the Aspect C41, wherein R^{1a} and R^{2a} are identical to or different from each other, and each represent a C1-C3 alkyl group or a hydrogen atom.
[Aspect C118] The compound according to the Aspect C42, wherein R^{1a} and R^{2a} are identical to or different from each other, and each represent a C1-C3 alkyl group or a hydrogen atom.
[Aspect C119] The compound according to the Aspect C43, wherein R^{1a} and R^{2a} are identical to or different from each other, and each represent a C1-C3 alkyl group or a hydrogen atom.
[Aspect C120] The compound according to the Aspect C44, wherein R^{1a} and R^{2a} are identical to or different from each other, and each represent a C1-C3 alkyl group or a hydrogen atom.
[Aspect C121] The compound according to the Aspect C45, wherein R^{1a} and R^{2a} are identical to or different from each other, and each represent a C1-C3 alkyl group or a hydrogen atom.
[Aspect C122] The compound according to the Aspect C46, wherein R^{1a} and R^{2a} are identical to or different from each other, and each represent a C1-C3 alkyl group or a hydrogen atom.
[Aspect C123] The compound according to the Aspect C47, wherein R^{1a} and R^{2a} are identical to or different from each other, and each represent a C1-C3 alkyl group or a hydrogen atom.
[Aspect C124] The compound according to the Aspect C48, wherein R^{1a} and R^{2a} are identical to or different from each other, and each represent a C1-C3 alkyl group or a hydrogen atom.
[Aspect C125] The compound according to the Aspect C49, wherein R^{1a} and R^{2a} are identical to or different from each other, and each represent a C1-C3 alkyl group or a hydrogen atom.
[Aspect C126] The compound according to the Aspect C50, wherein R^{1a} and R^{2a} are identical to or different from each other, and each represent a C1-C3 alkyl group or a hydrogen atom.
[Aspect C127] The compound according to the Aspect C51, wherein R^{1a} and R^{2a} are identical to or different from each other, and each represent a C1-C3 alkyl group or a hydrogen atom.
[Aspect C128] The compound according to the Aspect C52, wherein R^{1a} and R^{2a} are identical to or different from each other, and each represent a C1-C3 alkyl group or a hydrogen atom.
[Aspect C129] The compound according to the Aspect C53, wherein R^{1a} and R^{2a} are identical to or different from each other, and each represent a C1-C3 alkyl group or a hydrogen atom.
[Aspect C130] The compound according to the Aspect C54, wherein R^{1a} and R^{2a} are identical to or different from each other, and each represent a C1-C3 alkyl group or a hydrogen atom.
[Aspect C131] The compound according to the Aspect C55, wherein R^{1a} and R^{2a} are identical to or different from each other, and each represent a C1-C3 alkyl group or a hydrogen atom.
[Aspect C132] The compound according to the Aspect C56, wherein R^{1a} and R^{2a} are identical to or different from each other, and each represent a C1-C3 alkyl group or a hydrogen atom.
[Aspect C133] The compound according to the Aspect C57, wherein R^{1a} and R^{2a} are identical to or different from each other, and each represent a C1-C3 alkyl group or a hydrogen atom.
[Aspect C134] The compound according to the Aspect C58, wherein R^{1a} and R^{2a} are identical to or different from each other, and each represent a C1-C3 alkyl group or a hydrogen atom.
[Aspect C135] The compound according to the Aspect C59, wherein R^{1a} and R^{2a} are identical to or different from each other, and each represent a C1-C3 alkyl group or a hydrogen atom.
[Aspect C136] The compound according to the Aspect C60, wherein R^{1a} and R^{2a} are identical to or different from each other, and each represent a C1-C3 alkyl group or a hydrogen atom.
[Aspect C137] The compound according to the Aspect C61, wherein R^{1a} and R^{2a} are identical to or different from each other, and each represent a C1-C3 alkyl group or a hydrogen atom.
[Aspect C138] The compound according to the Aspect C62, wherein R^{1a} and R^{2a} are identical to or different from each other, and each represent a C1-C3 alkyl group or a hydrogen atom.
[Aspect C139] The compound according to the Aspect C63, wherein R^{1a} and R^{2a} are identical to or different from each other, and each represent a C1-C3 alkyl group or a hydrogen atom.
[Aspect C140] The compound according to the Aspect C64, wherein R^{1a} and R^{2a} are identical to or different from each other, and each represent a C1-C3 alkyl group or a hydrogen atom.
[Aspect C141] The compound according to the Aspect C65, wherein R^{1a} and R^{2a} are identical to or different from each other, and each represent a C1-C3 alkyl group or a hydrogen atom.
[Aspect C142] The compound according to the Aspect C66, wherein R^{1a} and R^{2a} are identical to or different from each other, and each represent a C1-C3 alkyl group or a hydrogen atom.
[Aspect C143] The compound according to the Aspect C67, wherein R^{1a} and R^{2a} are identical to or different from each other, and each represent a C1-C3 alkyl group or a hydrogen atom.
[Aspect C144] The compound according to the Aspect C68, wherein R^{1a} and R^{2a} are identical to or different from each other, and each represent a C1-C3 alkyl group or a hydrogen atom.
[Aspect C145] The compound according to the Aspect C69, wherein R^{1a} and R^{2a} are identical to or different from each other, and each represent a C1-C3 alkyl group or a hydrogen atom.
[Aspect C146] The compound according to the Aspect C70, wherein R^{1a} and R^{2a} are identical to or different from each other, and each represent a C1-C3 alkyl group or a hydrogen atom.
[Aspect C147] The compound according to the Aspect C71, wherein R^{1a} and R^{2a} are identical to or different from each other, and each represent a C1-C3 alkyl group or a hydrogen atom.
[Aspect C148] The compound according to the Aspect C72, wherein R^{1a} and R^{2a} are identical to or different from each other, and each represent a C1-C3 alkyl group or a hydrogen atom.
[Aspect C149] The compound according to the Aspect C73, wherein R^{1a} and R^{2a} are identical to or different from each other, and each represent a C1-C3 alkyl group or a hydrogen atom.
[Aspect C150] The compound according to the Aspect C74, wherein R^{1a} and R^{2a} are identical to or different from each other, and each represent a C1-C3 alkyl group or a hydrogen atom.
[Aspect C151] The compound according to the Aspect C75, wherein R^{1a} and R^{2a} are identical to or different from each other, and each represent a C1-C3 alkyl group or a hydrogen atom.
[Aspect C152] The Compound N of the present invention, wherein R^{1a} and R^{2a} each represent a hydrogen atom.
[Aspect C153] The compound according to the Aspect C1, wherein R^{1a} and R^{2a} each represent a hydrogen atom.
[Aspect C154] The compound according to the Aspect C2, wherein R^{1a} and R^{2a} each represent a hydrogen atom.
[Aspect C155] The compound according to the Aspect C3, wherein R^{1a} and R^{2a} each represent a hydrogen atom.
[Aspect C156] The compound according to the Aspect C4, wherein R^{1a} and R^{2a} each represent a hydrogen atom.
[Aspect C157] The compound according to the Aspect C5, wherein R^{1a} and R^{2a} each represent a hydrogen atom.
[Aspect C158] The compound according to the Aspect C6, wherein R^{1a} and R^{2a} each represent a hydrogen atom.
[Aspect C159] The compound according to the Aspect C7, wherein R^{1a} and R^{2a} each represent a hydrogen atom.
[Aspect C160] The compound according to the Aspect C8, wherein R^{1a} and R^{2a} each represent a hydrogen atom.
[Aspect C161] The compound according to the Aspect C9, wherein R^{1a} and R^{2a} each represent a hydrogen atom.
[Aspect C162] The compound according to the Aspect C10, wherein R^{1a} and R^{2a} each represent a hydrogen atom.
[Aspect C163] The compound according to the Aspect C11, wherein R^{1a} and R^{2a} each represent a hydrogen atom.
[Aspect C164] The compound according to the Aspect C12, wherein R^{1a} and R^{2a} each represent a hydrogen atom.
[Aspect C165] The compound according to the Aspect C13, wherein R^{1a} and R^{2a} each represent a hydrogen atom.
[Aspect C166] The compound according to the Aspect C14, wherein R^{1a} and R^{2a} each represent a hydrogen atom.
[Aspect C167] The compound according to the Aspect C15, wherein R^{1a} and R^{2a} each represent a hydrogen atom.
[Aspect C168] The compound according to the Aspect C16, wherein R^{1a} and R^{2a} each represent a hydrogen atom.
[Aspect C169] The compound according to the Aspect C17, wherein R^{1a} and R^{2a} each represent a hydrogen atom.
[Aspect C170] The compound according to the Aspect C18, wherein R^{1a} and R^{2a} each represent a hydrogen atom.
[Aspect C171] The compound according to the Aspect C19, wherein R^{1a} and R^{2a} each represent a hydrogen atom.
[Aspect C172] The compound according to the Aspect C20, wherein R^{1a} and R^{2a} each represent a hydrogen atom.
[Aspect C173] The compound according to the Aspect C21, wherein R^{1a} and R^{2a} each represent a hydrogen atom.
[Aspect C174] The compound according to the Aspect C22, wherein R^{1a} and R^{2a} each represent a hydrogen atom.
[Aspect C175] The compound according to the Aspect C23, wherein R^{1a} and R^{2a} each represent a hydrogen atom.
[Aspect C176] The compound according to the Aspect C24, wherein R^{1a} and R^{2a} each represent a hydrogen atom.
[Aspect C177] The compound according to the Aspect C25, wherein R^{1a} and R^{2a} each represent a hydrogen atom.
[Aspect C178] The compound according to the Aspect C26, wherein R^{1a} and R^{2a} each represent a hydrogen atom.
[Aspect C179] The compound according to the Aspect C27, wherein R^{1a} and R^{2a} each represent a hydrogen atom.
[Aspect C180] The compound according to the Aspect C28, wherein R^{1a} and R^{2a} each represent a hydrogen atom.
[Aspect C181] The compound according to the Aspect C29, wherein R^{1a} and R^{2a} each represent a hydrogen atom.
[Aspect C182] The compound according to the Aspect C30, wherein R^{1a} and R^{2a} each represent a hydrogen atom.
[Aspect C183] The compound according to the Aspect C31, wherein R^{1a} and R^{2a} each represent a hydrogen atom.
[Aspect C184] The compound according to the Aspect C32, wherein R^{1a} and R^{2a} each represent a hydrogen atom.
[Aspect C185] The compound according to the Aspect C33, wherein R^{1a} and R^{2a} each represent a hydrogen atom.
[Aspect C186] The compound according to the Aspect C34, wherein R^{1a} and R^{2a} each represent a hydrogen atom.
[Aspect C187] The compound according to the Aspect C35, wherein R^{1a} and R^{2a} each represent a hydrogen atom.
[Aspect C188] The compound according to the Aspect C36, wherein R^{1a} and R^{2a} each represent a hydrogen atom.
[Aspect C189] The compound according to the Aspect C37, wherein R^{1a} and R^{2a} each represent a hydrogen atom.
[Aspect C190] The compound according to the Aspect C38, wherein R^{1a} and R^{2a} each represent a hydrogen atom.
[Aspect C191] The compound according to the Aspect C39, wherein R^{1a} and R^{2a} each represent a hydrogen atom.
[Aspect C192] The compound according to the Aspect C40, wherein R^{1a} and R^{2a} each represent a hydrogen atom.
[Aspect C193] The compound according to the Aspect C41, wherein R^{1a} and R^{2a} each represent a hydrogen atom.
[Aspect C194] The compound according to the Aspect C42, wherein R^{1a} and R^{2a} each represent a hydrogen atom.
[Aspect C195] The compound according to the Aspect C43, wherein R^{1a} and R^{2a} each represent a hydrogen atom.
[Aspect C196] The compound according to the Aspect C44, wherein R^{1a} and R^{2a} each represent a hydrogen atom.
[Aspect C197] The compound according to the Aspect C45, wherein R^{1a} and R^{2a} each represent a hydrogen atom.
[Aspect C198] The compound according to the Aspect C46, wherein R^{1a} and R^{2a} each represent a hydrogen atom.
[Aspect C199] The compound according to the Aspect C47, wherein R^{1a} and R^{2a} each represent a hydrogen atom.
[Aspect C200] The compound according to the Aspect C48, wherein R^{1a} and R^{2a} each represent a hydrogen atom.
[Aspect C201] The compound according to the Aspect C49, wherein R^{1a} and R^{2a} each represent a hydrogen atom.
[Aspect C202] The compound according to the Aspect C50, wherein R^{1a} and R^{2a} each represent a hydrogen atom.
[Aspect C203] The compound according to the Aspect C51, wherein R^{1a} and R^{2a} each represent a hydrogen atom.
[Aspect C204] The compound according to the Aspect C52, wherein R^{1a} and R^{2a} each represent a hydrogen atom.
[Aspect C205] The compound according to the Aspect C53, wherein R^{1a} and R^{2a} each represent a hydrogen atom.
[Aspect C206] The compound according to the Aspect C54, wherein R^{1a} and R^{2a} each represent a hydrogen atom.
[Aspect C207] The compound according to the Aspect C55, wherein R^{1a} and R^{2a} each represent a hydrogen atom.
[Aspect C208] The compound according to the Aspect C56, wherein R^{1a} and R^{2a} each represent a hydrogen atom.
[Aspect C209] The compound according to the Aspect C57, wherein R^{1a} and R^{2a} each represent a hydrogen atom.
[Aspect C210] The compound according to the Aspect C58, wherein R^{1a} and R^{2a} each represent a hydrogen atom.
[Aspect C211] The compound according to the Aspect C59, wherein R^{1a} and R^{2a} each represent a hydrogen atom.
[Aspect C212] The compound according to the Aspect C60, wherein R^{1a} and R^{2a} each represent a hydrogen atom.
[Aspect C213] The compound according to the Aspect C61, wherein R^{1a} and R^{2a} each represent a hydrogen atom.
[Aspect C214] The compound according to the Aspect C62, wherein R^{1a} and R^{2a} each represent a hydrogen atom.
[Aspect C215] The compound according to the Aspect C63, wherein R^{1a} and R^{2a} each represent a hydrogen atom.
[Aspect C216] The compound according to the Aspect C64, wherein R^{1a} and R^{2a} each represent a hydrogen atom.
[Aspect C217] The compound according to the Aspect C65, wherein R^{1a} and R^{2a} each represent a hydrogen atom.
[Aspect C218] The compound according to the Aspect C66, wherein R^{1a} and R^{2a} each represent a hydrogen atom.
[Aspect C219] The compound according to the Aspect C67, wherein R^{1a} and R^{2a} each represent a hydrogen atom.
[Aspect C220] The compound according to the Aspect C68, wherein R^{1a} and R^{2a} each represent a hydrogen atom.
[Aspect C221] The compound according to the Aspect C69, wherein R^{1a} and R^{2a} each represent a hydrogen atom.
[Aspect C222] The compound according to the Aspect C70, wherein R^{1a} and R^{2a} each represent a hydrogen atom.
[Aspect C223] The compound according to the Aspect C71, wherein R^{1a} and R^{2a} each represent a hydrogen atom.
[Aspect C224] The compound according to the Aspect C72, wherein R^{1a} and R^{2a} each represent a hydrogen atom.
[Aspect C225] The compound according to the Aspect C73, wherein R^{1a} and R^{2a} each represent a hydrogen atom.
[Aspect C226] The compound according to the Aspect C74, wherein R^{1a} and R^{2a} each represent a hydrogen atom.
[Aspect C227] The compound according to the Aspect C75, wherein R^{1a} and R^{2a} each represent a hydrogen atom.
[Aspect C228] The Compound N of the present invention, wherein R^{1a} and R^{2a} are identical to or different from each other, and each represent a hydrogen atom or a halogen atom.
[Aspect C229] The compound according to the Aspect C1, wherein R^{1a} and R^{2a} are identical to or different from each other, and each represent a hydrogen atom or a halogen atom.
[Aspect C230] The compound according to the Aspect C2, wherein R^{1a} and R^{2a} are identical to or different from each other, and each represent a hydrogen atom or a halogen atom.
[Aspect C231] The compound according to the Aspect C3, wherein R^{1a} and R^{2a} are identical to or different from each other, and each represent a hydrogen atom or a halogen atom.
[Aspect C232] The compound according to the Aspect C4, wherein R^{1a} and R^{2a} are identical to or different from each other, and each represent a hydrogen atom or a halogen atom.
[Aspect C233] The compound according to the Aspect C5, wherein R^{1a} and R^{2a} are identical to or different from each other, and each represent a hydrogen atom or a halogen atom.
[Aspect C234] The compound according to the Aspect C6, wherein R^{1a} and R^{2a} are identical to or different from each other, and each represent a hydrogen atom or a halogen atom.
[Aspect C235] The compound according to the Aspect C7, wherein R^{1a} and R^{2a} are identical to or different from each other, and each represent a hydrogen atom or a halogen atom.
[Aspect C236] The compound according to the Aspect C8, wherein R^{1a} and R^{2a} are identical to or different from each other, and each represent a hydrogen atom or a halogen atom.
[Aspect C237] The compound according to the Aspect C9, wherein R^{1a} and R^{2a} are identical to or different from each other, and each represent a hydrogen atom or a halogen atom.
[Aspect C238] The compound according to the Aspect C10, wherein R^{1a} and R^{2a} are identical to or different from each other, and each represent a hydrogen atom or a halogen atom.
[Aspect C239] The compound according to the Aspect C11, wherein R^{1a} and R^{2a} are identical to or different from each other, and each represent a hydrogen atom or a halogen atom.
[Aspect C240] The compound according to the Aspect C12, wherein R^{1a} and R^{2a} are identical to or different from each other, and each represent a hydrogen atom or a halogen atom.
[Aspect C241] The compound according to the Aspect C13, wherein R^{1a} and R^{2a} are identical to or different from each other, and each represent a hydrogen atom or a halogen atom.
[Aspect C242] The compound according to the Aspect C14, wherein R^{1a} and R^{2a} are identical to or different from each other, and each represent a hydrogen atom or a halogen atom.
[Aspect C243] The compound according to the Aspect C15, wherein R^{1a} and R^{2a} are identical to or different from each other, and each represent a hydrogen atom or a halogen atom.
[Aspect C244] The compound according to the Aspect C16, wherein R^{1a} and R^{2a} are identical to or different from each other, and each represent a hydrogen atom or a halogen atom.
[Aspect C245] The compound according to the Aspect C17, wherein R^{1a} and R^{2a} are identical to or different from each other, and each represent a hydrogen atom or a halogen atom.
[Aspect C246] The compound according to the Aspect C18, wherein R^{1a} and R^{2a} are identical to or different from each other, and each represent a hydrogen atom or a halogen atom.
[Aspect C247] The compound according to the Aspect C19, wherein R^{1a} and R^{2a} are identical to or different from each other, and each represent a hydrogen atom or a halogen atom.
[Aspect C248] The compound according to the Aspect C20, wherein R^{1a} and R^{2a} are identical to or different from each other, and each represent a hydrogen atom or a halogen atom.
[Aspect C249] The compound according to the Aspect C21, wherein R^{1a} and R^{2a} are identical to or different from each other, and each represent a hydrogen atom or a halogen atom.
[Aspect C250] The compound according to the Aspect C22, wherein R^{1a} and R^{2a} are identical to or different from each other, and each represent a hydrogen atom or a halogen atom.
[Aspect C251] The compound according to the Aspect C23, wherein R^{1a} and R^{2a} are identical to or different from each other, and each represent a hydrogen atom or a halogen atom.
[Aspect C252] The compound according to the Aspect C24, wherein R^{1a} and R^{2a} are identical to or different from each other, and each represent a hydrogen atom or a halogen atom.
[Aspect C253] The compound according to the Aspect C25, wherein R^{1a} and R^{2a} are identical to or different from each other, and each represent a hydrogen atom or a halogen atom.
[Aspect C254] The compound according to the Aspect C26, wherein R^{1a} and R^{2a} are identical to or different from each other, and each represent a hydrogen atom or a halogen atom.
[Aspect C255] The compound according to the Aspect C27, wherein R^{1a} and R^{2a} are identical to or different from each other, and each represent a hydrogen atom or a halogen atom.
[Aspect C256] The compound according to the Aspect C28, wherein R^{1a} and R^{2a} are identical to or different from each other, and each represent a hydrogen atom or a halogen atom.
[Aspect C257] The compound according to the Aspect C29, wherein R^{1a} and R^{2a} are identical to or different from each other, and each represent a hydrogen atom or a halogen atom.
[Aspect C258] The compound according to the Aspect C30, wherein R^{1a} and R^{2a} are identical to or different from each other, and each represent a hydrogen atom or a halogen atom.
[Aspect C259] The compound according to the Aspect C31, wherein R^{1a} and R^{2a} are identical to or different from each other, and each represent a hydrogen atom or a halogen atom.
[Aspect C260] The compound according to the Aspect C32, wherein R^{1a} and R^{2a} are identical to or different from each other, and each represent a hydrogen atom or a halogen atom.
[Aspect C261] The compound according to the Aspect C33, wherein R^{1a} and R^{2a} are identical to or different from each other, and each represent a hydrogen atom or a halogen atom.
[Aspect C262] The compound according to the Aspect C34, wherein R^{1a} and R^{2a} are identical to or different from each other, and each represent a hydrogen atom or a halogen atom.
[Aspect C263] The compound according to the Aspect C35, wherein R^{1a} and R^{2a} are identical to or different from each other, and each represent a hydrogen atom or a halogen atom.
[Aspect C264] The compound according to the Aspect C36, wherein R^{1a} and R^{2a} are identical to or different from each other, and each represent a hydrogen atom or a halogen atom.
[Aspect C265] The compound according to the Aspect C37, wherein R^{1a} and R^{2a} are identical to or different from each other, and each represent a hydrogen atom or a halogen atom.
[Aspect C266] The compound according to the Aspect C38, wherein R^{1a} and R^{2a} are identical to or different from each other, and each represent a hydrogen atom or a halogen atom.
[Aspect C267] The compound according to the Aspect C39, wherein R^{1a} and R^{2a} are identical to or different from each other, and each represent a hydrogen atom or a halogen atom.
[Aspect C268] The compound according to the Aspect C40, wherein R^{1a} and R^{2a} are identical to or different from each other, and each represent a hydrogen atom or a halogen atom.
[Aspect C269] The compound according to the Aspect C41, wherein R^{1a} and R^{2a} are identical to or different from each other, and each represent a hydrogen atom or a halogen atom.
[Aspect C270] The compound according to the Aspect C42, wherein R^{1a} and R^{2a} are identical to or different from each other, and each represent a hydrogen atom or a halogen atom.
[Aspect C271] The compound according to the Aspect C43, wherein R^{1a} and R^{2a} are identical to or different from each other, and each represent a hydrogen atom or a halogen atom.
[Aspect C272] The compound according to the Aspect C44, wherein R^{1a} and R^{2a} are identical to or different from each other, and each represent a hydrogen atom or a halogen atom.
[Aspect C273] The compound according to the Aspect C45, wherein R^{1a} and R^{2a} are identical to or different from each other, and each represent a hydrogen atom or a halogen atom.
[Aspect C274] The compound according to the Aspect C46, wherein R^{1a} and R^{2a} are identical to or different from each other, and each represent a hydrogen atom or a halogen atom.
[Aspect C275] The compound according to the Aspect C47, wherein R^{1a} and R^{2a} are identical to or different from each other, and each represent a hydrogen atom or a halogen atom.
[Aspect C276] The compound according to the Aspect C48, wherein R^{1a} and R^{2a} are identical to or different from each other, and each represent a hydrogen atom or a halogen atom.
[Aspect C277] The compound according to the Aspect C49, wherein R^{1a} and R^{2a} are identical to or different from each other, and each represent a hydrogen atom or a halogen atom.
[Aspect C278] The compound according to the Aspect C50, wherein R^{1a} and R^{2a} are identical to or different from each other, and each represent a hydrogen atom or a halogen atom.
[Aspect C279] The compound according to the Aspect C51, wherein R^{1a} and R^{2a} are identical to or different from each other, and each represent a hydrogen atom or a halogen atom.
[Aspect C280] The compound according to the Aspect C52, wherein R^{1a} and R^{2a} are identical to or different from each other, and each represent a hydrogen atom or a halogen atom.
[Aspect C281] The compound according to the Aspect C53, wherein R^{1a} and R^{2a} are identical to or different from each other, and each represent a hydrogen atom or a halogen atom.
[Aspect C282] The compound according to the Aspect C54, wherein R^{1a} and R^{2a} are identical to or different from each other, and each represent a hydrogen atom or a halogen atom.
[Aspect C283] The compound according to the Aspect C55, wherein R^{1a} and R^{2a} are identical to or different from each other, and each represent a hydrogen atom or a halogen atom.
[Aspect C284] The compound according to the Aspect C56, wherein R^{1a} and R^{2a} are identical to or different from each other, and each represent a hydrogen atom or a halogen atom.
[Aspect C285] The compound according to the Aspect C57, wherein R^{1a} and R^{2a} are identical to or different from each other, and each represent a hydrogen atom or a halogen atom.
[Aspect C286] The compound according to the Aspect C58, wherein R^{1a} and R^{2a} are identical to or different from each other, and each represent a hydrogen atom or a halogen atom.
[Aspect C287] The compound according to the Aspect C59, wherein R^{1a} and R^{2a} are identical to or different from each other, and each represent a hydrogen atom or a halogen atom.
[Aspect C288] The compound according to the Aspect C60, wherein R^{1a} and R^{2a} are identical to or different from each other, and each represent a hydrogen atom or a halogen atom.
[Aspect C289] The compound according to the Aspect C61, wherein R^{1a} and R^{2a} are identical to or different from each other, and each represent a hydrogen atom or a halogen atom.
[Aspect C290] The compound according to the Aspect C62, wherein R^{1a} and R^{2a} are identical to or different from each other, and each represent a hydrogen atom or a halogen atom.
[Aspect C291] The compound according to the Aspect C63, wherein R^{1a} and R^{2a} are identical to or different from each other, and each represent a hydrogen atom or a halogen atom.
[Aspect C292] The compound according to the Aspect C64, wherein R^{1a} and R^{2a} are identical to or different from each other, and each represent a hydrogen atom or a halogen atom.
[Aspect C293] The compound according to the Aspect C65, wherein R^{1a} and R^{2a} are identical to or different from each other, and each represent a hydrogen atom or a halogen atom.
[Aspect C294] The compound according to the Aspect C66, wherein R^{1a} and R^{2a} are identical to or different from each other, and each represent a hydrogen atom or a halogen atom.
[Aspect C295] The compound according to the Aspect C67, wherein R^{1a} and R^{2a} are identical to or different from each other, and each represent a hydrogen atom or a halogen atom.
[Aspect C296] The compound according to the Aspect C68, wherein R^{1a} and R^{2a} are identical to or different from each other, and each represent a hydrogen atom or a halogen atom.
[Aspect C297] The compound according to the Aspect C69, wherein R^{1a} and R^{2a} are identical to or different from each other, and each represent a hydrogen atom or a halogen atom.
[Aspect C298] The compound according to the Aspect C70, wherein R^{1a} and R^{2a} are identical to or different from each other, and each represent a hydrogen atom or a halogen atom.
[Aspect C299] The compound according to the Aspect C71, wherein R^{1a} and R^{2a} are identical to or different from each other, and each represent a hydrogen atom or a halogen atom.
[Aspect C300] The compound according to the Aspect C72, wherein R^{1a} and R^{2a} are identical to or different from each other, and each represent a hydrogen atom or a halogen atom.
[Aspect C301] The compound according to the Aspect C73, wherein R^{1a} and R^{2a} are identical to or different from each other, and each represent a hydrogen atom or a halogen atom.
[Aspect C302] The compound according to the Aspect C74, wherein R^{1a} and R^{2a} are identical to or different from each other, and each represent a hydrogen atom or a halogen atom.
[Aspect C303] The compound according to the Aspect C75, wherein R^{1a} and R^{2a} are identical to or different from each other, and each represent a hydrogen atom or a halogen atom.
[Aspect C304] The Compound N of the present invention or the compound according to any one of the Aspects C1 to C303, wherein Z^{a} represents a 5-6 membered aromatic heterocyclic group optionally substituted with one or more substituent(s) selected from Group D^{a}.
[Aspect C305] The Compound N of the present invention or the compound according to any one of the Aspects C1 to C303, wherein Z^{a} represents a 5 membered aromatic heterocyclic group optionally substituted with one or more substituent(s) selected from Group D^{a}.
[Aspect C306] The Compound N of the present invention or the compound according to any one of the Aspects C1 to C303, wherein Z^{a} represents a 6 membered aromatic heterocyclic group optionally substituted with one or more substituent(s) selected from Group D^{a}.
[Aspect C307] The Compound N of the present invention or the compound according to any one of the Aspects C1 to C303, wherein Z^{a} represents a 5-6 membered aromatic heterocyclic group optionally substituted with one or more substituent(s) selected from Group D2^{a};
   Group D2^{a}: a group consisting of a C1-C3 alkyl group, a halogen atom, and a cyano group.
[Aspect C308] The Compound N of the present invention or the compound according to any one of the Aspects C1 to C303, wherein Z^{a} represents a 5 membered aromatic heterocyclic group optionally substituted with one or more substituent(s) selected from Group D2^{a}.
[Aspect C309] The Compound N of the present invention or the compound according to any one of the Aspects C1 to C303, wherein Z^{a} represents a 6 membered aromatic heterocyclic group optionally substituted with one or more substituent(s) selected from Group D2^{a}.
[Aspect C310] The Compound N of the present invention or the compound according to any one of the Aspects C1 to C303, wherein Z^{a} represents a 5-6 membered aromatic heterocyclic group {provided that the atom in said 5-6 membered aromatic heterocyclic group attached to the sulfonyl group is a carbon atom. Also, said 5-6 membered aromatic heterocyclic group may be optionally substituted with one or more substituent(s) selected from Group D^{a}}.
[Aspect C311] The Compound N of the present invention or the compound according to any one of the Aspects C1 to C303, wherein Z^{a} represents a 5-6 membered aromatic heterocyclic group optionally substituted with one or more substituent(s) selected from Group D2^{a} (provided that a 1,3-dimethyl-1H-pyrazol-4-yl group is excluded).
[Aspect C312] The Compound N of the present invention or the compound according to any one of the Aspects C1 to C303, wherein Z^{a} represents a 5 membered aromatic heterocyclic group optionally substituted with one or more substituent(s) selected from Group D2^{a} (provided that a 1,3-dimethyl-1H-pyrazol-4-yl group is excluded).
[Aspect C313] The Compound N of the present invention or the compound according to any one of the Aspects C1 to C303, wherein Z^{a} represents a 5-6 membered aromatic heterocyclic group optionally substituted with one or more substituent(s) selected from Group D2^{a} (provided that a 2-thienyl group is excluded).
[Aspect C314] The Compound N of the present invention or the compound according to any one of the Aspects C1 to C303, wherein Z^{a} represents a 5 membered aromatic heterocyclic group optionally substituted with one or more substituent(s) selected from Group D2^{a} (provided that a 2-thienyl group is excluded).
[Aspect C315] The Compound N of the present invention or the compound according to any one of the Aspects C1 to C303, wherein Z^{a} represents a 5-6 membered aromatic heterocyclic group optionally substituted with one or more substituent(s) selected from Group D2^{a} (provided that a 1,3-dimethyl-1H-pyrazol-4-yl group and a 2-thienyl group are excluded).
[Aspect C316] The Compound N of the present invention or the compound according to any one of the Aspects C1 to C303, wherein Z^{a} represents a 5 membered aromatic heterocyclic group optionally substituted with one or more substituent(s) selected from Group D2^{a} (provided that a 1,3-dimethyl-1H-pyrazol-4-yl group and a 2-thienyl group are excluded).
[Aspect C317] The Compound N of the present invention or the compound according to any one of the Aspects C1 to C303, wherein Z^{a} represents a furanyl group, a thienyl group, an imidazolyl group, a pyrazolyl group, or a pyridyl group {wherein said furanyl group, said thienyl group, said imidazolyl group, said pyrazolyl group, and said pyridyl group may be optionally substituted with one or more substituent(s) selected from Group D3^{a}};
   Group D3^{a}: a group consisting of a C1-C3 alkyl group and a halogen atom.
[Aspect C318] The Compound N of the present invention or the compound according to any one of the Aspects C1 to C303, wherein Z^{a} represents a furanyl group, an imidazolyl group, or a pyridyl group {wherein said furanyl group, said imidazolyl group, and said pyridyl group may be optionally substituted with one or more substituent(s) selected from Group D3^{a}}.
[Aspect C319] The Compound N of the present invention or the compound according to any one of the Aspects C1 to C303, wherein Z^{a} represents a 5 membered aromatic heterocyclic group {provided that the atom in said 5 membered aromatic heterocyclic group attached to the sulfonyl group is a carbon atom. Also, said 5 membered aromatic heterocyclic group may be optionally substituted with one or more substituent(s) selected from Group D^{a}}.
[Aspect C320] The Compound N of the present invention or the compound according to any one of the Aspects C1 to C303, wherein Z^{a} represents a 6 membered aromatic heterocyclic group {provided that the atom in said 6 membered aromatic heterocyclic group attached to the sulfonyl group is a carbon atom. Also, said 6 membered aromatic heterocyclic group may be optionally substituted with one or more substituent(s) selected from Group D^{a}}.
[Aspect C321] The Compound N of the present invention or the compound according to any one of the Aspects C1 to C303, wherein Z^{a} represents a 5-6 membered aromatic heterocyclic group optionally substituted with one or more substituent(s) selected from Group F^{a}.
[Aspect C322] The Compound N of the present invention or the compound according to any one of the Aspects C1 to C303, wherein Z^{a} represents a 5 membered aromatic heterocyclic group optionally substituted with one or more substituent(s) selected from Group F^{a}.
[Aspect C323] The Compound N of the present invention or the compound according to any one of the Aspects C1 to C303, wherein Z^{a} represents a 6 membered aromatic heterocyclic group optionally substituted with one or more substituent(s) selected from Group F^{a}.
[Aspect C324] The Compound N of the present invention, wherein
   R^{3a} represents a C1-C6 chain hydrocarbon group optionally substituted with one or more halogen atom(s), a C3-C8 alicyclic hydrocarbon group, a halogen atom, a nitro group, NR^{8a}R^{9a}, OR^{10a}, or SR^{11a}; and
   adjacent two R^{3a} and R^{3a} may be optionally combined with the carbon atoms to which they are attached to form a C4-C7 alicyclic hydrocarbon, a 5-7 membered nonaromatic heterocyclic ring, or a 5-7 membered aromatic heterocyclic ring {wherein said C4-C7 alicyclic hydrocarbon, said 5-7 membered nonaromatic heterocyclic ring, and said 5-7 membered aromatic heterocyclic ring may be optionally substituted with one or more halogen atom(s)}.
[Aspect C325] The Compound N of the present invention, wherein R^{3a} represents a C1-C6 chain hydrocarbon group optionally substituted with one or more halogen atom(s), a C3-C8 alicyclic hydrocarbon group, a halogen atom, a nitro group, NR^{8a}R^{9a}, OR^{10a}, or SR^{11a}.
[Aspect C326] The Compound N of the present invention, wherein adjacent two R^{3a} and R^{3a} are combined with the carbon atoms to which they are attached to form a C4-C7 alicyclic hydrocarbon, a 5-7 membered nonaromatic heterocyclic ring, or a 5-7 membered aromatic heterocyclic ring {wherein said C4-C7 alicyclic hydrocarbon, said 5-7 membered nonaromatic heterocyclic ring, and said 5-7 membered aromatic heterocyclic ring may be optionally substituted with one or more halogen atom(s)}.
[Aspect C327] The compound according to the Aspect C324, wherein q represents 1.
[Aspect C328] The compound according to the Aspect C324, wherein q represents 2.
[Aspect C329] The compound according to the Aspect C324, wherein q represents 3.
[Aspect C330] The compound according to the Aspect C324, wherein q represents 1 or 2.
[Aspect C331] The compound according to the Aspect C325, wherein q represents 1.
[Aspect C332] The compound according to the Aspect C325, wherein q represents 2.
[Aspect C333] The compound according to the Aspect C325, wherein q represents 3.
[Aspect C334] The compound according to the Aspect C325, wherein q represents 1 or 2.
[Aspect C335] The compound according to the Aspect C326, wherein q represents 2.
[Aspect C336] The compound according to the Aspect C326, wherein q represents 3.
[Aspect C337] The compound according to the Aspect C324, wherein R^{1a} and R^{2a} each represent a hydrogen atom.
[Aspect C338] The compound according to the Aspect C325, wherein R^{1a} and R^{2a} each represent a hydrogen atom.
[Aspect C339] The compound according to the Aspect C326, wherein R^{1a} and R^{2a} each represent a hydrogen atom.
[Aspect C340] The compound according to the Aspect C327, wherein R^{1a} and R^{2a} each represent a hydrogen atom.
[Aspect C341] The compound according to the Aspect C328, wherein R^{1a} and R^{2a} each represent a hydrogen atom.
[Aspect C342] The compound according to the Aspect C329, wherein R^{1a} and R^{2a} each represent a hydrogen atom.
[Aspect C343] The compound according to the Aspect C330, wherein R^{1a} and R^{2a} each represent a hydrogen atom.
[Aspect C344] The compound according to the Aspect C331, wherein R^{1a} and R^{2a} each represent a hydrogen atom.
[Aspect C345] The compound according to the Aspect C332, wherein R^{1a} and R^{2a} each represent a hydrogen atom.
[Aspect C346] The compound according to the Aspect C333, wherein R^{1a} and R^{2a} each represent a hydrogen atom.
[Aspect C347] The compound according to the Aspect C334, wherein R^{1a} and R^{2a} each represent a hydrogen atom.
[Aspect C348] The compound according to the Aspect C335, wherein R^{1a} and R^{2a} each represent a hydrogen atom.
[Aspect C349] The compound according to the Aspect C336, wherein R^{1a} and R^{2a} each represent a hydrogen atom.
[Aspect C350] The compound according to any one of the Aspects C324 to C349, wherein Z^{a} represents a 5-6 membered aromatic heterocyclic group optionally substituted with one or more substituent(s) selected from Group D^{a}.
[Aspect C351] The compound according to any one of the Aspects C324 to C349, wherein Z^{a} represents a 5 membered aromatic heterocyclic group optionally substituted with one or more substituent(s) selected from Group D^{a}.
[Aspect C352] The compound according to any one of the Aspects C324 to C349, wherein Z^{a} represents a 6 membered aromatic heterocyclic group optionally substituted with one or more substituent(s) selected from Group D^{a}.
[Aspect C353] The compound according to any one of the Aspects C324 to C349, wherein Z^{a} represents a 5-6 membered aromatic heterocyclic group optionally substituted with one or more substituent(s) selected from Group D2^{a}.
[Aspect C354] The compound according to any one of the Aspects C324 to C349, wherein Z^{a} represents a 5 membered aromatic heterocyclic group optionally substituted with one or more substituent(s) selected from Group D2^{a}.
[Aspect C355] The compound according to any one of the Aspects C324 to C349, wherein Z^{a} represents a 6 membered aromatic heterocyclic group optionally substituted with one or more substituent(s) selected from Group D2^{a}.
[Aspect C356] The compound according to any one of the Aspects C324 to C349, wherein Z^{a} represents a 5-6 membered aromatic heterocyclic group {provided that the atom in said 5-6 membered aromatic heterocyclic group attached to the sulfonyl group is a carbon atom. Also, said 5-6 membered aromatic heterocyclic group may be optionally substituted with one or more substituent(s) selected from Group D^{a}}.
[Aspect C357] The compound according to any one of the Aspects C324 to C349, wherein Z^{a} represents a 5-6 membered aromatic heterocyclic group optionally substituted with one or more substituent(s) selected from Group D2^{a} (provided that a 1,3-dimethyl-1H-pyrazol-4-yl group is excluded).
[Aspect C358] The compound according to any one of the Aspects C324 to C349, wherein Z^{a} represents a 5 membered aromatic heterocyclic group optionally substituted with one or more substituent(s) selected from Group D2^{a} (provided that a 1,3-dimethyl-1H-pyrazol-4-yl group is excluded).
[Aspect C359] The compound according to any one of the Aspects C324 to C349, wherein Z^{a} represents a 5-6 membered aromatic heterocyclic group optionally substituted with one or more substituent(s) selected from Group D2^{a} (provided that a 2-thienyl group is excluded).
[Aspect C360] The compound according to any one of the Aspects C324 to C349, wherein Z^{a} represents a 5 membered aromatic heterocyclic group optionally substituted with one or more substituent(s) selected from Group D2^{a} (provided that a 2-thienyl group is excluded).
[Aspect C361] The compound according to any one of the Aspects C324 to C349, wherein Z^{a} represents a 5-6 membered aromatic heterocyclic group optionally substituted with one or more substituent(s) selected from Group D2^{a} (provided that a 1,3-dimethyl-1H-pyrazol-4-yl group and a 2-thienyl group are excluded).
[Aspect C362] The compound according to any one of the Aspects C324 to C349, wherein Z^{a} represents a 5 membered aromatic heterocyclic group optionally substituted with one or more substituent(s) selected from Group D2^{a} (provided that a 1,3-dimethyl-1H-pyrazol-4-yl group and a 2-thienyl group are excluded).
[Aspect C363] The compound according to any one of the Aspects C324 to C349, wherein Z^{a} represents a furanyl group, a thienyl group, an imidazolyl group, a pyrazolyl group, or a pyridyl group {wherein said furanyl group, said thienyl group, said imidazolyl group, said pyrazolyl group, and said pyridyl group may be optionally substituted with one or more substituent(s) selected from Group D3^{a}}.
[Aspect C364] The compound according to any one of the Aspects C324 to C349, wherein Z^{a} represents a furanyl group, an imidazolyl group, or a pyridyl group {wherein said furanyl group, said imidazolyl group, and said pyridyl group may be optionally substituted with one or more substituent(s) selected from Group D3^{a}}.
[Aspect C365] The compound according to any one of the Aspects C324 to C349, wherein Z^{a} represents a 5 membered aromatic heterocyclic group {provided that the atom in said 5 membered aromatic heterocyclic group attached to the sulfonyl group is a carbon atom. Also, said 5 membered aromatic heterocyclic group may be optionally substituted with one or more substituent(s) selected from Group D^{a}}.
[Aspect C366] The compound according to any one of the Aspects C324 to C349, wherein Z^{a} represents a 6 membered aromatic heterocyclic group {provided that the atom in said 6 membered aromatic heterocyclic group attached to the sulfonyl group is a carbon atom. Also, said 6 membered aromatic heterocyclic group may be optionally substituted with one or more substituent(s) selected from Group D^{a}}.
[Aspect C367] The compound according to any one of the Aspects C324 to C349, wherein Z^{a} represents a 5-6 membered aromatic heterocyclic group optionally substituted with one or more substituent(s) selected from Group F^{a}.
[Aspect C368] The compound according to any one of the Aspects C324 to C349, wherein Z^{a} represents a 5 membered aromatic heterocyclic group optionally substituted with one or more substituent(s) selected from Group F^{a}.
[Aspect C369] The compound according to any one of the Aspects C324 to C349, wherein Z^{a} represents a 6 membered aromatic heterocyclic group optionally substituted with one or more substituent(s) selected from Group F^{a}.
[Aspect A1] A method for controlling Phakopsora pachyrhizi which comprises applying the Present compound to a soybean or soil for cultivating a soybean.
[Aspect AC1] A method for controlling Phakopsora pachyrhizi which comprises applying the Compound of the present invention to a soybean or soil for cultivating a soybean.

Aspects of the Intermediate A include the following compounds.
[Aspect B1] The compound according to the Intermediate A, wherein X^{b} represents a chlorine atom, a bromine atom, or an iodine atom.
[Aspect B2] The compound according to the Intermediate A, wherein X^{b} represents an iodine atom.
[Aspect B3] The compound according to the Intermediate A, wherein X^{b} represents B(OH)₂ or a 4,4,5,5-tetramethyl-1,3,2-dioxaborolan-2-yl group.
[Aspect B4] The compound according to the Intermediate A or any one of the Aspects B1 to B3, wherein Z^{b} represents a 5-10 membered aromatic heterocyclic group optionally substituted with one or more substituent(s) selected from Group F^{b};
   Group F^{b}: a group consisting of a C1-C6 chain hydrocarbon group and a halogen atom.
[Aspect B5] The compound according to the Intermediate A or any one of the Aspects B1 to B3, wherein Z^{b} represents a 5-6 membered aromatic heterocyclic group optionally substituted with one or more substituent(s) selected from Group F^{b}.
[Aspect B6] The compound according to the Intermediate A or any one of the Aspects B1 to B3, wherein Z^{b} represents a 5 membered aromatic heterocyclic group optionally substituted with one or more substituent(s) selected from Group F^{b}.
[Aspect B7] The compound according to the Intermediate A or any one of the Aspects B1 to B3, wherein Z^{b} represents a 6 membered aromatic heterocyclic group optionally substituted with one or more substituent(s) selected from Group F^{b}.
[Aspect B8] The compound according to the Intermediate A or any one of the Aspects B1 to B3, wherein Z^{b} represents a 5 membered aromatic heterocyclic group.
[Aspect B9] The compound according to the Intermediate A or any one of the Aspects B1 to B3, wherein Z^{b} represents a 6 membered aromatic heterocyclic group.

Next, Production methods for the Present compounds (including the Compounds of the present invention) are described.

### Production method A

A compound represented by formula (I) (i.e., Present compound N) may be prepared by reacting a compound represented by formula (A1) (hereinafter referred to as "Compound (A1)") with a compound represented by formula (A2) (hereinafter referred to as "Compound (A2)") in the presence of a base. 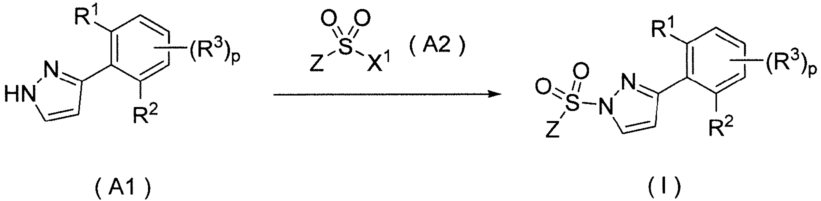 [wherein X¹ represents a chlorine atom, a bromine atom, or OS(O)₂Z; and the other symbols are the same as defined above.]

The reaction is usually carried out in a solvent. Examples of the solvent to be used in the reaction include hydrocarbons such as hexane, toluene, and xylene (hereinafter collectively referred to as "hydrocarbons"); ethers such as methyl tert-butyl ether (hereinafter referred to as "MTBE"), tetrahydrofuran (hereinafter referred to as "THF"), and dimethoxyethane (hereinafter collectively referred to as "ethers"); halogenated hydrocarbons such as chloroform and chlorobenzene (hereinafter collectively referred to as "halogenated hydrocarbons"); amides such as dimethylformamide (hereinafter referred to as "DMF") and N-methylpyrrolidone (hereinafter collectively referred to as "amides"); esters such as methyl acetate and ethyl acetate (hereinafter collectively referred to as "esters"); nitrile such as acetonitrile and propionitrile (hereinafter collectively referred to as "nitriles"); water; and mixtures of two or more of them.

Examples of the base to be used in the reaction include organic bases such as triethylamine and pyridine (hereinafter collectively referred to as "organic bases"); alkali metal carbonates such as sodium carbonate and potassium carbonate (hereinafter collectively referred to as "alkali metal carbonates"); alkali metal hydrogen carbonates such as sodium hydrogen carbonate and potassium hydrogen carbonate (hereinafter collectively referred to as "alkali metal hydrogen carbonates"); alkali metal hydroxides such as lithium hydroxide, sodium hydroxide, potassium hydroxide, and cesium hydroxide (hereinafter collectively referred to as "alkali metal hydroxides"); alkali metal hydrides such as lithium hydride and sodium hydride (hereinafter collectively referred to as "alkali metal hydrides"); and alkali metal alkoxides such as sodium tert-butoxide and potassium tert-butoxide (hereinafter collectively referred to as "alkali metal alkoxides").

In the reaction, the Compound (A2) is usually used at a ratio of 1 to 10 mol, and the base is usually used at a ratio of 1 to 10 mol, relative to 1 mol of the Compound (A1).

The reaction temperature is usually within the range of -20 to 150°C. The reaction time is usually within the range of 0.1 to 120 hour(s).

When the reaction is completed, the reaction mixture may be subjected to a work-up such as adding water to the reaction mixture, then subjecting the resulting mixture to extraction with organic solvent(s), and drying and/or concentrating the resulting organic layer to isolate the Present compound N.

The Compound (A2) is a commercially available compound or may be prepared by using known method(s).

### Production method B

The Present compound N may also be prepared by reacting a compound represented by formula (B1) (hereinafter referred to as "Compound (B1)") with a compound represented by formula (B2) (hereinafter referred to as "Compound (B2)") in the presence of a palladium catalyst and a base. 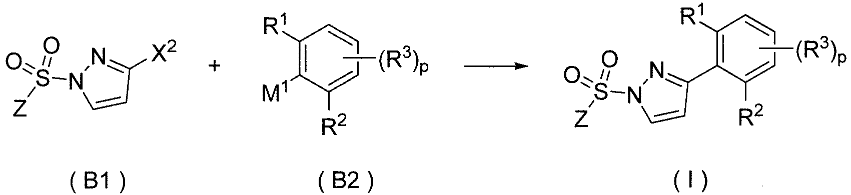 [wherein X² represents a chlorine atom, a bromine atom, an iodine atom, or a trifluoromethanesulfonyloxy group; M¹ represents B(OH)₂ or a 4,4,5,5-tetramethyl-1,3,2-dioxaborolan-2-yl group; and the other symbols are the same as defined above.]

The reaction is usually carried out in a solvent. Examples of the solvent to be used in the reaction include hydrocarbons; ethers; halogenated hydrocarbons; amides; esters; sulfoxides such as dimethyl sulfoxide (hereinafter referred to as "DMSO") (hereinafter collectively referred to as "sulfoxides"); ketones such as acetone and methyl isobutyl ketone (hereinafter collectively referred to as "ketones"); nitriles; alcohols such as methanol and ethanol (hereinafter collectively referred to as "alcohols"); water; and mixtures of two or more of them.

Examples of the palladium catalyst to be used in the reaction include palladium(II) acetate, dichlorobis(triphenylphosphine)palladium(II) , tetrakis(triphenylphosphine)palladium(0), [1,1'-bis(diphenylphosphino)ferrocene]palladium(II) dichloride, and tris(dibenzylideneacetone)dipalladium.

Examples of the base to be used in the reaction include organic bases; alkali metal carbonates; alkali metal hydrogen carbonates; alkali metal hydroxides; alkali metal fluorides such as sodium fluoride, potassium fluoride, and cesium fluoride (hereinafter collectively referred to as "alkali metal fluorides"); alkali metal hydrides; alkali metal alkoxides; and tripotassium phosphate.

In the reaction, the Compound (B2) is usually used at a ratio of 1 to 10 mol, the base is usually used at a ratio of 1 to 10 mol, and the palladium catalyst is usually used at a ratio of 0.0001 to 1 mol, relative to 1 mol of the Compound (B1).

The reaction temperature is usually within the range of 0 to 150°C. The reaction time is usually within the range of 0.1 to 24 hour(s).

When the reaction is completed, the reaction mixture may be subjected to a work-up such as adding water to the reaction mixture, then subjecting the resulting mixture to extraction with organic solvent(s), and drying and/or concentrating the resulting organic layer to isolate the Present compound.

The Compound (B2) is a commercially available compound or may be prepared according to the method disclosed in, for example, Chem. Rev., 1995, 95, 2457.

### Production method C

The Present compound N may also be prepared by reacting a compound represented by formula (C1) (hereinafter referred to as "Compound (C1)") with a compound represented by formula (C2) (hereinafter referred to as "Compound (C2)") in the presence of a palladium catalyst and a base. 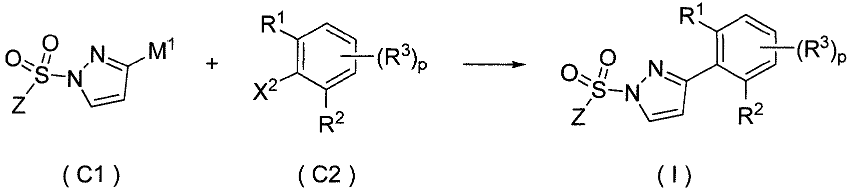 [wherein the symbols are the same as defined above.]

The reaction may be carried out according to the Production method B using the Compound (C2) instead of the Compound (B1), and using the Compound (C1) instead of the Compound (B2).

The Compound (C2) is a commercially available compound or may be prepared according to the method disclosed in, for example, Tetrahedron Letters, 2003, 8781.

Next, production methods of the intermediates of the Present compounds (including the Compounds of the present invention) are described.

### Reference Production method A

The Compound (A1) may be prepared by reacting a compound represented by formula (MA1) (hereinafter referred to as "Compound (MA1)") with hydrazine. 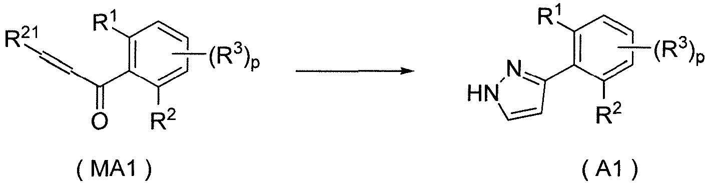 [wherein R²¹ represents a hydroxy group, a dimethylamino group, or a diethylamino group; and the other symbols are the same as defined above.]

The reaction may be carried out according to the method disclosed in, for example, Bioorganic and Medicinal Chemistry, 2014, 22, 4189. or J. Med. Chem., 2003, 46, 5416.

### Reference Production method B

A compound represented by formula (MA2) (hereinafter referred to as "Compound (MA2)") may be prepared by reacting a compound represented by formula (MB1) (hereinafter referred to as "Compound (MB1)") with a compound represented by formula (MB2) (hereinafter referred to as "Compound (MB2)"). 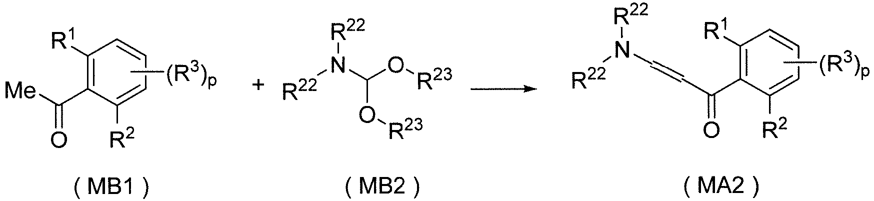 [wherein R²² are identical to or different from each other, and each represent a methyl group or an ethyl group; R²³ are identical to or different from each other, and each represent a C1-C6 alkyl group or a benzyl group; and the other symbols are the same as defined above.]

The reaction may be carried out according to the method disclosed in, for example, European Journal of Medicinal Chemistry, 2011, 46, 5817.

The Compound (MB2) is a commercially available compound or may be prepared by using known method(s).

### Reference Production method C

A compound represented by formula (MA3) (hereinafter referred to as "Compound (MA3)") may be prepared by reacting the Compound (MB1) with a compound represented by formula (MC1) (hereinafter referred to as "Compound (MC1)") in the presence of a base. 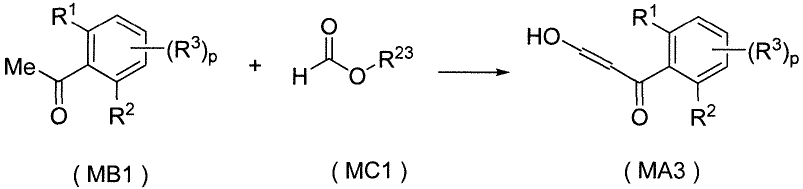 [wherein the symbols are the same as defined above.]

The reaction may be carried out according to the method disclosed in, for example, J. Am. Chem. Soc., 2017, 139, 2421.

The Compound (MC1) is a commercially available compound or may be prepared by using known method(s).

### Reference Production method D

The Compound (MB1) may be prepared by reacting a compound represented by formula (MD1) (hereinafter referred to as "Compound (MD1)") with a compound represented by formula (MD2) (hereinafter referred to as "Compound (MD2)"). 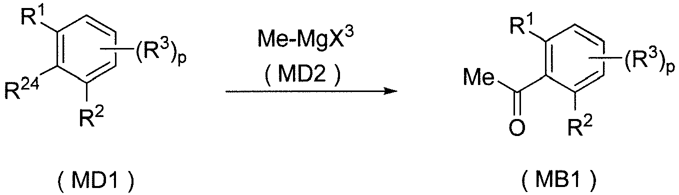 [wherein R²⁴ represents a cyano group, a chlorocarbonyl group, a di(C1-C4alkyl)carbamoyl group, a N-methoxy-N-methylcarbamoyl group, a morpholinocarbonyl group, a pyrrol-1-ylcarbonyl group, a pyrazol-1-ylcarbonyl group, an imidazol-1-ylcarbonyl group, or a benzotriazol-1-ylcarbonyl group; X³ represents a chlorine atom, a bromine atom, or an iodine atom; and the other symbols are the same as defined above.]

The reaction may be carried out according to the method disclosed in, for example, Synthesis, 2008, 28, 3707.

The Compound (MD1) is a commercially available compound or may be prepared according to the method disclosed in Synthesis, 2008, 28, 3707. The Compound (MD2) is a commercially available compound or may be prepared by using known method(s).

### Reference Production method E

A compound represented by formula (MB3) (hereinafter referred to as "Compound (MB3)") may be prepared according to the following scheme. 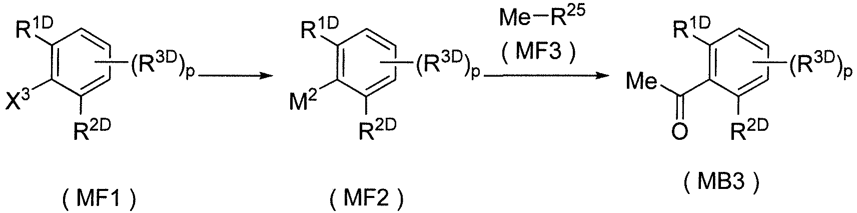 [wherein X³ represents a bromine atom or an iodine atom; M² represents Li or MgX³; R^{1D} and R^{2D} are identical to or different from each other, and each represent a C1-C6 chain hydrocarbon group optionally substituted with one or more substituent(s) selected from Group A, a C3-C8 alicyclic hydrocarbon group, a 3-8 membered nonaromatic heterocyclic group, a C6-C10 aryl group, a 5-10 membered aromatic heterocyclic group {wherein said C3-C8 alicyclic hydrocarbon group, said 3-8 membered nonaromatic heterocyclic group, said C6-C10 aryl group, and said 5-10 membered aromatic heterocyclic group may be optionally substituted with one or more substituent(s) selected from Group C}, a hydrogen atom, a nitro group, a cyano group, NR⁸R⁹, OR¹⁰, S(O) ₖR¹¹, or C(O)NR⁴R⁵; R^{3D} represents a C1-C6 chain hydrocarbon group optionally substituted with one or more substituent(s) selected from Group A, a C3-C8 alicyclic hydrocarbon group, a 3-8 membered nonaromatic heterocyclic group, a C6-C10 aryl group, a 5-10 membered aromatic heterocyclic group {wherein said C3-C8 alicyclic hydrocarbon group, said 3-8 membered nonaromatic heterocyclic group, said C6-C10 aryl group, and said 5-10 membered aromatic heterocyclic group may be optionally substituted with one or more substituent(s) selected from Group C} , a nitro group, a cyano group, NR⁸R⁹, OR¹⁰, S (O) ₖR¹¹, or C(O)NR⁴R⁵; R²⁵ represents a cyano group, a chlorocarbonyl group, a di(C1-C4alkyl)carbamoyl group, a N-methoxy-N-methylcarbamoyl group, a morpholinocarbonyl group, a pyrrol-1-ylcarbonyl group, a pyrazol-1-ylcarbonyl group, an imidazol-1-ylcarbonyl group, a benzotriazol-1-ylcarbonyl group, -C(O)OC(O)CH₃, or a C2-C4 alkoxycarbonyl group; and the other symbols are the same as defined above.]

A compound represented by formula (MF2) (hereinafter referred to as "Compound (MF2)") may be prepared by reacting a compound represented by formula (MF1) (hereinafter referred to as "Compound (MF1)") with a Grignard reagent such as butyllithium or isopropylmagnesium chloride.

The Compound (MB3) may be prepared by reacting the Compound (MF2) with a compound represented by formula (MF3) (hereinafter referred to as "Compound (MF3)").

These reactions may be carried out according to the method disclosed in, for example, CN 105461538 A.

The Compound (MF1) is a commercially available compound or may be prepared according to the method disclosed in, for example, J. Am. Chem. Soc., 2012, 134, 20597.

The Compound (MF3) is a commercially available compound or may be prepared by using known method(s).

### Reference Production method F

The Compound (B1) may be prepared by reacting a compound represented by formula (MG1) (hereinafter referred to as "Compound (MG1)") with the Compound (A2) in the presence of a base. 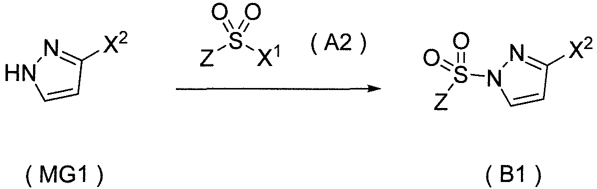 [wherein the symbols are the same as defined above.]

The reaction may be carried out according to the Production method A using the Compound (MG1) instead of the Compound (A1).

The Compound (MG1) is a commercially available compound or may be prepared according to the method disclosed in, for example, Medicinal Chemistry Letters, 2007, 17, 6274.

### Reference Production method G

The Compound (C1) may be prepared by reacting a compound represented by formula (MG2) (hereinafter referred to as "Compound (MG2)") with the Compound (A2) in the presence of a base. 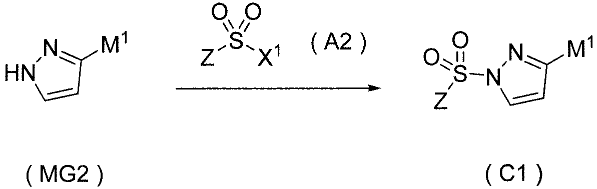 [wherein the symbols are the same as defined above.]

The reaction may be carried out according to the Reference Production method F using the Compound (MG2) instead of the Compound (MG1).

The Compound (MG2) is a commercially available compound or may be prepared according to the method disclosed in, for example, Medicinal Chemistry Letters, 2007, 17, 6274.

The Compound of the present invention may be mixed with or used in combination with one or more ingredient(s) selected from the group consisting of the following Group (a), Group (b), Group (c), and Group (d) (hereinafter referred to as "Present ingredient").

When the Compound of the present invention is mixed with or used in combination with the Present ingredient, they are used simultaneously, separately, or at time intervals with each other.

When the Compound of the present invention is used simultaneously with the Present ingredient, the Compound of the present invention and the Present ingredient may be contained in separate formulations or contained in one formulation.

One aspect of the present invention provides a composition comprising one or more ingredient(s) selected from the group consisting of Group (a), Group (b), Group (c), and Group (d) (i.e., Present ingredient), and the Compound of the present invention (hereinafter referred to as "Composition A").

Group (a) is a group consisting of acetylcholinesterase inhibitors (for example, carbamate insecticides and organophosphate insecticides), GABA-gated chloride channel blockers (for example, phenylpyrazole insecticides), sodium channel modulators (for example, pyrethroid insecticides), nicotinic acetylcholine receptor competitive modulators (for example, neonicotinoid insecticides), nicotinic acetylcholine receptor allosteric modulators, glutamate-gated chloride channel allosteric modulators (for example, macrolide insecticides), juvenile hormone mimics, multisite inhibitors, chordotonal organ TRPV channel modulators, mite growth inhibitors, microbial disruptors of insect midgut membranes, inhibitors of mitochondrial ATP synthase, uncouplers of oxidative phosphorylation, nicotinic acetylcholine receptor channel blockers (for example, nereistoxin insecticides), inhibitors of chitin biosynthesis, moulting disruptors, ecdysone receptor agonists, octopamine receptor agonists, mitochondrial complexes I, II, III, and IV electron transport inhibitors, voltage-dependent sodium channel blockers, inhibitors of acetyl CoA carboxylase, ryanodine receptor modulators (for example, diamide insecticides), chordotonal organ modulators, and microbial insecticides, and other insecticidal active ingredients, miticidal active ingredients, and nematicidal active ingredients. These ingredients are described in the classification on the basis of action mechanism by IRAC.

Group (b) is a group consisting of nucleic acids synthesis inhibitors (for example, phenylamide fungicides and acylamino acid fungicides), cell division and cytoskeleton inhibitors (for example, MBC fungicides), respiration inhibitors (for example, QoI fungicides and QiI fungicides), amino acids synthesis and protein synthesis inhibitors (for example, anilino-pyrimidine fungicides), signal transduction inhibitors, lipid synthesis and membrane synthesis inhibitors, sterol biosynthesis inhibitors (for example, DMI fungicides such as triazole fungicides), cell wall biosynthesis inhibitors, melanin synthesis inhibitors, plant defense inducers, fungicides with multi-site contact activity, microbial fungicides, and other fungicidal active ingredients. These ingredients are described in the classification on the basis of action mechanism by FRAC.

Group (c) is a group of plant growth regulatory ingredients (including mycorrhizal fungi and root nodule bacteria).

Group (d) is a group of repellent ingredients.

Hereinafter, examples of the combination of the Present ingredient and the Compound of the present invention are described. For example, "alanycarb + SX" indicates a combination of alanycarb and SX.

The abbreviation of "SX" indicates any one of the Compound of the present invention selected from the Compound groups SX1 to SX145. Also, all of the following Present ingredient are known ingredients, and may be obtained from commercially available formulations, or may be prepared by known methods. When the Present ingredient is a microorganism, it may also be available from a bacterial authority depository. Further, the number in parentheses represents the CAS RN (registered trademark).

Combinations of the Present ingredient in the above Group (a) and the Compound of the present invention:
abamectin + SX, acephate + SX, acequinocyl + SX, acetamiprid + SX, acetoprole + SX, acrinathrin + SX, acynonapyr + SX, afidopyropen + SX, afoxolaner + SX, alanycarb + SX, aldicarb + SX, allethrin + SX, alphacypermethrin + SX, alpha-endosulfan + SX, aluminium phosphide + SX, amitraz + SX, azadirachtin + SX, azamethiphos + SX, azinphos-ethyl + SX, azinphos-methyl + SX, azocyclotin + SX, bark of Celastrus angulatus + SX, bendiocarb + SX, benfluthrin + SX, benfuracarb + SX, bensultap + SX, benzoximate + SX, benzpyrimoxan + SX, betacyfluthrin + SX, beta-cypermethrin + SX, bifenazate + SX, bifenthrin + SX, bioallethrin + SX, bioresmethrin + SX, bistrifluron + SX, borax + SX, boric acid + SX, broflanilide + SX, bromopropylate + SX, buprofezin + SX, butocarboxim + SX, butoxycarboxim + SX, cadusafos + SX, calcium phosphide + SX, carbaryl + SX, carbofuran + SX, carbosulfan + SX, cartap hydrochloride + SX, cartap + SX, chinomethionat + SX, chlorantraniliprole + SX, chlordane + SX, chlorethoxyfos + SX, chlorfenapyr + SX, chlorfenvinphos + SX, chlorfluazuron + SX, chlormephos + SX, chloropicrin + SX, chlorpyrifos + SX, chlorpyrifos-methyl + SX, chromafenozide + SX, clofentezine + SX, clothianidin + SX, concanamycin A + SX, coumaphos + SX, cryolite + SX, cyanophos + SX, cyantraniliprole + SX, cyclaniliprole + SX, cyclobutrifluram + SX, cycloprothrin + SX, cycloxaprid + SX, cyenopyrafen + SX, cyetpyrafen + SX, cyflumetofen + SX, cyfluthrin + SX, cyhalodiamide + SX, cyhalothrin + SX, cyhexatin + SX, cypermethrin + SX, cyphenothrin + SX, cyproflanilide + SX, cyromazine + SX, dazomet + SX, deltamethrin + SX, demeton-S-methyl + SX, diafenthiuron + SX, diazinon + SX, dichlorvos + SX, dicloromezotiaz + SX, dicofol + SX, dicrotophos + SX, diflovidazin + SX, diflubenzuron + SX, dimefluthrin + SX, dimethoate + SX, dimethylvinphos + SX, dimpropyridaz + SX, dinotefuran + SX, disodium octaborate + SX, disulfoton + SX, DNOC (2-methyl-4,6-dinitrophenol) + SX, doramectin + SX, dried leaves of Dryopteris filix-mas + SX, emamectin-benzoate + SX, empenthrin + SX, endosulfan + SX, EPN (O-ethyl O-(4-nitrophenyl) phenylphosphonothioate) + SX, epsilonmetofluthrin + SX, epsilon-momfluorothrin + SX, esfenvalerate + SX, ethiofencarb + SX, ethion + SX, ethiprole + SX, ethoprophos + SX, etofenprox + SX, etoxazole + SX, extract of Artemisia absinthium + SX, extract of Azadirachta indica + SX, extract of Cassia nigricans + SX, extract of clitoria ternatea + SX, extract of Symphytum officinale + SX, extracts of Chenopodium ambrosioides + SX, extract of Tanacetum vulgare + SX, extract of Urtica dioica + SX, extract of Viscum album + SX, famphur + SX, fenamiphos + SX, fenazaquin + SX, fenbutatin oxide + SX, fenitrothion + SX, fenmezoditiaz + SX, fenobucarb + SX, fenoxycarb + SX, fenpropathrin + SX, fenpyroximate + SX, fenthion + SX, fenvalerate + SX, fipronil + SX, flometoquin + SX, flonicamid + SX, fluacrypyrim + SX, fluazaindolizine + SX, fluazuron + SX, flubendiamide + SX, fluchlordiniliprole + SX, flucycloxuron + SX, flucythrinate + SX, fluensulfone + SX, flufenoprox + SX, flufenoxuron + SX, flufiprole + SX, flumethrin + SX, flupentiofenox + SX, flupyradifurone + SX, flupyrimin + SX, flupyroxystrobin + SX, fluralaner + SX, fluvalinate + SX, fluxametamide + SX, formetanate + SX, fosthiazate + SX, furamethrin + SX, furathiocarb + SX, gamma-cyhalothrin + SX, GS-omega/kappa HXTX-Hvla peptide + SX, halfenprox + SX, halofenozide + SX, heptafluthrin + SX, heptenophos + SX, hexaflumuron + SX, hexythiazox + SX, potassium salt of hop beta acid + SX, hydramethylnon + SX, hydroprene + SX, imicyafos + SX, imidacloprid + SX, imidaclothiz + SX, imiprothrin + SX, indazapyroxamet + SX, indoxacarb + SX, isocycloseram + SX, isofenphos + SX, isoprocarb + SX, isopropyl-O-(methoxyaminothiophosphoryl) salicylate + SX, isoxathion + SX, ivermectin + SX, kadethrin + SX, kappatefluthrin + SX, kappa-bifenthrin + SX, kinoprene + SX, lambda-cyhalothrin + SX, ledprona + SX, lenoremycin + SX, lepimectin + SX, lime sulfur + SX, lotilaner + SX, lufenuron + SX, machine oil + SX, malathion + SX, mecarbam + SX, meperfluthrin + SX, metaflumizone + SX, metam + SX, methamidophos + SX, methidathion + SX, methiocarb + SX, methomyl + SX, methoprene + SX, methoxychlor + SX, methoxyfenozide + SX, methyl bromide + SX, metofluthrin + SX, metolcarb + SX, metoxadiazone + SX, mevinphos + SX, milbemectin + SX, milbemycin oxime + SX, mivorilaner + SX, modoflaner + SX, momfluorothrin + SX, monocrotophos + SX, moxidectin + SX, naled + SX, nicofluprole + SX, nicotine + SX, nicotine-sulfate + SX, nitenpyram + SX, novaluron + SX, noviflumuron + SX, oil of the seeds of Chenopodium anthelminticum + SX, omethoate + SX, oxamyl + SX, oxazosulfyl + SX, oxydemeton-methyl + SX, parathion + SX, parathion-methyl + SX, permethrin + SX, phenothrin + SX, phenthoate + SX, phorate + SX, phosalone + SX, phosmet + SX, phosphamidon + SX, phosphine + SX, phoxim + SX, pirimicarb + SX, pirimiphos-methyl + SX, prallethrin + SX, profenofos + SX, profluthrin + SX, propargite + SX, propetamphos + SX, propoxur + SX, propylene glycol alginate + SX, prothiofos + SX, pyflubumide + SX, pymetrozine + SX, pyraclofos + SX, pyrethrins + SX, pyridaben + SX, pyridalyl + SX, pyridaphenthion + SX, pyrifluquinazone + SX, pyrimidifen + SX, pyriminostrobin + SX, pyriprole + SX, pyriproxyfen + SX, quinalphos + SX, resmethrin + SX, rotenone + SX, ryanodine + SX, sarolaner + SX, selamectin + SX, sigma-cypermethrin + SX, silafluofen + SX, sodium borate + SX, sodium metaborate + SX, spidoxamat + SX, spinetoram + SX, spinosad + SX, spirobudifen + SX, spirodiclofen + SX, spiromesifen + SX, spiropidion + SX, spirotetramat + SX, sulfiflumin + SX, sulfluramid + SX, sulfotep + SX, sulfoxaflor + SX, sulfur + SX, sulfuryl fluoride + SX, tartar emetic + SX, tau-fluvalinate + SX, tebufenozide + SX, tebufenpyrad + SX, tebupirimfos + SX, teflubenzuron + SX, tefluthrin + SX, temephos + SX, terbufos + SX, terpene constituents of the extract of chenopodium ambrosioides near ambrosioides + SX, tetrachlorantraniliprole + SX, tetrachlorvinphos + SX, tetradifon + SX, tetramethrin + SX, tetramethylfluthrin + SX, tetraniliprole + SX, theta-cypermethrin + SX, thiacloprid + SX, thiamethoxam + SX, thiocyclam + SX, thiodicarb + SX, thiofanox + SX, thiometon + SX, thiosultap-disodium + SX, thiosultap-monosodium + SX, tigolaner + SX, tiorantraniliprole + SX, tioxazafen + SX, tolfenpyrad + SX, tralomethrin + SX, transfluthrin + SX, triazamate + SX, triazophos + SX, trichlorfon + SX, trifluenfuronate + SX, triflumezopyrim + SX, triflumuron + SX, trimethacarb + SX, tyclopyrazoflor + SX, umifoxolaner + SX, vamidothion + SX, wood extract of Quassia amara + SX, XMC (3,5-dimethylphenyl N-methylcarbamate) + SX, xylylcarb + SX, zeta-cypermethrin + SX, zinc phosphide + SX, 4-[5-(3,5-dichlorophenyl)-5-(trifluoromethyl)-4,5-dihydro-1,2-oxazol-3-yl]-2-methyl-N-(1-oxothietan-3-yl)benzamide (1241050-20-3) + SX, 3-methoxy-N-(5-{5-(trifluoromethyl)-5-[3-(trifluoromethyl)phenyl]-4,5-dihydro-1,2-oxazol-3-yl}indan-1-yl)propanamide (1118626-57-5) + SX, N-{4-chloro-3-[(1-cyanocyclopropyl)carbamoyl]phenyl}-1-methyl-4-(methanesulfonyl)-3-(1,1,2,2,2-pentafluoroethyl)-1H-pyrazole-3-carboxamide (1400768-21-9) + SX, N-[3-chloro-1-(pyridin-3-yl)-1H-pyrazol-4-yl]-2-(methanesulfonyl)propanamide (2396747-83-2) + SX, N-[4-chloro-2-(pyridin-3-yl)-1,3-thiazol-5-yl]-N-ethyl-3-(methanesulfonyl)propanamide + SX, 1,4-dimethyl-2-[2-(pyridin-3-yl)-2H-indazol-5-yl]-1,2,4-triazolidine-3,5-dione (2171099-09-3) + SX, 2-isopropyl-5-[(3,4,4-trifluoro-3-buten-1-yl)sulfonyl]-1,3,4-thiadiazole (2058052-95-0) + SX, N-({2-fluoro-4-[(2S,3S)-2-hydroxy-3-(3,4,5-trichlorophenyl)-3-(trifluoromethyl)pyrrolidin-1-yl]phenyl}methyl)cyclopropanecarboxamide + SX, 7-fluoro-N-[1-(methylsulfanyl)-2-methylpropan-2-yl]-2-(pyridin-3-yl)-2H-indazole-4-carboxamide + SX, 7-fluoro-N-[1-(methanesulfinyl)-2-methylpropan-2-yl]-2-(pyridin-3-yl)-2H-indazole-4-carboxamide + SX, 7-fluoro-N-[1-(methanesulfonyl)-2-methylpropan-2-yl]-2-(pyridin-3-yl)-2H-indazole-4-carboxamide + SX, N-[1-(difluoromethyl)cyclopropyl]-2-(pyridin-3-yl)-2H-indazole-4-carboxamide + SX, 2,9-dihydro-9-(methoxymethyl)-2-(pyridin-3-yl)-10H-pyrazolo[3,4-f]pyrido[2,3-b][1,4]oxazepin-10-one (2607927-97-7) + SX, BT crop protein Cry1Ab + SX, BT crop protein Cry1Ac + SX, BT crop protein CrylFa + SX, BT crop protein Cry1A.105 + SX, BT crop protein Cry2Ab + SX, BT crop protein Vip3A + SX, BT crop protein mCry3A + SX, BT crop protein Cry3Ab + SX, BT crop protein Cry3Bb + SX, BT crop protein Cry34Ab1/Cry35Ab1 + SX, Adoxophyes orana granulosis virus strain BV-0001 + SX, Anticarsia gemmatalis mNPV + SX, Autographa californica mNPV + SX, Cydia pomonella GV strain V15 + SX, Cydia pomonella GV strain V22 + SX, Cryptophlebia leucotreta GV + SX, Dendrolimus punctatus cypovirus + SX, Helicoverpa armigera NPV strain BV-0003 + SX, Helicoverpa zea NPV + SX, Lymantria dispar NPV + SX, Mamestra brassicae NPV + SX, Mamestra configurata NPV + SX, Neodiprion abietis NPV + SX, Neodiprion lecontei NPV + SX, Neodiprion sertifer NPV + SX, Nosema locustae + SX, Orgyia pseudotsugata NPV + SX, Pieris rapae GV + SX, Plodia interpunctella GV + SX, Spodoptera exigua mNPV + SX, Spodoptera littoralis mNPV + SX, Spodoptera litura NPV + SX, Arthrobotrys dactyloides + SX, Bacillus firmus strain GB-126 + SX, Bacillus firmus strain I-1582 + SX, Bacillus firmus strain NCIM2637 + SX, Bacillus megaterium + SX, Bacillus sp. strain AQ175 + SX, Bacillus sp. strain AQ177 + SX, Bacillus sp. strain AQ178 + SX, Bacillus sphaericus strain 2362 serotype H5a5b + SX, Bacillus sphaericus strain ABTS1743 + SX, Bacillus thuringiensis strain AQ52 + SX, Bacillus thuringiensis strain BD#32 + SX, Bacillus thuringiensis strain CR-371 + SX, Bacillus thuringiensis subsp. Aizawai strain ABTS-1857 + SX, Bacillus thuringiensis subsp. Aizawai strain AM65-52 + SX, Bacillus thuringiensis subsp. Aizawai strain GC-91 + SX, Bacillus thuringiensis subsp. Aizawai strain NB200 + SX, Bacillus thuringiensis subsp. Aizawai Serotype strain H-7 + SX, Bacillus thuringiensis subsp. Kurstaki strain ABTS351 + SX, Bacillus thuringiensis subsp. Kurstaki strain BMP123 + SX, Bacillus thuringiensis subsp. Kurstaki strain CCT1306 + SX, Bacillus thuringiensis subsp. Kurstaki strain EG2348 + SX, Bacillus thuringiensis subsp. Kurstaki strain EG7841 + SX, Bacillus thuringiensis subsp. Kurstaki strain EVB113-19 + SX, Bacillus thuringiensis subsp. Kurstaki strain F810 + SX, Bacillus thuringiensis subsp. Kurstaki strain HD-1 + SX, Bacillus thuringiensis subsp. Kurstaki strain PB54 + SX, Bacillus thuringiensis subsp. Kurstaki strain SA-11 + SX, Bacillus thuringiensis subsp. Kurstaki strain SA-12 + SX, Bacillus thuringiensis subsp. Tenebriosis strain NB176 + SX, Bacillus thuringiensis subsp. Thuringiensis strain MPPL002 + SX, Bacillus thuringiensis subsp. morrisoni + SX, Bacillus thuringiensis var. colmeri + SX, Bacillus thuringiensis var. darmstadiensis strain 24-91 + SX, Bacillus thuringiensis var. dendrolimus + SX, Bacillus thuringiensis var. galleriae + SX, Bacillus thuringiensis var. israelensis strain BMP144 + SX, Bacillus thuringiensis var. israelensis serotype strain H-14 + SX, Bacillus thuringiensis var. japonensis strain buibui + SX, Bacillus thuringiensis var. san diego strain M-7 + SX, Bacillus thuringiensis var. 7216 + SX, Bacillus thuringiensis var. aegypti + SX, Bacillus thuringiensis var. T36 + SX, Beauveria bassiana strain ANT-03 + SX, Beauveria bassiana strain ATCC74040 + SX, Beauveria bassiana strain GHA + SX, Beauveria brongniartii + SX, Burkholderia rinojensis strain A396 + SX, Chromobacterium subtsugae strain PRAA4-1T + SX, Dactyllela ellipsospora + SX, Dectylaria thaumasia + SX, Hirsutella minnesotensis + SX, Hirsutella rhossiliensis + SX, Hirsutella thompsonii + SX, Lagenidium giganteum + SX, Lecanicillium lecanii strain KV01 + SX, Lecanicillium lecanii conidia of strain DAOM198499 + SX, Lecanicillium lecanii conidia of strain DAOM216596 + SX, Lecanicillium muscarium strain Ve6 + SX, Metarhizium anisopliae strain F52 + SX, Metarhizium anisopliae var. acridum + SX, Metarhizium anisopliae var. anisopliae BIPESCO 5/F52 + SX, Metarhizium flavoviride + SX, Monacrosporium phymatopagum + SX, Paecilomyces fumosoroseus Apopka strain 97 + SX, Paecilomyces lilacinus strain 251 + SX, Paecilomyces tenuipes strain T1 + SX, Paenibacillus popilliae + SX, Pasteuria nishizawae strain Pn1 + SX, Pasteuria penetrans + SX, Pasteuria usgae + SX, Pasteuria thornei + SX, Serratia entomophila + SX, Verticillium chlamydosporium + SX, Verticillium lecani strain NCIM1312 + SX, Wolbachia pipientis + SX.

Combinations of the Present ingredient in the above Group (b) and the Compound of the present invention:
acibenzolar-S-methyl + SX, aldimorph + SX, ametoctradin + SX, aminopyrifen + SX, amisulbrom + SX, anilazine + SX, azaconazole + SX, azoxystrobin + SX, basic copper sulfate + SX, benalaxyl + SX, benalaxyl-M + SX, benodanil + SX, benomyl + SX, benthiavalicarb + SX, benthiavalicarb-isopropyl + SX, benzovindiflupyr + SX, binapacryl + SX, biphenyl + SX, bitertanol + SX, bixafen + SX, blasticidin-S + SX, Bordeaux mixture + SX, boscalid + SX, bromothalonil + SX, bromuconazole + SX, bupirimate + SX, captafol + SX, captan + SX, carbendazim + SX, carboxin + SX, carpropamid + SX, chinomethionat + SX, chitin + SX, chloroinconazide + SX, chloroneb + SX, chlorothalonil + SX, chlozolinate + SX, colletochlorin B + SX, copper(II) acetate + SX, copper(II) hydroxide + SX, copper oxychloride + SX, copper(II) sulfate + SX, coumoxystrobin + SX, cyazofamid + SX, cyflufenamid + SX, cymoxanil + SX, cyproconazole + SX, cyprodinil + SX, dichlobentiazox + SX, dichlofluanid + SX, diclocymet + SX, diclomezine + SX, dicloran + SX, diethofencarb + SX, difenoconazole + SX, diflumetorim + SX, dimethachlone + SX, dimethirimol + SX, dimethomorph + SX, dimoxystrobin + SX, diniconazole + SX, diniconazole-M + SX, dinocap + SX, dipotassium hydrogenphosphite + SX, dipymetitrone + SX, dithianon + SX, dodecylbenzenesulphonic acid bisethylenediamine copper(II) salt + SX, dodemorph + SX, dodine + SX, edifenphos + SX, enoxastrobin + SX, epoxiconazole + SX, etaconazole + SX, ethaboxam + SX, ethirimol + SX, etridiazole + SX, extract of Melaleuca alternifolia + SX, extract of Reynoutria sachalinensis + SX, extract of the cotyledons of lupine plantlets ("BLAD") + SX, extract of Allium sativum + SX, extract of Equisetum arvense + SX, extract of Tropaeolum majus + SX, famoxadone + SX, fenamidone + SX, fenaminstrobin + SX, fenarimol + SX, fenbuconazole + SX, fenfuram + SX, fenhexamid + SX, fenoxanil + SX, fenpiclonil + SX, fenpicoxamid + SX, fenpropidin + SX, fenpropimorph + SX, fenpyrazamine + SX, fentin acetate + SX, fentin chloride + SX, fentin hydroxide + SX, ferbam + SX, ferimzone + SX, florylpicoxamid + SX, fluazinam + SX, flubeneteram + SX, fludioxonil + SX, flufenoxadiazam + SX, flufenoxystrobin + SX, fluindapyr + SX, flumetylsulforim + SX, flumorph + SX, fluopicolide + SX, fluopyram + SX, fluopimomide + SX, fluoroimide + SX, fluoxapiprolin +SX, fluoxastrobin+ SX, fluoxytioconazole + SX, fluquinconazole + SX, flusilazole + SX, flusulfamide + SX, flutianil + SX, flutolanil + SX, flutriafol + SX, fluxapyroxad + SX, folpet + SX, fosetyl + SX, fosetyl-aluminium + SX, fuberidazole + SX, furalaxyl + SX, furametpyr + SX, guazatine + SX, hexaconazole + SX, hymexazole + SX, imazalil + SX, imibenconazole + SX, iminoctadine + SX, iminoctadine triacetate + SX, inpyrfluxam + SX, iodocarb + SX, ipconazole + SX, ipfentrifluconazole + SX, ipflufenoquin + SX, iprobenfos + SX, iprodione + SX, iprovalicarb + SX, isofetamid + SX, isoflucypram + SX, isoprothiolane + SX, isopyrazam + SX, isotianil + SX, kasugamycin + SX, kresoxim-methyl + SX, laminarin + SX, leaves and bark of Quercus + SX, mancozeb + SX, mandestrobin + SX, mandipropamid + SX, maneb + SX, mefentrifluconazole + SX, mepanipyrim + SX, mepronil + SX, meptyldinocap + SX, metalaxyl + SX, metalaxyl-M + SX, metarylpicoxamid + SX, metconazole + SX, methasulfocarb + SX, metiram + SX, metominostrobin + SX, metrafenone + SX, metyltetraprole + SX, myclobutanil + SX, naftifine + SX, nuarimol + SX, octhilinone + SX, ofurace + SX, orysastrobin + SX, oxadixyl + SX, oxathiapiprolin + SX, oxine-copper + SX, oxolinic acid + SX, oxpoconazole + SX, oxpoconazole fumarate + SX, oxycarboxin + SX, oxytetracycline + SX, pefurazoate + SX, penconazole + SX, pencycuron + SX, penflufen + SX, penthiopyrad + SX, phenamacril + SX, phosphorous acid + SX, phthalide + SX, picarbutrazox + SX, picoxystrobin + SX, piperalin + SX, polyoxins + SX, potassium hydrogencarbonate + SX, potassium dihydrogenphosphite + SX, probenazole + SX, prochloraz + SX, procymidone + SX, propamidine + SX, propamocarb + SX, propiconazole + SX, propineb + SX, proquinazid + SX, prothiocarb + SX, prothioconazole + SX, pydiflumetofen + SX, pyraclostrobin + SX, pyrametostrobin + SX, pyraoxystrobin + SX, pyrapropoyne + SX, pyraziflumid + SX, pyrazophos + SX, pyribencarb + SX, pyributicarb + SX, pyridachlometyl + SX, pyrifenox + SX, pyrimethanil + SX, pyrimorph + SX, pyriofenone + SX, pyrisoxazole + SX, pyroquilon + SX, Quillaja extract + SX, quinconazole + SX, quinofumelin + SX, quinoxyfen + SX, quintozene + SX, Saponins of Chenopodium quinoa + SX, seboctylamine + SX, sedaxane + SX, silthiofam + SX, simeconazole + SX, sodium hydrogencarbonate + SX, spiroxamine + SX, streptomycin + SX, sulfur + SX, tebuconazole + SX, tebufloquin + SX, teclofthalam + SX, tecnazene + SX, terbinafine + SX, tetraconazole + SX, thiabendazole + SX, thifluzamide + SX, thiophanate + SX, thiophanate-methyl + SX, thiram + SX, thymol + SX, tiadinil + SX, tolclofos-methyl + SX, tolfenpyrad + SX, tolprocarb + SX, tolylfluanid + SX, triadimefon + SX, triadimenol + SX, triazoxide + SX, triclopyricarb + SX, tricyclazole + SX, tridemorph + SX, trifloxystrobin + SX, triflumizole + SX, triforine + SX, triticonazole + SX, validamycin + SX, valifenalate + SX, vinclozolin + SX, yellow mustard powder + SX, zinc thiazole + SX, zineb + SX, ziram + SX, zoxamide + SX, N'-[4-({3-[(4-chlorophenyl)methyl]-1,2,4-thiadiazol-5-yl}oxy)-2,5-dimethylphenyl]-N-ethyl-N-methylmethanimidamide (1202781-91-6) + SX, N'-{4-[(4,5-dichlorothiazol-2-yl)oxy]-2,5-dimethylphenyl}-N-ethyl-N-methylmethanimidamide (929908-57-6) + SX, N'-(2,5-dimethyl-4-phenoxyphenyl)-N-ethyl-N-methylmethanimidamide (1052688-31-9) + SX, N'-[5-chloro-4-(2-fluorophenoxy)-2-methylphenyl]-N-ethyl-N-methylmethanimidamide (2055589-28-9) + SX, N'-[2-chloro-4-(2-fluorophenoxy)-5-methylphenyl]-N-ethyl-N-methylmethanimidamide (2055756-21-1) + SX, N'-(2-chloro-4-phenoxy-5-methylphenyl)-N-ethyl-N-methylmethanimidamide (2062599-39-5) + SX, N'-[4-(1-hydroxy-1-phenyl-2,2,2-trifluoroethyl)-2-methyl-5-methoxyphenyl]-N-isopropyl-N-methylmethanimidamide (2101814-55-3) + SX, N'-[5-bromo-6-(1-methyl-2-propoxyethoxy)-2-methylpyridin-3-yl]-N-ethyl-N-methylmethanimidamide (1817828-69-5) + SX, 4-(2-bromo-4-fluorophenyl)-N-(2-chloro-6-fluorophenyl)-1,3-dimethyl-1H-pyrazol-5-amine (1362477-26-6) + SX, 2-[6-(3-fluoro-4-methoxyphenyl)-5-methylpyridin-2-yl]quinazoline (1257056-97-5) + SX, ethyl (2Z)-3-amino-2-cyano-3-phenylacrylate (39491-78-6) + SX, N-[(2-chlorothiazol-5-yl)methyl]-N-ethyl-6-methoxy-3-nitropyridin-2-amine (1446247-98-8) + SX, 5-(4-chlorobenzyl)-2-(chloromethyl)-2-methyl-1-(1H-1,2,4-triazol-1-ylmethyl)cyclopentan-1-ol (1394057-11-4) + SX, (1R, 2S, 5S)-5-(4-chlorobenzyl)-2-(chloromethyl)-2-methyl-1-(1H-1,2,4-triazol-1-ylmethyl)cyclopentan-1-ol (1801930-06-2) + SX, (1S, 2R, 5R)-5-(4-chlorobenzyl)-2-(chloromethyl) -2-methyl-1-(1H-1,2,4-triazol-1-ylmethyl)cyclopentan-1-ol (1801930-07-3) + SX, 2-(chloromethyl)-5-(4-fluorobenzyl)-2-methyl-1-(1H-1,2,4-triazol-1-ylmethyl)cyclopentan-1-ol (1394057-13-6) + SX, (1R, 2S, 5S)-2-(chloromethyl)-5-(4-fluorobenzyl)-2-methyl-1-(1H-1,2,4-triazol-1-ylmethyl)cyclopentan-1-ol (1801930-08-4) + SX, (1S, 2R, 5R)-2-(chloromethyl)-5-(4-fluorobenzyl)-2-methyl-1-(1H-1,2,4-triazol-1-ylmethyl)cyclopentan-1-ol (1801930-09-5) + SX, methyl 3-[(4-chlorophenyl)methyl]-2-hydroxy-1-methyl-2-(1H-1,2,4-triazol-1-ylmethyl)cyclopentan-1-carboxylate (1791398-02-1) + SX, 1-(2,4-difluorophenyl)-2-(1H-1,2,4-triazol-1-yl)-1-[1-(4-bromo-2,6-difluorophenoxy)cyclopropyl]ethanol (2019215-86-0) + SX, 1-(2,4-difluorophenyl)-2-(1H-1,2,4-triazol-1-yl)-1-[1-(4-chloro-2,6-difluorophenoxy)cyclopropyl]ethanol (2019215-84-8) + SX, 1-[2-(1-chlorocyclopropyl)-3-(2-fluorophenyl)-2-hydroxypropyl]-1H-imidazole-5-carbonitrile (2018316-13-5) + SX, 1-[2-(1-chlorocyclopropyl)-3-(2,3-difluorophenyl)-2-hydroxypropyl]-1H-imidazole-5-carbonitrile (2018317-25-2) + SX, 2-[6-(4-bromophenoxy)-2-(trifluoromethyl)pyridin-3-yl]-1-(1H-1,2,4-triazol-1-yl)propan-2-ol (2082661-43-4) + SX, 2-[6-(4-chlorophenoxy)-2-(trifluoromethyl)pyridin-3-yl]-1-(1H-1,2,4-triazol-1-yl)propan-2-ol (2082660-27-1) + SX, methyl ({2-methyl-5-[1-(4-methoxy-2-methylphenyl)-1H-pyrazol-3-yl]phenyl}methyl)carbamate (1605879-98-8) + SX, 2-(difluoromethyl)-N-[1,1,3-trimethyl-2,3-dihydro-1H-inden-4-yl]pyridine-3-carboxamide (1616239-21-4) + SX, 2-(difluoromethyl)-N-[3-ethyl-1,1-dimethyl-2,3-dihydro-1H-inden-4-yl]pyridine-3-carboxamide (1847460-02-9) + SX, 2-(difluoromethyl)-N-[3-propyl-1,1-dimethyl-2,3-dihydro-1H-inden-4-yl]pyridine-3-carboxamide (1847460-05-2) + SX, (2E,3Z)-5-{[1-(4-chlorophenyl)-1H-pyrazol-3-yl]oxy}-2-(methoxyimino)-N,3-dimethylpent-3-enamide (1445331-27-0) + SX, (2E,3Z)-5-{[1-(2,4-dichlorophenyl)-1H-pyrazol-3-yl]oxy}-2-(methoxyimino)-N,3-dimethylpent-3-enamide (1445331-54-3) + SX, 5-chloro-4-({2-[6-(4-chlorophenoxy)pyridin-3-yl]ethyl}amino)-6-methylpyrimidine (1605340-92-8) + SX, N-(1-benzyl-1,3-dimethylbutyl)-8-fluoroquinoline-3-carboxamide (2132414-04-9) + SX, N-(1-benzyl-3,3,3-trifluoro-1-methylpropyl)-8-fluoroquinoline-3-carboxamide (2132414-00-5) + SX, 4,4-dimethyl-2-({4-[5-(trifluoromethyl)-1,2,4-oxadiazol-3-yl]phenyl}methyl)isoxazolidin-3-one (2098918-25-1) + SX, 5,5-dimethyl-2-({4-[5-(trifluoromethyl)-1,2,4-oxadiazol-3-yl]phenyl}methyl)isoxazolidin-3-one (2098918-26-2) + SX, N-ethyl-2-methyl-N-(14-[5-(trifluoromethyl)-1,2,4-oxadiazol-3-yl]phenyl}methyl)propanamide + SX, N,2-dimethoxy-N-({4-[5-(trifluoromethyl)-1,2,4-oxadiazol-3-yl]phenyl}methyl)propanamide + SX, N-methoxy-N-({4-[5-(trifluoromethyl)-1,2,4-oxadiazol-3-yl]phenyl}methyl)cyclopropanecarboxamide + SX, N-methoxy-N'-methyl-N-({4-[5-(trifluoromethyl)-1,2,4-oxadiazol-3-yl]phenyl}methyl)urea + SX, N'-ethyl-N-methoxy-N-({4-[5-(trifluoromethyl)-1,2,4-oxadiazol-3-yl]phenyl}methyl)urea + SX, N,N'-dimethoxy-N-({4-[5-(trifluoromethyl)-1,2,4-oxadiazol-3-yl]phenyl}methyl)urea + SX, N-acetyl-2-(ethanesulfonyl)-N-[2-(methoxycarbonyl)-4-(trifluoromethoxy)phenyl]-4-(trifluoromethyl)benzamide (2043675-28-9) + SX, 3-(4-bromo-7-fluoroindol-1-yl)butan-2-yl N-[(3-hydroxy-4-methoxypyridin-2-yl)carbonyl]-L-alaninate + SX, 3-(7-bromoindol-1-yl)butan-2-yl N-[(3-hydroxy-4-methoxypyridin-2-yl)carbonyl]-L-alaninate + SX, 3-(7-bromo-4-fluoroindol-1-yl)butan-2-yl N-[(3-hydroxy-4-methoxypyridin-2-yl)carbonyl]-L-alaninate + SX, 3-(3,5-dichloropyridin-2-yl)butan-2-yl N-[(3-hydroxy-4-methoxypyridin-2-yl)carbonyl]-L-alaninate + SX, 3-(3,5-dichloropyridin-2-yl)butan-2-yl N-{[3-(acetoxymethoxy)-4-methoxypyridin-2-yl]carbonyl}-L-alaninate + SX, (1S)-1-[1-(naphthalen-1-yl)cyclopropyl]ethyl N-[(3-hydroxy-4-methoxypyridin-2-yl)carbonyl]-L-alaninate + SX, (1S)-1-[1-(naphthalen-1-yl)cyclopropyl]ethyl N-[(3-acetoxy-4-methoxypyridin-2-yl)carbonyl]-L-alaninate + SX, (1S)-1-[1-(naphthalen-1-yl)cyclopropyl]ethyl N-{[3-(acetoxymethoxy)-4-methoxypyridin-2-yl]carbonyl}-L-alaninate + SX, N-({4-[5-(trifluoromethyl)-1,2,4-oxadiazol-3-yl]phenyl}methyl)cyclopropanecarboxamide + SX, N-allyl-N-({4-[5-(trifluoromethyl)-1,2,4-oxadiazol-3-yl]phenyl}methyl)acetamide + SX, N-allyl-N-({4-[5-(trifluoromethyl)-1,2,4-oxadiazol-3-yl]phenyl}methyl)propanamide + SX, N-({4-[5-(trifluoromethyl)-1,2,4-oxadiazol-3-yl]phenyl}methyl)propanamide + SX, 3,3,3-trifluoro-N-({2-fluoro-4-[5-(trifluoromethyl)-1,2,4-oxadiazol-3-yl]phenyl}methyl)propanamide + SX, 3,3,3-trifluoro-N-({3-fluoro-4-[5-(trifluoromethyl)-1,2,4-oxadiazol-3-yl]phenyl}methyl)propanamide + SX, 3,3,3-trifluoro-N-({2,3-difluoro-4-[5-(trifluoromethyl)-1,2,4-oxadiazol-3-yl]phenyl}methyl)propanamide + SX, N-({2,3-difluoro-4-[5-(trifluoromethyl)-1,2,4-oxadiazol-3-yl]phenyl}methyl)butanamide + SX, N-methoxy-N-methyl-N'-({ 4-[5-(trifluoromethyl)-1,2,4-oxadiazol-3-yl]phenyl}methyl)urea + SX, N,N-diethyl-N'-({ 4-[5-(trifluoromethyl)-1,2,4-oxadiazol-3-yl]phenyl}methyl)urea + SX, N-methyl-N'-({ 4-[5-(trifluoromethyl)-1,2,4-oxadiazol-3-yl]phenyl}methyl)urea + SX, 1-({4-[5-(trifluoromethyl)-1,2,4-oxadiazol-3-yl]phenyl}methyl)pyrrolidin-2-one + SX, 1-({4-[5-(trifluoromethyl)-1,2,4-oxadiazol-3-yl]phenyl}methyl)piperidin-2-one + SX, 4-({4-[5-(trifluoromethyl)-1,2,4-oxadiazol-3-yl]phenyl}methyl)morpholin-3-one + SX, 2-({4-[5-(trifluoromethyl)-1,2,4-oxadiazol-3-yl]phenyl}methyl)isoxazolidin-3-one + SX, 3,3-dimethyl-1-({4-[5-(trifluoromethyl)-1,2,4-oxadiazol-3-yl]phenyl}methyl)piperidin-2-one + SX, 2-({4-[5-(trifluoromethyl)-1,2,4-oxadiazol-3-yl]phenyl}methyl)-1,2-oxazinan-3-one + SX, 1-({3-fluoro-4-[5-(trifluoromethyl)-1,2,4-oxadiazol-3-yl]phenyl}methyl)azepan-2-one + SX, 4,4-dimethyl-1-({4-[5-(trifluoromethyl)-1,2,4-oxadiazol-3-yl]phenyl}methyl)pyrrolidin-2-one + SX, 5-methyl-1-({4-[5-(trifluoromethyl)-1,2,4-oxadiazol-3-yl]phenyl}methyl)pyrrolidin-2-one + SX, ethyl 1-({4-[5-(trifluoromethyl)-1,2,4-oxadiazol-3-yl]phenyl}methyl)-1H-pyrazole-4-carboxylate + SX, N-methyl-1-({4-[5-(trifluoromethyl)-1,2,4-oxadiazol-3-yl]phenyl}methyl)-1H-pyrazole-4-carboxamide + SX, N-propyl-1-({4-[5-(trifluoromethyl)-1,2,4-oxadiazol-3-yl]phenyl}methyl)-1H-pyrazole-4-carboxamide + SX, N-methoxy-1-({4-[5-(trifluoromethyl)-1,2,4-oxadiazol-3-yl]phenyl}methyl)-1H-pyrazole-4-carboxamide + SX, N-methoxy-N-methyl-1-({4-[5-(trifluoromethyl)-1,2,4-oxadiazol-3-yl]phenyl}methyl)-1H-pyrazole-4-carboxamide + SX, N,N-dimethyl-1-({4-[5-(trifluoromethyl) -1,2,4-oxadiazol-3-yl]phenyl}methyl)-1H-1,2,4-triazol-3-amine + SX, N-methyl-4-[5-(trifluoromethyl)-1,2,4-oxadiazol-3-yl]benzamide + SX, methyl 2-[2-chloro-4-(4-chlorophenoxy)phenyl]-2-hydroxy-3-(1H-1,2,4-triazol-1-yl)propanoate + SX, ethyl 2-[2-chloro-4-(4-chlorophenoxy)phenyl]-2-hydroxy-3-(1H-1,2,4-triazol-1-yl)propanoate + SX, methyl 2-[2-(trifluoromethyl)-4-(4-chlorophenoxy)phenyl]-2-hydroxy-3-(1H-1,2,4-triazol-1-yl)propanoate + SX, 1-(2,3-dimethylpyridin-5-yl)-4,4-difluoro-3,3-dimethyl-3,4-dihydroisoquinoline + SX, 1-[2-(difluoromethyl)-3-methylpyridin-5-yl]-4,4-difluoro-3,3-dimethyl-3,4-dihydroisoquinoline + SX, 2,2-difluoro-N-[6-({[1-(1-methyl-1H-tetrazol-5-yl)benzimidazol-2-yl]oxy}methyl)pyridin-2-yl]-2-phenoxyacetamide + SX, 1-[2-(1-chlorocyclopropyl)-3-(3-chloro-2-fluorophenyl)-2-hydroxypropyl]-1H-imidazole-5-carbonitrile + SX, ethyl 1-[(4-{[2-(trifluoromethyl)-1,3-dioxolan-2-yl]methoxy}phenyl)methyl]-1H-pyrazole-4-carboxylate + SX, ethyl 1-[(4-{[(1Z)-2-ethoxy-3,3,3-trifluoro-1-propen-1-yl]oxy}phenyl)methyl]-1H-pyrazole-4-carboxylate + SX, 6-chloro-3-(3-cyclopropyl-2-fluorophenoxy)-N-[2-(2,4-dimethylphenyl)-2,2-difluoroethyl]-5-methylpyridazine-4-carboxamide + SX, 6-chloro-3-(3-cyclopropyl-2-fluorophenoxy)-N-[2-(3,4-dimethylphenyl)-2,2-difluoroethyl]-5-methylpyridazine-4-carboxamide + SX, 6-chloro-N-[2-(2-chloro-4-methylphenyl)-2,2-difluoroethyl]-3-(3-cyclopropyl-2-fluorophenoxy)-5-methylpyridazine-4-carboxamide + SX, 2-[cyano(2,6-difluoropyridin-4-yl)amino]-N-(2,2-dimethylcyclobutyl)-5-methylthiazole-4-carboxamide + SX, 2-[cyano(2,6-difluoropyridin-4-yl)amino]-N-(spiro[3.4]octan-1-yl)-5-methylthiazole-4-carboxamide + SX, 2-[cyano(2,6-difluoropyridin-4-yl)amino]-N-hexyl-5-methylthiazole-4-carboxamide + SX, 2-[acetyl(2,6-difluoropyridin-4-yl)amino]-N-(2,2-dimethylcyclobutyl)-5-methylthiazole-4-carboxamide + SX, 2-[(2-methoxyacetyl)(2,6-difluoropyridin-4-yl)amino]-N-(2,2-dimethylcyclobutyl)-5-methylthiazole-4-carboxamide + SX, 2-[(2-methylpropanoyl)(2,6-difluoropyridin-4-yl)amino]-N-(2,2-dimethylcyclobutyl)-5-methylthiazole-4-carboxamide + SX, 2-[(2,6-difluoropyridin-4-yl)amino]-N-(2,2-dimethylcyclobutyl)-5-methylthiazole-4-carboxamide + SX, 2-{[(oxetan-3-yl)carbonyl] (2,6-difluoropyridin-4-yl)amino}-N-(2,2-dimethylcyclobutyl)-5-methylthiazole-4-carboxamide + SX, 2-{[(oxolan-3-yl)carbonyl] (2,6-difluoropyridin-4-yl)amino}-N-(2,2-dimethylcyclobutyl)-5-methylthiazole-4-carboxamide + SX, 2-{[(oxan-4-yl)carbonyl] (2,6-difluoropyridin-4-yl)amino}-N-(2,2-dimethylcyclobutyl)-5-methylthiazole-4-carboxamide + SX, 5-[5-(difluoromethyl)-1,3,4-oxadiazol-2-yl]-N-[1-(2-fluorophenyl)ethyl]pyrimidin-2-amine + SX, 5-[5-(difluoromethyl)-1,3,4-oxadiazol-2-yl]-N-[1-(2,6-difluorophenyl)ethyl]pyrimidin-2-amine + SX, 5-[5-(difluoromethyl)-1,3,4-oxadiazol-2-yl]-N-[1-(3,5-difluorophenyl)ethyl]pyrimidin-2-amine + SX, 5-[5-(difluoromethyl)-1,3,4-oxadiazol-2-yl]-N-[1-(6-chloropyridin-3-yl)ethyl]pyrimidin-2-amine + SX, 5-[5-(difluoromethyl)-1,3,4-oxadiazol-2-yl]-N-[1-(2-fluorophenyl)cyclopropyl]pyrimidin-2-amine + SX, 5-[5-(difluoromethyl)-1,3,4-oxadiazol-2-yl]-N-[1-(2,6-difluorophenyl)cyclopropyl]pyrimidin-2-amine + SX, 5-[5-(difluoromethyl)-1,3,4-oxadiazol-2-yl]-N-[1-(2-fluoro-3-methoxyphenyl)cyclopropyl]pyrimidin-2-amine + SX, 5-[5-(difluoromethyl)-1,3,4-oxadiazol-2-yl]-2-{[1-(2,6-difluorophenyl)cyclopropyl]oxy}pyrimidine + SX, 3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, (5S)-3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, 3-[3-(3-chloro-2-fluorophenoxy)-6-methylpyridazin-4-yl]-5-[(4-bromo-2-methylphenyl)methyl]-5,6-dihydro-4H-1,2,4-oxadiazine + SX, 3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5S)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5R)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2-methylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2,2-dimethylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(S-fluoro-2-methoxyphenyl)-2-(2-cyanoethoxy)ethyl]-5-[1-(isopropoxyimino)ethyl]-2,6-dioxo-3,6-dihydropyrimidin-1(2H)-yl}propan-2-yl)-2-methylpropanamide + SX, methyl ({5-[1-(2,6-difluoro-4-isopropylphenyl)-1H-pyrazol-3-yl]-2-methylphenyl}methyl)carbamate + SX, methyl ({5-[1-(2,6-difluoro-4-cyclopropylphenyl)-1H-pyrazol-3-yl]-2-methylphenyl}methyl)carbamate + SX, methyl ({5-[1-(2,6-difluoro-4-methoxyphenyl)-1H-pyrazol-3-yl]-2-methylphenyl}methyl)carbamate + SX, methyl (Z)-2-(5-cyclopentyl-2-methylphenoxy)-3-methoxyprop-2-enoate + SX, methyl (Z)-2-(5-cyclohexyl-2-methylphenoxy)-3-methoxyprop-2-enoate + SX, methyl (Z)-2-[(3-isopropyl-1H-pyrazol-1-yl)-2-methylphenoxy]-3-methoxyprop-2-enoate + SX, 1-(4,5-dimethyl-1H-benzimidazol-1-yl)-4,4-difluoro-3,3-dimethyl-3,4-dihydroisoquinoline + SX, 1-(4,5-dimethyl-1H-benzimidazol-1-yl)-4,4,5-trifluoro-3,3-dimethyl-3,4-dihydroisoquinoline + SX, 1-(pyrazolo[1,5-a]pyridin-3-yl)-4,4-difluoro-3,3-dimethyl-3,4-dihydroisoquinoline + SX, 1-(6,7-dimethylpyrazolo[1,5-a]pyridin-3-yl)-6-fluoro-3,3-dimethylisoquinolin-4(3H)-one + SX, methyl (2Z)-3-methoxy-2-[(4-methyl[1,1'-biphenyl]-3-yl)oxy]prop-2-enoate + SX, Agrobacterium radiobactor strain K1026 + SX, Agrobacterium radiobactor strain K84 + SX, Bacillus amyloliquefaciens strain PTA-4838 (Aveo (trademark) EZ Nematicide) + SX, Bacillus amyloliquefaciens strain AT332 + SX, Bacillus amyloliquefaciens strain B3 + SX, Bacillus amyloliquefaciens strain D747 + SX, Bacillus amyloliquefaciens strain DB101 + SX, Bacillus amyloliquefaciens strain DB102 + SX, Bacillus amyloliquefaciens strain GB03 + SX, Bacillus amyloliquefaciens strain FZB24 + SX, Bacillus amyloliquefaciens strain FZB42 + SX, Bacillus amyloliquefaciens strain IN937a + SX, Bacillus amyloliquefaciens strain MBI600 + SX, Bacillus amyloliquefaciens strain QST713 + SX, Bacillus amyloliquefaciens isolate strain B246 + SX, Bacillus amyloliquefaciens strain F727 + SX, Bacillus amyloliquefaciens subsp. plantarum strain D747 + SX, Bacillus licheniformis strain HB-2 + SX, Bacillus licheniformis strain SB3086 + SX, Bacillus pumilus strain AQ717 + SX, Bacillus pumilus strain BUF-33 + SX, Bacillus pumilus strain GB34 + SX, Bacillus pumilus strain QST2808 + SX, Bacillus simplex strain CGF2856 + SX, Bacillus subtilis strain AQ153 + SX, Bacillus subtilis strain AQ743 + SX, Bacillus subtilis strain BU1814 + SX, Bacillus subtilis strain D747 + SX, Bacillus subtilis strain DB101 + SX, Bacillus subtilis strain FZB24 + SX, Bacillus subtilis strain GB03 + SX, Bacillus subtilis strain HAI0404 + SX, Bacillus subtilis strain IAB/BS03 + SX, Bacillus subtilis strain MBI600 + SX, Bacillus subtilis strain QST30002/AQ30002 + SX, Bacillus subtilis strain QST30004/AQ30004 + SX, Bacillus subtilis strain QST713 + SX, Bacillus subtilis strain QST714 + SX, Bacillus subtilis var. Amyloliquefaciens strain FZB24 + SX, Bacillus subtilis strain Y1336 + SX, Burkholderia cepacia + SX, Burkholderia cepacia type Wisconsin strain J82 + SX, Burkholderia cepacia type Wisconsin strain M54 + SX, Candida oleophila strain O + SX, Candida saitoana + SX, Chaetomium cupreum + SX, Clonostachys rosea + SX, Coniothyrium minitans strain CGMCC8325 + SX, Coniothyrium minitans strain CON/M/91-8 + SX, cryptococcus albidus + SX, Erwinia carotovora subsp. carotovora strain CGE234M403 + SX, Fusarium oxysporum strain Fo47 + SX, Gliocladium catenulatum strain J1446 + SX, Paenibacillus polymyxa strain AC-1 + SX, Paenibacillus polymyxa strain BS-0105 + SX, Pantoea agglomerans strain E325 + SX, Phlebiopsis gigantea strain VRA1992 + SX, Pseudomonas aureofaciens strain TX-1 + SX, Pseudomonas chlororaphis strain 63-28 + SX, Pseudomonas chlororaphis strain AFS009 + SX, Pseudomonas chlororaphis strain MA342 + SX, Pseudomonas fluorescens strain 1629RS + SX, Pseudomonas fluorescens strain A506 + SX, Pseudomonas fluorescens strain CL145A + SX, Pseudomonas fluorescens strain G7090 + SX, Pseudomonas sp. strain CAB-02 + SX, Pseudomonas syringae strain 742RS + SX, Pseudomonas syringae strain MA-4 + SX, Pseudozyma flocculosa strain PF-A22UL + SX, Pseudomonas rhodesiae strain HAI-0804 + SX, Pythium oligandrum strain DV74 + SX, Pythium oligandrum strain M1 + SX, Streptomyces griseoviridis strain K61 + SX, Streptomyces lydicus strain WYCD108US + SX, Streptomyces lydicus strain WYEC108 + SX, Talaromyces flavus strain SAY-Y-94-01 + SX, Talaromyces flavus strain V117b + SX, Trichoderma asperellum strain ICC012 + SX, Trichoderma asperellum SKT-1 + SX, Trichoderma asperellum strain T25 + SX, Trichoderma asperellum strain T34 + SX, Trichoderma asperellum strain TV1 + SX, Trichoderma atroviride strain CNCM 1-1237 + SX, Trichoderma atroviride strain LC52 + SX, Trichoderma atroviride strain IMI 206040 + SX, Trichoderma atroviride strain SC1 + SX, Trichoderma atroviride strain SKT-1 + SX, Trichoderma atroviride strain T11 + SX, Trichoderma gamsii strain ICC080 + SX, Trichoderma harzianum strain 21 + SX, Trichoderma harzianum strain DB104 + SX, Trichoderma harzianum strain DSM 14944 + SX, Trichoderma harzianum strain ESALQ-1303 + SX, Trichoderma harzianum strain ESALQ-1306 + SX, Trichoderma harzianum strain IIHR-Th-2 + SX, Trichoderma harzianum strain ITEM908 + SX, Trichoderma harzianum strain kd + SX, Trichoderma harzianum strain MO1 + SX, Trichoderma harzianum strain SF + SX, Trichoderma harzianum strain T22 + SX, Trichoderma harzianum strain T39 + SX, Trichoderma harzianum strain T78 + SX, Trichoderma harzianum strain TH35 + SX, Trichoderma polysporum strain IMI206039 + SX, trichoderma stromaticum + SX, Trichoderma virens strain G-41 + SX, Trichoderma virens strain GL-21 + SX, Trichoderma viride + SX, Variovorax paradoxus strain CGF4526 + SX, Harpin protein + SX.

Combinations of the Present ingredient in the above Group (c) and the Compound of the present invention:
1-methylcyclopropene + SX, 1,3-diphenylurea + SX, 2,3,5-triiodobenzoic acid + SX, IAA ((1H-indol-3-yl)acetic acid) + SX, IBA (4-(1H-indol-3-yl)butyric acid) + SX, MCPA (2-(4-chloro-2-methylphenoxy)acetic acid) + SX, MCPB (4-(4-chloro-2-methylphenoxy)butyric acid) + SX, 4-CPA (4-chlorophenoxyacetic acid) + SX, 5-aminolevulinic acid hydrochloride + SX, 6-benzylaminopurine + SX, abscisic acid + SX, AVG (aminoethoxyvinylglycine) + SX, anisiflupurin + SX, ancymidol + SX, butralin + SX, calcium carbonate + SX, calcium chloride + SX, calcium formate + SX, calcium peroxide + SX, calcium polysulfide + SX, calcium sulfate + SX, chlormequat-chloride + SX, chlorpropham + SX, choline chloride + SX, cloprop + SX, cyanamide + SX, cyclanilide + SX, daminozide + SX, decan-1-ol + SX, dichlorprop + SX, dikegulac + SX, dimethipin + SX, diquat + SX, ethephon + SX, ethychlozate + SX, flumetralin + SX, flurprimidol + SX, forchlorfenuron + SX, formononetin + SX, Gibberellin A + SX, Gibberellin A3 + SX, inabenfide + SX, Kinetin + SX, lipochitooligosaccharide SP104 + SX, maleic hydrazide + SX, mefluidide + SX, mepiquat-chloride + SX, oxidized glutathione + SX, paclobutrazol + SX, pendimethalin + SX, prohexadione-calcium + SX, prohydrojasmon + SX, pyraflufen-ethyl + SX, sintofen + SX, sodium 1-naphthaleneacetate + SX, sodium cyanate + SX, thidiazuron + SX, triapenthenol + SX, tribufos + SX, trinexapac-ethyl + SX, uniconazole-P + SX, 2-(naphthalen-1-yl)acetamide + SX, [4-oxo-4-(2-phenylethyl)amino]butyric acid + SX, methyl 5-(trifluoromethyl)benzo[b]thiophene-2-carboxylate + SX, 3-[(6-chloro-4-phenylquinazolin-2-yl)amino]propan-1-ol + SX, Claroideoglomus etunicatum + SX, Claroideoglomus claroideum + SX, Funneliformis mosseae + SX, Gigaspora margarita + SX, Gigaspora rosea + SX, Glomus aggregatum + SX, Glomus deserticola + SX, Glomus monosporum + SX, Paraglomus brasillianum + SX, Rhizophagus clarus + SX, Rhizophagus intraradices RTI-801 + SX, Rhizophagus irregularis DAOM 197198 + SX, Azorhizobium caulinodans + SX, Azospirillum amazonense + SX, Azospirillum brasilense XOH + SX, Azospirillum brasilense Ab-V5 + SX, Azospirillum brasilense Ab-V6 + SX, Azospirillum caulinodans + SX, Azospirillum halopraeferens + SX, Azospirillum irakense + SX, Azospirillum lipoferum + SX, Bradyrhizobium elkanii SEMIA 587 + SX, Bradyrhizobium elkanii SEMIA 5019 + SX, Bradyrhizobium japonicum TA-11 + SX, Bradyrhizobium japonicum USDA 110 + SX, Bradyrhizobium liaoningense + SX, Bradyrhizobium lupini + SX, Delftia acidovorans RAY209 + SX, Mesorhizobium ciceri + SX, Mesorhizobium huakii + SX, Mesorhizobium loti + SX, Rhizobium etli + SX, Rhizobium galegae + SX, Rhizobium leguminosarum bv. Phaseoli + SX, Rhizobium leguminosarum bv. Trifolii + SX, Rhizobium leguminosarum bv. Viciae + SX, Rhizobium trifolii + SX, Rhizobium tropici + SX, Sinorhizobium fredii + SX, Sinorhizobium meliloti + SX, Zucchini Yellow Mosaik Virus weak strain + SX.

Combinations of the Present ingredient in the above Group (d) and the Compound of the present invention:
anthraquinone + SX, deet + SX, icaridin + SX.

Examples of the ratio of the Compound of the present invention and the Present ingredient include, but are not limited to, 1000:1 to 1:1000, 500:1 to 1:500, 100:1 to 1:100, 50:1, 20:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:20, and 1:50, in the ratio by weight (Compound of the present invention : Present ingredient).

The Present compound, the Compound of the present invention, or the Composition A can control plant diseases caused by plant pathogenic microorganisms such as fungi, Oomycete, Phytomyxea, and bacteria. Examples of the fungi include Ascomycota, Basidiomycota, Blasocladiomycota, Chytridiomycota, Mucoromycota, and Olpidiomycota. Specific examples thereof include the followings. The scientific name of plant pathogenic microorganism which causes each disease is shown in parentheses.

Rice diseases:
   blast (Pyricularia oryzae), brown spot (Cochliobolus miyabeanus), sheath blight (Rhizoctonia solani), bakanae disease (Gibberella fujikuroi), downy mildew (Sclerophthora macrospora), false blast and head blight (Epicoccum nigrum), seedling blight (Trichoderma viride and Rhizopus oryzae), pseudo sheath blight (Waitea circinate, Ceratobasidium setariae, and Thanatephorus cucumeris), Rice false smut (Ustilaginoidea virens), and Kernel smut (Tilletia horrida and Tilletia barclayana);
Wheat diseases:
   powdery mildew (Blumeria graminis), fusarium blight (Fusarium graminearum, Fusarium avenaceum, Fusarium culmorum, and Microdochium nivale), stripe rust (Puccinia striiformis), stem rust (Puccinia graminis), leaf rust (Puccinia recondita), snow mould (Microdochium nivale and Microdochium majus), typhula snow blight (Typhula incarnata and Typhula ishikariensis), Sclerotinia snow blight (Sclerotinia borealis), Browning root rot (Pythium spp.), loose smut (Ustilago tritici), stinking smut (Tilletia caries and Tilletia controversa), eyespot (Pseudocercosporella herpotrichoides), leaf blotch (Septoria tritici), glume blotch (Stagonospora nodorum), tan spot (Pyrenophora tritici-repentis), rhizoctonia seeding blight (Rhizoctonia solani), take-all disease (Gaeumannomyces graminis), and blast (Pyricularia graminis-tritici);
Barley diseases:
   powdery mildew (Blumeria graminis), fusarium head blight (Fusarium graminearum, Fusarium avenaceum, Fusarium culmorum, and Microdochium nivale), stripe rust (Puccinia striiformis), stem rust (Puccinia graminis), dwarf leaf rust (Puccinia hordei), loose smut (Ustilago nuda), scald (Rhynchosporium secalis), net blotch (Pyrenophora teres), spot blotch (Cochliobolus sativus), stripe (Pyrenophora graminea), Ramularia disease (Ramularia collo-cygni), and rhizoctonia seeding blight (Rhizoctonia solani);
Corn diseases:
   rust (Puccinia sorghi), southern rust (Puccinia polysora), northern leaf blight (Setosphaeria turcica), tropical rust (Physopella zeae), southern leaf blight (Cochliobolus heterostrophus), anthracnose (Colletotrichum graminicola), gray leaf spot (Cercospora zeae-maydis), eyespot (Kabatiella zeae), phaeosphaeria leaf spot (Phaeosphaeria maydis), diplodia rot (Stenocarpella maydis and Stenocarpella macrospora), stalk rot (Fusarium graminearum, Fusarium verticilioides, and Colletotrichum graminicola), smut (Ustilago maydis), Physoderma brown spot and Physoderma stalk rot (Physoderma maydis), and tar spot disease (Phyllachora maydis);
Cotton diseases:
   anthracnose (Colletotrichum gossypii), grey mildew (Ramularia areola), alternaria leaf spot (Alternaria macrospora and Alternaria gossypii), and black root rot (Thielaviopsis basicola);
Coffee diseases:
   rust (Hemileia vastatrix) and leaf spot (Cercospora coffeicola);
Rape seed diseases:
   sclerotinia rot (Sclerotinia sclerotiorum), gray leaf spot (Alternaria brassicae), root rot (Phoma lingam), and light leaf spot (Pyrenopeziza brassicae);
Sugar cane diseases:
   rust (Puccinia melanocephela and Puccinia kuehnii), and smut (Ustilago scitaminea);
Sunflower diseases:
   rust (Puccinia helianthi) and downy mildew (Plasmopara halstedii);
Citrus diseases:
   melanose (Diaporthe citri), scab (Elsinoe fawcetti), green mold (Penicillium digitatum), blue mold (Penicillium italicum), Phytophthora rot (Phytophthora parasitica and Phytophthora citrophthora), and aspergillus rot (Aspergillus niger) ;
Apple diseases:
   blossom blight (Monilinia mali), valsa canker (Valsa ceratosperma), powdery mildew (Podosphaera leucotricha), alternaria leaf spot (Alternaria alternata apple pathotype), scab (Venturia inaequalis), bitter rot (Glomerella cingulata and Colletotrichum acutatum), blotch (Diplocarpon mali), ring rot (Botryosphaeria berengeriana), crown rot (Phytophtora cactorum), and rust (Gymnosporangium juniperi-virginianae and Gymnosporangium yamadae);
Pear diseases:
   scab (Venturia nashicola and Venturia pirina), black spot (Alternaria alternata Japanese pear pathotype), and rust (Gymnosporangium haraeanum);
Peach diseases:
   brown rot (Monilinia fructicola), scab (Cladosporium carpophilum), Phomopsis rot (Phomopsis sp.), leaf curl (Taphrina deformans), and powdery mildew (Podosphaera leucotricha) ;
Grapes diseases:
   anthracnose (Elsinoe ampelina), ripe rot (Glomerella cingulata and Colletotrichum acutatum), powdery mildew (Uncinula necator), rust (Neophysopella sp.), black rot (Guignardia bidwellii), downy mildew (Plasmopara viticola), leaf blight (Pseudocercospora vitis), and white rot (Coniella castaneicola);
Japanese persimmon diseases:
   anthracnose (Gloeosporium kaki and Colletotrichum acutatum), leaf spot (Cercospora kaki and Mycosphaerella nawae), and powdery mildew (Phyllactinia kakicola);
Fig disease:
   rust (Phakopsora nishidana);
Diseases of gourd family:
   anthracnose (Colletotrichum lagenarium), powdery mildew (Sphaerotheca fuliginea), gummy stem blight (Didymella bryoniae), Corynespora leaf spot (Corynespora cassiicola), fusarium wilt (Fusarium oxysporum), downy mildew (Pseudoperonospora cubensis), phytophthora rot (Phytophthora capsici), damping-off (Pythium sp.), and Black root rot (Phomopsis sp.);
Tomato diseases:
   early blight (Alternaria solani), leaf mold (Cladosporium fulvum), Cercospora leaf mold (Pseudocercospora fuligena), late blight (Phytophthora infestans), and powdery mildew (Leveillula taurica);
Eggplant diseases:
   brown spot (Phomopsis vexans), powdery mildew (Erysiphe cichoracearum), black blight (Corynespora melongenae), leaf mold (Mycovellosiella nattrassii), and brown leaf spot (Thanatephorus cucumeris);
Cruciferous vegetables diseases:
   alternaria leaf spot (Alternaria japonica), white spot (Cercosporella brassicae), clubroot (Plasmodiophora brassicae), downy mildew (Peronospora parasitica), white rust (Albugo candida), and Alternaria sooty spot (Alternaria brassicicola);
Welsh onion diseases:
   rust (Puccinia allii), black spot (Alternaria porri), white rot (Sclerotium cepivorum), leaf blight (Botrytis squamosa), leaf spot (Stemphylium vesicarium and Stemphylium botryosum), southern blight (Sclerotium rolfsii), leaf blight (Botrytis squamosa);
Soybean diseases:
   purple stain (Cercospora kikuchii), sphaceloma scab (Elsinoe glycines), pod and stem blight (Diaporthe phaseolorum var. sojae), rust (Phakopsora pachyrhizi), target spot (Corynespora cassiicola), anthracnose (Colletotrichum glycines and Colletotrichum truncatum), Rhizoctonia rot (Rhizoctonia solani), septoria brown spot (Septoria glycines), Cercospora leaf spot (Cercospora sojina), stem rot (Sclerotinia sclerotiorum), powdery mildew (Microsphaera diffusa), phytophthora stem and root rot (Phytophthora sojae), downy mildew (Peronospora manshurica), sudden death syndrome (Fusarium virguliforme), red crown rot (Calonectria ilicicola), and Diaporthe/Phomopsis complex (Diaporthe longicolla, Diaporthe phaseolorum, and Phomopsis longicolla);
Kidney bean diseases:
   stem rot (Sclerotinia sclerotiorum), rust (Uromyces appendiculatus), angular leaf spot (Phaeoisariopsis griseola), anthracnose (Colletotrichum lindemuthianum), and Fusarium root-rot (Fusarium solani);
Peanut diseases:
   leaf spot (Cercospora personata), brown leaf spot (Cercospora arachidicola), southern blight (Sclerotium rolfsii), and Cylindrocladium black rot (Calonectria ilicicola);
Garden pea diseases:
   powdery mildew (Erysiphe pisi) and root rot (Fusarium solani);
Potato diseases:
   early blight (Alternaria solani), late blight (Phytophthora infestans), Pink rot (Phytophthora erythroseptica), powdery scab (Spongospora subterranea f. sp. subterranea), verticillium wilt (Verticillium albo-atrum, Verticillium dahliae, and Verticillium nigrescens), dry rot (Fusarium solani), and potato wart (Synchytrium endobioticum);
Strawberry diseases:
   powdery mildew (Sphaerotheca humuli) and crown rot (Glomerella cingulata and Colletotrichum acutatum);
Tea diseases:
   net blister blight (Exobasidium reticulatum), white scab (Elsinoe leucospila), gray blight (Pestalotiopsis sp.), and anthracnose (Colletotrichum theae-sinensis);
Tobacco diseases:
   brown spot (Alternaria longipes), anthracnose (Colletotrichum tabacum), blue mold (Peronospora tabacina), and black shank (Phytophthora nicotianae);
Sugar beet diseases:
   cercospora leaf spot (Cercospora beticola), leaf blight (Thanatephorus cucumeris), root rot (Thanatephorus cucumeris), aphanomyces root rot (Aphanomyces cochlioides), and rust (Uromyces betae);
Rose diseases:
   black spot (Diplocarpon rosae) and powdery mildew (Sphaerotheca pannosa);
Chrysanthemum diseases:
   leaf blight (Septoria chrysanthemi-indici) and white rust (Puccinia horiana);
Onion diseases:
   botrytis leaf blight (Botrytis cinerea, Botrytis byssoidea, and Botrytis squamosa), gray-mold neck rot (Botrytis allii), and small sclerotial neck rot (Botrytis squamosa);
Various crops diseases:
   Botrytis rot (Botrytis cinerea), sclerotinia rot (Sclerotinia sclerotiorum), white rot (Sclerotium cepivorum), southern blight (Sclerotium rolfsii), and seedling blight (Pythium aphanidermatum, Pythium irregulare, and Pythium ultimum);
Japanese radish disease:
   alternaria leaf spot (Alternaria brassicicola);
Sweet potato diseases:
   stem rot (Fusarium oxysporum f. sp. batatas), foot rot (Diaporthe destruens), and black rot(Ceratocystis fimbriata);
Turfgrass diseases:
   dollar spot (Sclerotinia homoeocarpa), brown patch and large patch (Rhizoctonia solani), pythium blight (Pythium aphanidermatum), and anthracnose (Colletotrichum caudatum);
Banana disease:
   Sigatoka disease (Mycosphaerella fijiensis and Mycosphaerella musicola);
Lentils disease:
   ascochyta blight (Ascochyta lentis);
Chickpea disease:
   ascochyta blight (Ascochyta rabiei);
Green pepper diseases:
   anthracnose (Colletotrichum scovillei), powdery mildew (Leveillula taurica), and southern blight (Sclerotium rolfsii);
Mango disease:
   anthracnose (Colletotrichum acutatum);
ocarina disease:
   foot rot (Diaporthe destruens);
Fruit trees diseases:
   white root rot (Rosellinia necatrix) and violet root rot (Helicobasidium mompa);
Postharvest disease of fruits (for example, apple and pear) :
   Mucor rot diseases (Mucor piriformis);
Seed diseases or diseases in the early stages of the growth of various plants caused by Aspergillus spp., Penicillium spp., Fusarium spp., Gibberella spp., Tricoderma spp., Thielaviopsis spp., Rhizopus spp., Mucor spp., Corticium spp., Phoma spp., Rhizoctonia spp. or Diplodia spp., or the like;
Viral diseases:
   Lettuce big-vein disease transmitted by Olpidium brassicae, and viral diseases of several crops transmitted by Polymyxa spp. (e.g. Polymyxa betae and Polymyxa graminis);
Diseases caused by bacteria:
   bacterial seedling blight of rice (Burkholderia plantarii), bacterial palea browning of rice (Pantoea ananatis), Bacterial leaf blight of rice (Xanthomonas oryzae pv. oryzae.), bacterial spot of cucumber (Pseudomonas syringae pv. lachrymans), bacterial wilt of eggplant (Ralstonia solanacearum), canker of citrus (Xanthomonas citri), bacterial soft rot of Chinese cabbage (Erwinia carotovora), scab of potato (Streptomyces scabiei), black leg of potato (Pectobacterium carotovorum and Dickeya sp.), bacterial leaf spot of peach (Pseudomona syringae, Erwinia nigrifluens, and Xanthomonas campestris), bacterial canker of Japanese apricot (Prunus mume) (Pseudomonas syringae pv. morsprunorum and Erwinia sp.), Goss's wilt of corn (Clavibacter michiganensis), Pierce's disease of grapes, olive, peach, and the like (Xylella fastidiosa), and crown gall of Rosaceae plants such as apple, peach, and cherries (Agrobacterium tumefaciens);
and the others.

A rust fungus of soybean having an amino acid substitution of F129L in the mitochondrial cytochrome b protein means a rust fungus of soybean (scientific name: Phakopsora pachyrhizi) which has a mutation in the mitochondrial cytochrome b gene encoding the mitochondrial cytochrome protein, and as a result of said mutation, has an amino acid substitution of F129L, thereby shows resistance to QoI fungicides.

One embodiment of the method for controlling a plant disease of the present invention is a method for controlling Phakopsora pachyrhizi which comprises applying the Present compound to a soybean or soil for cultivating a soybean.

The method for controlling plant diseases of the present invention is carried out by applying an effective amount of the Present compound, the Compound of the present invention, or the Composition A to plants or soil for cultivating plants. Examples of the treatment method include foliage treatment, soil treatment, and seed treatment.

The Present compound, the Compound of the present invention, or the Composition A is usually used by mixing it with inert carrier(s) such as solid carrier(s) and liquid carrier(s), surfactant(s), and the like, and as needed, adding thereto auxiliary agent(s) for formulation such as binder(s), dispersant(s), and stabilizer(s) to be formulated into an aqueous suspension formulation, an oily suspension formulation, an oil solution, an emulsifiable concentrate, an emulsion formulation, a microemulsion formulation, a microcapsule formulation, a wettable powder, a granular wettable powder, a dust formulation, a granule, or the like. In addition to these formulations, the Present compound or the Composition A may be used by formulating it into a dosage form described in Manual on development and use of FAO and WHO Specifications for pesticides, FAO Plant Production and Protection Papers-271-276, prepared by the FAO/WHO Joint Meeting on Pesticide Specifications, 2016, ISSN:0259-2517.

These formulations usually comprise 0.0001 to 99% by weight ratio of the Present compound, the Compound of the present invention, or the Composition A.

Examples of the solid carrier include fine powders and granules of clays (for example, pyrophyllite clay and kaolin clay), talc, calcium carbonate, diatomaceous earth, zeolite, bentonite, acid white clay, attapulgite, white carbon, ammonium sulfate, vermiculite, perlite, pumice, silica sand, chemical fertilizers (for example, ammonium sulfate, ammonium phosphate, ammonium nitrate, urea, and ammonium chloride), and the others; as well as resins (for example, polyethylene, polypropylene, polyester, polyurethane, polyamide, and polyvinyl chloride).

Examples of the liquid carrier include water, alcohols (for example, ethanol, cyclohexanol, benzyl alcohol, propylene glycol, and polyethylene glycol), ketones (for example, acetone and cyclohexanone), aromatic hydrocarbons (for example, xylene, phenyl xylyl ethane, and methylnaphthalene), aliphatic hydrocarbons (for example, hexane and cyclohexane), esters (for example, ethyl acetate, methyl oleate, and propylene carbonate), nitriles (for example, acetonitrile), ethers (for example, ethylene glycol dimethyl ether), amides (for example, N,N-dimethylformamide and N,N-dimethyloctanamide), sulfoxides (for example, dimethyl sulfoxide), lactams (for example, N-methylpyrrolidone and N-octylpyrrolidone), fatty acids (for example, oleic acid), and vegetable oils (for example, soybean oil).

Examples of the surfactant include nonionic surfactants (for example, polyoxyethylene alkyl ethers, polyoxyethylene alkyl aryl ethers, and polyethylene glycol fatty acid esters), and anionic surfactants (for example, alkyl sulfonates, alkyl aryl sulfonates, and alkyl sulfates).

Examples of the other auxiliary agent for formulation include binders, dispersants, colorants, and stabilizers, and the specific examples thereof include polysaccharides (for example, starch, gum arabic, cellulose derivatives, and alginic acid), lignin derivatives, water-soluble synthetic polymers (for example, polyvinyl alcohol, polyvinyl pyrrolidone, and polyacrylic acids), acidic isopropyl phosphate, and dibutylhydroxytoluene.

Also, an adjuvant may be used as an ingredient to enhance or assist the efficacy of the Present compound, the Compound of the present invention, or the Composition A. The specific examples thereof include Nimbus (registered trademark), Assist (registered trademark), Aureo (registered trademark), Iharol (registered trademark), Silwet L-77 (registered trademark), BreakThru (registered trademark), SundanceII (registered trademark), Induce (registered trademark), Penetrator (registered trademark), AgriDex (registered trademark), Lutensol A8 (registered trademark), NP-7 (registered trademark), Triton (registered trademark), Nufilm (registered trademark), Emulgator NP7 (registered trademark), Emulad (registered trademark), TRITON X 45 (registered trademark), AGRAL 90 (registered trademark), AGROTIN (registered trademark), ARPON (registered trademark), EnSpray N (registered trademark), and BANOLE (registered trademark).

In the present invention, the plants include whole plants and specific parts of plants. Examples of the specific parts of plants include foliages, flowers, ears, fruits, tree stems, branches, tree crowns, seeds, vegetative reproductive organs, and nursery plants.

A vegetative reproductive organ means a part of plant such as root, stem, and leaf which has a growth capability even when said part is separated from the plant body and placed into soil. Examples of the vegetative reproductive organ include tuberous root, creeping root, bulb, corm or solid bulb, tuber, rhizome, stolon, rhizophore, cane cuttings, propagule, and vine cutting. Stolon is also referred to as "runner", and propagule is also referred to as "propagulum" and categorized into broad bud and bulbil. Vine cutting means a shoot (collective term of leaf and stem) of sweet potato, glutinous yam, or the like. Bulb, corm or solid bulb, tuber, rhizome, cane cuttings, rhizophore, and tuberous root are also collectively referred to as "bulb". For example, cultivation of potato starts with planting a tuber into soil, and the tuber to be used is generally referred to as "seed potato".

Nursery plants include seedlings and saplings.

Examples of a method for controlling plant diseases by applying an effective amount of the Present compound, the Compound of the present invention, or the Composition A to soil include a method of applying an effective amount of the Present compound, the Compound of the present invention, or Composition A to soil before planting plants or after planting plants. More specifically, examples of the application method include planting hole treatment (for example, spraying into planting holes and soil mixing after planting hole treatment), plant foot treatment (for example, plant foot spraying, soil mixing after plant foot treatment, irrigation at plant foot, and plant foot treatment at a later seeding raising stage), planting furrow treatment (for example, planting furrow spraying and soil mixing after planting furrow treatment), planting row treatment (for example, planting row spraying, soil mixing after planting row treatment, and planting row spraying at a growing stage), planting row treatment at the time of sowing (for example, planting row spraying at the time of sowing and soil mixing after planting row treatment at the time of sowing), broadcast treatment (for example, overall soil surface spraying and soil mixing after broadcast treatment), side-article treatment, treatment of water surface (for example, application to water surface and application to water surface after flooding), other soil spraying treatment (for example, spraying of a granular formulation on leaves at a growing stage, spraying under a canopy or around a tree stem, spraying on the soil surface, mixing with surface soil, spraying into seed holes, spraying on the ground surfaces of furrows, and spraying between plants), other irrigation treatment (for example, soil irrigation, irrigation at a seedling raising stage, chemical solution injection treatment, irrigation of a plant part just above the ground, chemical solution drip irrigation, and chemigation), seedling raising box treatment (for example, spraying into a seedling raising box, irrigation of a seedling raising box, and flooding into a seedling raising box with chemical solution), seedling raising tray treatment (for example, spraying on a seedling raising tray, irrigation of a seedling raising tray, and flooding into a seedling raising tray with chemical solution), seedbed treatment (for example, spraying on a seedbed, irrigation of a seedbed, spraying on a lowland rice nursery, and immersion of seedlings), seedbed soil incorporation treatment (for example, mixing with seedbed soil, mixing with seedbed soil before sowing, spraying at sowing before covering with soils, spraying at sowing after covering with soils, and mixing with covering with soils), and other treatment (for example, mixing with culture soil, plowing under, mixing with surface soil, mixing with soil at the place where raindrops fall from a canopy, treatment at a planting position, spraying of a granule formulation on flower clusters, and mixing with a paste fertilizer).

Examples of the seed treatment include application of the Present compound, the Compound of the present invention, or the Composition A to seeds or vegetative reproductive organs. Specific examples thereof include spray treatment wherein mist of a suspension of the Present compound, the Compound of the present invention, or the Composition A is sprayed to seed surfaces or vegetative reproductive organ surfaces; smear treatment wherein the Present compound, the Compound of the present invention, or the Composition A is smeared to seeds or vegetative reproductive organs; immersion treatment wherein seeds or vegetative reproductive organs are immersed in a drug solution of the Present compound, the Compound of the present invention, or the Composition A for a period of time; and methods for coating seeds or vegetative reproductive organs by a carrier comprising the Present compound, the Compound of the present invention, or the Composition A (for example, film coat treatment and pellet coat treatment). Examples of the above vegetative reproductive organ include seed potato.

In the present invention, the seed or the vegetative reproductive organ holding the Present compound, the Compound of the present invention, or the Composition A means a seed or a vegetative reproductive organ in which the Present compound, the Compound of the present invention, or the Composition A is attached to the surface of the seed or the vegetative reproductive organ. A material other than the Present compound, the Compound of the present invention, or the Composition A may be attached to the above seed or vegetative reproductive organ holding the Present compound, the Compound of the present invention, or the Composition A before or after the Present compound, the Compound of the present invention, or the Composition A is attached to the seed or the vegetative reproductive organ.

Also, when the Composition A is attached to surfaces of seeds or vegetative reproductive organs to form layer(s), said layer(s) consist(s) of a layer or a plurality of layers. When said layer(s) consist(s) of a plurality of layers, each layer consists of a layer comprising one or more active ingredient(s), or consists of a layer comprising one or more active ingredient(s) and a layer comprising no active ingredient.

The seeds or the vegetative reproductive organs holding the Present compound, the Compound of the present invention, or the Composition A may be prepared by, for example, applying a formulation comprising the Present compound, the Compound of the present invention, or the Composition A to seeds or vegetative reproductive organs by the above seed treatment method.

The amount of the Present compound, the Compound of the present invention, or the Composition A to be applied varies depending on the climate condition, the dosage form, the application period, the application method, the application site, diseases to be controlled, crops to be protected, and the like, and is usually within the range of 1 to 500 g per 1000 m² in case of foliage treatment or soil treatment. In case of seed treatment, the amount of the Present compound, the Compound of the present invention, or the Composition A is usually within the range of 0.001 to 100 g per 1 Kg of seed. When the Present compound, the Compound of the present invention, or the Composition A is formulated into an emulsifiable concentrate, a wettable powder, a flowable, or the like, such formulation is usually applied after diluting it with water in such a way that a concentration of the active ingredient is within the range of 0.01 to 10000 ppm, and then sparging it. In the case of being formulated into a dust formulation, a granule, or the like, such formulation is usually used as itself without diluting it.

The Present compound, the Compound of the present invention, or the Composition A may be used as an agent for controlling plant diseases in croplands such as fields, paddy fields, grasses, and orchards. Examples of the plants include the followings.
corn (dent corn, flint corn, flour corn, popcorn, waxy corn, sweet corn, and field corn), rice (long grain rice, short grain rice, medium grain rice, japonica rice, tropical japonica rice, indica rice, javanica rice, paddy rice, upland rice, floating rice, direct-seeded rice, transplanted rice, and glutinous rice), wheat (bread wheat (hard wheat, soft wheat, medium wheat, red wheat, and white wheat), durum wheat, spelt wheat, and club wheat, winter wheat and spring wheat of them), barley (two-rowed barley (= barley for brewery), six-rowed barley, hull-less barley, and pearl barley, winter barley and spring barley of them), rye (winter rye and spring rye), triticale (winter triticale and spring triticale), oat (winter oat and spring oat), sorghum, cotton (upland cotton and Pima cotton), soybean (ripe seed harvest soybean, green soybeans, and early harvest soybeans, indeterminate type, determinate type, and semi-determinate type of them), peanut, buckwheat, beet (beets for sugar production, beets for feed, beets for root vegetable, beets for leaf vegetable, and beets for fuel), rapeseed (winter rapeseed and spring rapeseed), canola (winter canola and spring canola), sunflower (sunflowers for oil extraction, edible sunflowers, and sunflowers for ornamental purpose), sugar cane, tobacco, tea, mulberry, solanaceous vegetables (for example, eggplant, tomato, pimento, pepper, and potato), cucurbitaceous vegetables (for example, cucumber, pumpkin, zucchini, water melon, and melon), cruciferous vegetables (for example, Japanese radish, white turnip, horseradish, kohlrabi, Chinese cabbage, cabbage, leaf mustard, broccoli, and cauliflower), asteraceous vegetables (for example, burdock, crown daisy, artichoke, and lettuce), liliaceous vegetables (for example, welsh onion, onion, garlic, and asparagus), ammiaceous vegetables (for example, carrot, parsley, celery, and parsnip), chenopodiaceous vegetables (for example, spinach and Swiss chard), lamiaceous vegetables (for example, perilla, mint, and basil), strawberry, sweet potato, glutinous yam, eddoe, pomaceous fruits (for example, apple, pear, Japanese pear, Chinese white pear, Chinese quince, and quince), stone fleshy fruits (for example, peach, plum, nectarine, Japanese apricot (Prunus mume), cherry fruit, apricot, and prune), citrus fruits (for example, Citrus unshiu, orange, lemon, lime, and grapefruit), nuts (for example, chestnuts, walnuts, hazelnuts, almond, pistachio, cashew nuts, and macadamia nuts), berry fruits (for example, blueberry, cranberry, blackberry, and raspberry), grapes, Japanese persimmon, fig, olive, Japanese plum, banana, coffee, date palm, coconuts, ornamental plants, forest plants, turfs, grasses, and the others.

The above plants are not specifically limited as long as they are generally cultivated cultivars. The above plants also include plants which may be produced by natural breeding, plants which may be generated by mutation, F1 hybrid plants, and genetically modified crops. Examples of the genetically modified crops include plants which have resistance to HPPD (4-hydroxyphenylpyruvate dioxygenase enzyme) inhibitors such as isoxaflutole, ALS (acetolactate synthase) inhibitors such as imazethapyr and thifensulfuron-methyl, EPSP (5-enolpyruvylshikimate-3-phosphate synthase) inhibitors, glutamine synthetase inhibitors, PPO (protoporphyrinogen oxidase) inhibitors, or herbicide such as bromoxynil and dicamba; plants which can synthesize a selective toxin known in Bacillus spp. such as Bacillus thuringiensis or the like; and plants which can synthesize a gene fragment or the like which is partially identical to an endogenous gene derived from a harmful insect, and induce a gene silencing (RNAi; RNA interference) in the target harmful insect to achieve a specific insecticidal activity.

### EXAMPLES

Hereinafter, the present invention is illustrated more in detail by Preparation Examples, Reference Preparation Examples, Formulation Examples, and Test Example, but the present invention is not limited to these Examples only.

In the present description, Me represents a methyl group, Et represents an ethyl group, Pr represents a propyl group, i-Pr represents an isopropyl group, c-Pr represents a cyclopropyl group, Bu represents a butyl group, i-Bu represents an isobutyl group, s-Bu represents a sec-butyl group, Ph represents a phenyl group, Bn represents a benzyl group, 2-Thie represents a 2-thienyl group, 2-Fura represents a 2-furanyl group, 3-Fura represents a 3-furanyl group, 3-Pyraz represents a pyrazol-3-yl group, 4-Pyraz represents a pyrazol-4-yl group, 5-Pyraz represents a pyrazol-5-yl group, 2-Imidaz represents an imidazol-2-yl group, 4-Imidaz represents an imidazol-4-yl group, 3-Py represents a 3-pyridyl group, and 1-Bnfura-2-yl represents a 1-benzofuran-2-yl group. When Ph, 2-Thie, 2-Fura, 3-Pyraz, 4-Pyraz, 5-Pyraz, 2-Imidaz, and 3-Py are substituted with substituent(s), the substituent(s) is/are indicated before the symbol with the substitution position(s). For example, 2-Me-Ph represents a 2-methylphenyl group, 2,6-Cl₂-Ph represents a 2,6-dichlorophenyl group, 1-Me-4-Imidaz represents a 1-methyl-imidazol-4-yl group, and 5-Cl-2-Thie represents a 5-chloro-a 2-thienyl group.

When a physical property of a compound is measured by liquid chromatography / mass spectrometry (hereinafter referred to as "LCMS"), the measured molecular ion value [M+H]⁺ or [M-H]⁻, and retention time (hereinafter referred to as "RT") are described. The conditions of liquid chromatography (hereinafter referred to as "LC") and mass spectrometry (hereinafter referred to as "MS") are as follows.

### [LC conditions]

Column: L-column2 ODS, inner diameter: 4.6 mm, length: 30 mm, particle size: 3 µm (Chemicals Evaluation and Research Institute, Japan)
UV measurement wavelength: 254 nm
Mobile phase: Solution A: 0.1% formic acid in water,
Solution B: 0.1% formic acid in acetonitrile
Flow rate: 2.0 mL/min
Pump: two LC-20ADs (manufactured by Shimadzu Corporation)
(high-pressure gradient)
Gradient conditions: sending a solution with the concentration gradient described in Table LC1.

**Table LC1**

| Time (min) | Solution A (%) | Solution B (%) |
|---|---|---|
| 0.01 | 90 | 10 |
| 2.00 | 0 | 100 |
| 4.00 | 0 | 100 |
| 4.01 | 90 | 10 |

### [MS conditions]

Detector: LCMS-2020 (manufactured by Shimadzu Corporation)
Ionization method: DUIS

First, Preparation Examples of the Present compounds (including the Compounds of the present invention) are shown below.

### Preparation Example 1

To a mixture of 3-(4-chlorophenyl)-1H-pyrazole (0.30 g), triethylamine (0.62 mL), and THF (5 mL) was added p-toluenesulfonyl chloride (0.84 g), and the resulting mixture was stirred at 60°C for 5 hours. The resulting mixture was subjected to silica gel column chromatography (hexane : ethyl acetate = 7 : 3) to give the Present compound 1 represented by the following formula (0.37 g). 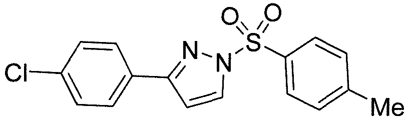 Present compound 1: ¹H-NMR (CDCl₃) δ: 8.13 (1H, d), 7.95-7.92 (2H, m), 7.76-7.72 (2H, m), 7.38-7.32 (4H, m), 6.66 (1H, d), 2.42 (3H, s).

### Preparation Example 2

To a mixture of 3-phenyl-1H-pyrazole (0.30 g), sodium hydride (oily, 60%) (0.10 g), and THF (3 mL) was added pyridine-3-sulfonyl chloride (0.44 g), and the resulting mixture was stirred at room temperature for 3 hours. To the resulting mixture was added water, and the resulting mixture was subjected to extraction with ethyl acetate. The resulting organic layer was dried over anhydrous magnesium sulfate, and concentrated under reduced pressure. The resulting residue was subjected to silica gel column chromatography (hexane : ethyl acetate = 7 : 3) to give the Present compound 55 represented by the following formula (0.53 g). 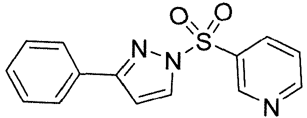 Present compound 55: ¹H-NMR (CDCl₃) δ: 9.26 (1H, d), 8.85 (1H, dd), 8.37-8.34 (1H, m), 8.15 (1H, d), 7.82-7.80 (2H, m), 7.49 (1H, ddd), 7.43-7.38 (3H, m), 6.75 (1H, d).

### Preparation Example 1-1

The compounds prepared according to the Preparation Example 1 or Preparation Example 2 and physical properties thereof are shown below.

A compound represented by formula (IA-1) 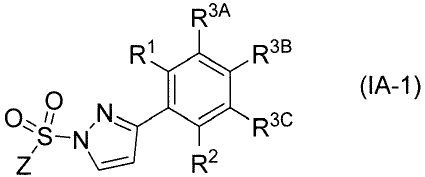 (hereinafter referred to as "Compound (IA-1)"), wherein the combination of R¹, R², R^{3A}, R^{3B}, R^{3C}, and Z represents any one combination indicated in Table A-1, Table A-2, Table A-3, and Table A-4.

**Table A-1**

| Present compound | R¹ | R² | R^{3A} | R^{3B} | R^{3C} | Z |
|---|---|---|---|---|---|---|
| 2 | H | H | Cl | Br | H | 2-Me-Ph |
| 3 | H | H | H | Cl | H | 3-Me-Ph |
| 4 | H | H | H | Cl | H | Ph |
| 5 | H | H | H | Cl | H | 2-Me-Ph |
| 6 | H | H | C(O)NH(c-Pr) | H | H | 4-Me-Ph |
| 7 | H | H | C(O)NH(c-Pr) | H | H | Ph |
| 8 | H | H | C(O)NH(Ph) | H | H | Ph |
| 9 | H | H | F | Br | F | Ph |
| 10 | H | H | F | Br | F | 3-Py |
| 11 | H | H | F | Br | F | 2-Cl-Ph |
| 12 | H | H | F | Br | F | 4-Cl-Ph |
| 13 | H | H | F | Br | F | 2-F-Ph |
| 14 | H | H | F | Br | F | 4-F-Ph |
| 15 | H | H | F | Br | F | 2, 6-Cl₂-Ph |
| 16 | H | H | F | Br | F | 3, 5-Cl₂-Ph |
| 17 | H | H | F | Br | F | 3-F-Ph |
| 18 | H | H | F | Br | F | 3-Cl-Ph |
| 19 | H | H | F | Br | F | 2-Me-Ph |
| 20 | H | H | F | Br | F | 3-Me-Ph |
| 21 | H | H | F | Br | F | 4-Me-Ph |
| 22 | H | H | F | Br | F | 4-OMe-Ph |
| 23 | H | H | F | Br | F | 3-OMe-Ph |
| 24 | H | H | F | Br | F | 2-OMe-Ph |
| 25 | H | H | F | Br | F | 2-CN-Ph |
| 26 | H | H | F | Br | F | 3-CN-Ph |
| 27 | H | H | F | Br | F | 4-CN-Ph |
| 28 | H | H | F | H | H | 4-Me-Ph |
| 29 | H | H | H | F | H | 4-Me-Ph |
| 30 | H | H | F | Br | F | 1-Bnfura-2-yl |

**Table A-2**

| Present compound | R¹ | R² | R^{3A} | R^{3B} | R^{3 C} | Z |
|---|---|---|---|---|---|---|
| 31 | H | H | CONH(c-Pr) | H | H | 1-Me-4-Imidaz |
| 32 | H | H | F | Br | F | 1-Me-4-Imidaz |
| 33 | H | H | F | Br | F | 2-Thie |
| 34 | H | H | F | Br | F | 1-Me-3-Pyraz |
| 35 | H | H | F | Br | F | 1-Me-4-Pyraz |
| 36 | H | H | F | Br | F | 3 - Fura |
| 37 | H | H | F | Br | F | 2 - Fura |
| 38 | H | H | F | Br | F | 1-Me-2-Imidaz |
| 39 | H | H | F | CF₃ | H | 2-Thie |
| 40 | H | H | F | Br | H | 2-Thie |
| 41 | H | H | H | SMe | H | 2-Thie |
| 42 | H | H | H | c-Pr | H | 2-Thie |
| 43 | H | H | H | Me | H | 2-Thie |
| 44 | H | H | H | NO₂ | H | 2-Thie |
| 45 | H | H | H | C(O)OMe | H | 2-Thie |
| 46 | H | H | H | NMe₂ | H | 2-Thie |
| 47 | H | H | F | Br | F | 5-Cl-2-Thie |
| 48 | H | H | F | Br | F | 1-Me-5-Pyraz |
| 49 | H | H | H | Br | H | 2-Fura |
| 50 | H | H | F | Br | H | 2-Fura |
| 51 | H | H | H | Br | H | 3-Fura |
| 52 | H | H | F | Br | H | 3-Fura |
| 53 | H | H | F | Br | H | 3-Py |
| 54 | H | H | F | Br | F | 2-Cl-3-Py |
| 56 | H | H | F | H | H | 3-Py |
| 57 | H | H | H | F | H | 3-Py |
| 58 | H | H | H | OMe | H | 3-Py |

**Table A-3**

| Present compound | 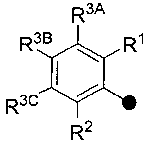 | Z | Present compound | 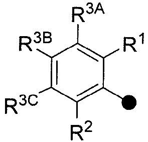 | Z |
|---|---|---|---|---|---|
| 61 | 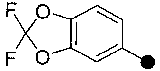 | 4-Me-Ph | 64 | 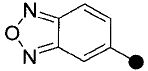 | 2-Thie |
| 62 | 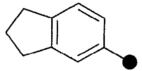 | 2-Thie | 65 | 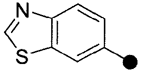 | 2-Thie |
| 63 | 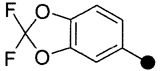 | 2-Thie | | | |

**Table A-4**

| Present compound | R¹ | R² | R^{3A} | R^{3B} | R^{3 C} | Z |
|---|---|---|---|---|---|---|
| 69 | H | H | F | Me | H | 2-Fura |
| 70 | H | H | F | Me | H | 3-Fura |
| 71 | H | H | F | Me | H | 2-Thie |
| 72 | H | H | F | Br | F | 5-Me-2-Fura |
| 73 | H | H | F | H | F | 2-Fura |
| 74 | H | H | F | H | F | 3-Fura |
| 75 | H | H | F | H | F | 2-Thie |
| 76 | H | H | H | Me | H | 2-Fura |
| 77 | H | H | H | Me | H | 3-Fura |
| 78 | H | H | H | Me | H | 2-Thie |
| 79 | H | H | H | Cl | H | 2-Fura |
| 80 | H | H | H | Cl | H | 3-Fura |
| 81 | H | H | H | Cl | H | 2-Thie |
| 82 | H | H | H | I | H | 2-Fura |
| 83 | H | H | H | I | H | 3-Fura |
| 84 | H | H | H | I | H | 2-Thie |
| 85 | H | H | H | H | H | 2-Fura |
| 86 | H | H | H | H | H | 3-Fura |
| 87 | H | H | H | H | H | 2-Thie |
| 88 | H | H | H | F | H | 2-Fura |
| 89 | H | H | H | F | H | 3-Fura |
| 90 | H | H | H | F | H | 2-Thie |
| 91 | H | H | F | F | H | 2-Fura |
| 92 | H | H | F | F | H | 3-Fura |
| 93 | H | H | F | F | H | 2-Thie |
| 94 | H | H | F | H | H | 2-Fura |
| 95 | H | H | F | H | H | 3-Fura |
| 96 | H | H | F | H | H | 2-Thie |
| 97 | H | H | F | Me | H | 3-Py |
| 98 | H | H | F | H | F | 3-Py |
| 99 | H | H | H | Me | H | 3-Py |
| 100 | H | H | F | Br | F | 5-F-3-Py |
| 101 | H | H | F | F | H | 3-Py |
| 102 | H | H | F | H | H | 3-Py |

Present compound 2: ¹H-NMR (CDCl₃) δ: 8.21 (1H, d), 8.13 (1H, dd), 7.88 (1H, d), 7.62 (1H, d), 7.57-7.51 (2H, m), 7.41 (1H, t), 7.32 (1H, d), 6.70 (1H, d), 2.67 (3H, s).
Present compound 3: ¹H-NMR (CDCl₃) δ: 8.14 (1H, d), 7.86-7.83 (2H, m), 7.76-7.73 (2H, m), 7.45-7.42 (2H, m), 7.38-7.35 (2H, m), 6.68 (1H, d), 2.43 (3H, s).
Present compound 4: ¹H-NMR (CDCl₃) δ: 8.15 (1H, d), 8.08-8.05 (2H, m), 7.83-7.82 (1H, m), 7.69-7.63 (2H, m), 7.59-7.54 (2H, m), 7.34-7.32 (2H, m), 6.70 (1H, d).
Present compound 5: LCMS [M+H]⁺ = 333, RT=2.26 min
Present compound 6: ¹H-NMR (CDCl₃) δ: 8.15-8.14 (2H, m), 7.93 (2H, dt), 7.90 (1H, dt), 7.76 (1H, dt), 7.45 (1H, t), 7.34 (2H, d), 6.75 (1H, d), 6.33 (1H, s), 2.96-2.89 (1H, m), 2.42 (3H, s), 0.92-0.88 (2H, m), 0.68-0.64 (2H, m).
Present compound 7: ¹H-NMR (CDCl₃) δ: 8.17-8.15 (2H, m), 8.08-8.05 (2H, m), 7.91 (1H, dq), 7.76 (1H, dq), 7.66 (1H, tt), 7.58-7.52 (2H, m), 7.46 (1H, t), 6.77 (1H, d), 6.32 (1H, s), 2.96-2.90 (1H, m), 0.92-0.88 (2H, m), 0.68-0.64 (2H, m).
Present compound 8: ¹H-NMR (CDCl₃) δ: 8.31 (1H, t), 8.19 (1H, d), 8.09-8.06 (2H, m), 7.97 (1H, dt), 7.89 (2H, dt), 7.69-7.64 (3H, m), 7.59-7.52 (3H, m), 7.43-7.38 (2H, m), 7.20-7.16 (1H, m), 6.80 (1H, d).
Present compound 9: ¹H-NMR (CDCl₃) δ: 8.17 (1H, d), 8.09-8.06 (2H, m), 7.70-7.66 (1H, m), 7.60-7.55 (2H, m), 7.43-7.39 (2H, m), 6.67 (1H, d).
Present compound 10: ¹H-NMR (CDCl₃) δ: 9.27 (1H, s), 8.91 (1H, d), 8.39-8.36 (1H, m), 8.19 (1H, d), 7.54 (1H, dd), 7.42-7.38 (2H, m), 6.71 (1H, d).
Present compound 11: ¹H-NMR (CDCl₃) δ: 8.41-8.35 (2H, m), 7.63 (1H, td), 7.55 (1H, td), 7.51 (1H, dd), 7.37 (2H, d), 6.70 (1H, d).
Present compound 12: ¹H-NMR (CDCl₃) δ: 8.16 (1H, d), 8.01 (2H, d), 7.54 (2H, d), 7.40 (2H, d), 6.68 (1H, d).
Present compound 13: ¹H-NMR (CDCl₃) δ: 8.30 (1H, dd), 8.18 (1H, m), 7.73-7.66 (1H, m), 7.43-7.37 (3H, m), 7.20 (1H, m), 6.71 (1H, d).
Present compound 14: ¹H-NMR (CDCl₃) δ: 8.16 (1H, d), 8.14-8.07 (2H, m), 7.40 (2H, d), 7.27-7.21 (2H, m), 6.68 (1H, d).
Present compound 15: ¹H-NMR (CDCl₃) δ: 8.28 (1H, d), 7.68-7.60 (1H, m), 7.42 (2H, d), 7.07 (2H, t), 6.73 (1H, d).
Present compound 16: ¹H-NMR (CDCl₃) δ: 8.16 (1H, d), 7.63-7.59 (2H, m), 7.42 (2H, d), 7.12 (1H, tt), 6.72 (1H, d).
Present compound 17: ¹H-NMR (CDCl₃) δ: 8.17 (1H, d), 7.88 (1H, d), 7.76 (1H, dt), 7.57 (1H, td), 7.44-7.35 (3H, m), 6.70 (1H, d).
Present compound 18: ¹H-NMR (CDCl₃) δ: 8.17 (1H, d), 8.03 (1H, t), 7.97 (1H, dq), 7.64 (1H, dq), 7.52 (1H, t), 7.41 (2H, d), 6.70 (1H, d).
Present compound 19: ¹H-NMR (CDCl₃) δ: 8.23 (1H, d), 8.15 (1H, dd), 7.56 (1H, td), 7.42 (1H, t), 7.38 (2H, d), 7.33 (1H, d), 6.69 (1H, d), 2.68 (3H, s).
Present compound 20: ¹H-NMR (CDCl₃) δ: 8.17 (1H, d), 7.86 (2H, d), 7.48-7.44 (2H, d), 7.41 (2H, d), 6.67 (1H, d), 2.44 (3H, s).
Present compound 21: ¹H-NMR (CDCl₃) δ: 8.16 (1H, d), 7.94 (2H, d), 7.40 (2H, d), 7.36 (2H, d), 6.65 (1H, d), 2.43 (3H, s).
Present compound 22: ¹H-NMR (CDCl₃) δ: 8.15 (1H, d), 8.00 (2H, d), 7.40 (2H, d), 7.01 (2H, d), 6.64 (1H, d), 3.87 (3H, s).
Present compound 23: ¹H-NMR (CDCl₃) δ: 8.16 (1H, d), 7.63 (1H, d), 7.55 (1H, m), 7.46 (1H, t), 7.41 (2H, d), 7.18 (1H, dd), 6.67 (1H, d), 3.87 (3H, s).
Present compound 24: ¹H-NMR (CDCl₃) δ: 8.30 (1H, d), 8.18 (1H, dd), 7.64 (1H, m), 7.39 (2H, d), 7.16 (1H, td), 6.96 (1H, d), 6.66 (1H, d), 3.81 (3H, s).
Present compound 25: ¹H-NMR (CDCl₃) δ: 8.47-8.43 (2H, m), 7.92-7.79 (3H, m), 7.38 (2H, d), 6.75 (1H, d).
Present compound 26: ¹H-NMR (CDCl₃) δ: 8.34-8.30 (2H, m), 8.19 (1H, d), 7.95 (1H, dt), 7.75 (1H, dd), 7.40 (2H, d), 6.72 (1H, d).
Present compound 27: ¹H-NMR (CDCl₃) δ: 8.22-8.16 (3H, m), 7.87 (2H, d), 7.39 (2H, d), 6.71 (1H, d).
Present compound 28: ¹H-NMR (CDCl₃) δ: 8.14 (1H, d), 7.96-7.93 (2H, m), 7.58-7.51 (2H, m), 7.38-7.33 (3H, m), 7.05 (1H, tdd), 6.68 (1H, d), 2.42 (3H, s).
Present compound 29: ¹H-NMR (CDCl₃) δ: 8.12 (1H, d), 7.95-7.91 (2H, m), 7.81-7.75 (2H, m), 7.33 (2H, d), 7.10-7.04 (2H, m), 6.64 (1H, d), 2.41 (3H, s).
Present compound 30: ¹H-NMR (CDCl₃) δ: 8.29 (1H, d), 7.81 (1H, d), 7.76-7.73 (1H, m), 7.56-7.49 (2H, m), 7.44-7.40 (2H, m), 7.40-7.36 (1H, m), 6.75 (1H, d).
Present compound 31: ¹H-NMR (CDCl₃) δ: 8.28 (1H, d), 8.17 (1H, t), 7.90-7.87 (1H, m), 7.80 (1H, d), 7.79-7.76 (1H, m), 7.47-7.43 (2H, m), 6.77 (1H, d), 6.35 (1H, s), 3.77 (3H, s), 2.95-2.89 (1H, m), 0.92-0.87 (2H, m), 0.68-0.64 (2H, m).
Present compound 32: ¹H-NMR (CDCl₃) δ: 8.29 (1H, d), 7.83 (1H, d), 7.49 (1H, d), 7.42 (2H, d), 6.69 (1H, d), 3.79 (3H, s).
Present compound 33: ¹H-NMR (CDCl₃) δ: 8.16 (1H, d), 7.91 (1H, dd), 7.77 (1H, dd), 7.43 (2H, d), 7.15 (1H, dd), 6.68 (1H, d).
Present compound 34: ¹H-NMR (CDCl₃) δ: 8.24 (1H, d), 7.47-7.41 (3H, m), 6.98 (1H, d), 6.70 (1H, d), 3.98 (3H, s). Present compound 35: ¹H-NMR (CDCl₃) δ: 8.14 (1H, d), 8.04 (1H, s), 7.91 (1H, d), 7.42 (2H, d), 6.66 (1H, d), 3.97 (3H, s).
Present compound 36: ¹H-NMR (CDCl₃) δ: 8.20 (1H, s), 8.15 (1H, d), 7.51 (1H, t), 7.44 (2H, d), 6.77 (1H, dd), 6.70 (1H, d).
Present compound 37: ¹H-NMR (CDCl₃) δ: 8.22 (1H, d), 7.63 (1H, dd), 7.46 (1H, dd), 7.43 (2H, d), 6.72 (1H, d), 6.60 (1H, dd).
Present compound 38: ¹H-NMR (CDCl₃) δ: 8.26 (1H, d), 7.39 (2H, d), 7.19 (1H, d), 7.10 (1H, s), 6.73 (1H, d), 4.21 (3H, s).
Present compound 39: ¹H-NMR (CDCl₃) δ: 8.18 (1H, d), 7.92 (1H, dd), 7.77 (1H, dd), 7.71-7.62 (3H, m), 7.15 (1H, dd), 6.74 (1H, d).
Present compound 40: ¹H-NMR (CDCl₃) δ: 8.15 (1H, d), 7.90 (1H, dd), 7.75 (1H, dd), 7.63-7.56 (2H, m), 7.48 (1H, dq), 7.14 (1H, dd), 6.69 (1H, d).
Present compound 41: ¹H-NMR (CDCl₃) δ: 8.11 (1H, d), 7.87 (1H, dd), 7.77-7.74 (2H, m), 7.72 (1H, dd), 7.28-7.25 (2H, m), 7.11 (1H, dd), 6.69 (1H, d), 2.51 (3H, s).
Present compound 42: ¹H-NMR (CDCl₃) δ: 8.10 (1H, d), 7.86 (1H, dd), 7.74-7.69 (3H, m), 7.11-7.08 (3H, m), 6.68 (1H, d), 1.94-1.88 (1H, m), 1.02-0.97 (2H, m), 0.74-0.70 (2H, m).
Present compound 43: ¹H-NMR (CDCl₃) δ: 8.11 (1H, d), 7.87 (1H, dd), 7.74-7.69 (3H, m), 7.22-7.20 (2H, m), 7.10 (1H, dd), 6.69 (1H, d), 2.37 (3H, s).
Present compound 44: ¹H-NMR (CDCl₃) δ: 8.29-8.26 (2H, m), 8.20 (1H, d), 8.03-7.99 (2H, m), 7.93 (1H, dd), 7.78 (1H, dd), 7.16 (1H, dd), 6.81 (1H, d).
Present compound 45: ¹H-NMR (CDCl₃) δ: 8.16 (1H, d), 8.09-8.06 (2H, m), 7.92-7.90 (3H, m), 7.75 (1H, dd), 7.13 (1H, dd), 6.78 (1H, d), 3.94 (3H, s).
Present compound 46: ¹H-NMR (CDCl₃) δ: 8.06 (1H, d), 7.84 (1H, dd), 7.74-7.70 (2H, m), 7.67 (1H, dd), 7.08 (1H, dd), 6.71 (2H, d), 6.64 (1H, d), 2.99 (6H, s).
Present compound 47: ¹H-NMR (CDCl₃) δ: 8.13 (1H, d), 7.71 (1H, d), 7.46-7.42 (2H, m), 6.98 (1H, d), 6.70 (1H, d).
Present compound 48: ¹H-NMR (CDCl₃) δ: 8.16 (1H, d), 7.53 (1H, d), 7.43-7.38 (2H, m), 7.00 (1H, d), 6.7 4 (1H, d), 4.30 (3H, s).
Present compound 49: ¹H-NMR (CDCl₃) δ: 8.19 (1H, d), 7.72-7.69 (2H, m), 7.61 (1H, dd), 7.55-7.52 (2H, m), 7.43 (1H, dd), 6.74 (1H, d), 6.58 (1H, dd).
Present compound 50: ¹H-NMR (CDCl₃) δ: 8.20 (1H, d), 7.63-7.56 (3H, m), 7.48 (1H, ddd), 7.45 (1H, dd), 6.73 (1H, d), 6.59 (1H, dd).
Present compound 51: ¹H-NMR (CDCl₃) δ: 8.18 (1H, dd), 8.12 (1H, d), 7.73-7.70 (2H, m), 7.56-7.53 (2H, m), 7.48 (1H, t), 6.75 (1H, dd), 6.71 (1H, d).
Present compound 52: ¹H-NMR (CDCl₃) δ: 8.19 (1H, dd), 8.14 (1H, d), 7.64-7.57 (2H, m), 7.50-7.47 (2H, m), 6.76 (1H, dd), 6.71 (1H, d).
Present compound 53: ¹H-NMR (CDCl₃) δ: 9.25 (1H, dd), 8.88 (1H, dd), 8.37 (1H, ddd), 8.18 (1H, d), 7.60-7.57 (2H, m), 7.52 (1H, ddd), 7.46 (1H, ddd), 6.72 (1H, d).
Present compound 54: ¹H-NMR (CDCl₃) δ: 8.72-8.66 (2H, m), 8.40 (1H, d), 7.57 (1H, dd), 7.38-7.34 (2H, m), 6.74 (1H, d).
Present compound 56: ¹H-NMR (CDCl₃) δ: 9.26 (1H, dd), 8.87 (1H, dd), 8.37 (1H, ddd), 8.17 (1H, d), 7.58-7.50 (3H, m), 7.40-7.35 (1H, m), 7.08 (1H, m), 6.73 (1H, d).
Present compound 57: ¹H-NMR (CDCl₃) δ: 9.25 (1H, dd), 8.86 (1H, dd), 8.35 (1H, ddd), 8.15 (1H, d), 7.81-7.76 (2H, m), 7.52-7.49 (1H, m), 7.13-7.07 (2H, m), 6.70 (1H, d).
Present compound 58: ¹H-NMR (CDCl₃) δ: 9.24 (1H, dd), 8.84 (1H, dd), 8.34 (1H, ddd), 8.12 (1H, d), 7.76-7.73 (2H, m), 7.49 (1H, ddd), 6.92 (2H, dd), 6.69 (1H, d), 3.84 (3H, s).
Present compound 61: ¹H-NMR (CDCl₃) δ: 8.13 (1H, d), 7.95-7.92 (2H, m), 7.57 (1H, d), 7.49 (1H, dd), 7.34 (2H, d), 7.06 (1H, d), 6.63 (1H, d), 2.42 (3H, s).
Present compound 62: ¹H-NMR (CDCl₃) δ: 8.10 (1H, d), 7.86 (1H, dd), 7.73 (1H, s), 7.70 (1H, dd), 7.59 (1H, dd), 7.27-7.24 (1H, m), 7.10 (1H, dd), 6.69 (1H, d), 2.93 (4H, q), 2.13-2.06 (2H, m).
Present compound 63: ¹H-NMR (CDCl₃) δ: 8.13 (1H, d), 7.89 (1H, dd), 7.74 (1H, dd), 7.60 (1H, d), 7.53 (1H, dd), 7.13 (1H, dd), 7.08 (1H, d), 6.66 (1H, d).
Present compound 64: ¹H-NMR (CDCl₃) δ: 8.22 (1H, d), 8.16 (1H, t), 8.11 (1H, dd), 7.94 (1H, dd), 7.89 (1H, dd), 7.79 (1H, dd), 7.16 (1H, dd), 6.86 (1H, d).
Present compound 65: ¹H-NMR (CDCl₃) δ: 9.03 (1H, s), 8.49 (1H, dd), 8.18 (1H, d), 8.16 (1H, dd), 7.98 (1H, dd), 7.92 (1H, dd), 7.74 (1H, dd), 7.14 (1H, dd), 6.81 (1H, d).
Present compound 69: ¹H-NMR (CDCl₃) δ: 8.18 (1H, d), 7.60 (1H, dd), 7.51-7.47 (2H, m), 7.43 (1H, dd), 7.23-7.19 (1H, m), 6.72 (1H, d), 6.58 (1H, dd), 2.29 (3H, d).
Present compound 70: ¹H-NMR (CDCl₃) δ: 8.18 (1H, dd), 8.11 (1H, d), 7.52-7.47 (3H, m), 7.24-7.20 (1H, m), 6.76 (lH, dd), 6.69 (1H, d), 2.30 (3H, d).
Present compound 71: ¹H-NMR (CDCl₃) δ: 8.12 (1H, d), 7.88 (1H, dd), 7.73 (1H, dd), 7.52-7.47 (2H, m), 7.23-7.19 (1H, m), 7.12 (1H, dd), 6.67 (1H, d), 2.29 (3H, d).
Present compound 72: ¹H-NMR (CDCl₃) δ: 8.20 (1H, d), 7.46-7.42 (2H, m), 7.38-7.37 (1H, m), 6.71 (1H, d), 6.22-6.21 (1H, m), 2.37 (3H, s).
Present compound 73: ¹H-NMR (CDCl₃) δ: 8.21 (1H, d), 7.62 (1H, dd), 7.46 (1H, dd), 7.38-7.32 (2H, m), 6.82 (1H, tt), 6.73 (1H, d), 6.60 (1H, dd).
Present compound 74: ¹H-NMR (CDCl₃) δ: 8.20 (1H, dd), 8.15 (1H, d), 7.50 (1H, dd), 7.39-7.34 (2H, m), 6.83 (1H, tt), 6.77 (1H, dd), 6.70 (1H, d).
Present compound 75: ¹H-NMR (CDCl₃) δ: 8.16 (1H, d), 7.91 (1H, dd), 7.76 (1H, dd), 7.39-7.33 (2H, m), 7.14 (1H, dd), 6.82 (1H, tt), 6.68 (1H, d).
Present compound 76: ¹H-NMR (CDCl₃) δ: 8.17 (1H, d), 7.72 (2H, dd), 7.59 (1H, dd), 7.41 (1H, dd), 7.21 (2H, dd), 6.74 (1H, d), 6.56 (1H, dd), 2.37 (3H, s).
Present compound 77: ¹H-NMR (CDCl₃) δ: 8.16 (1H, dd), 8.10 (1H, d), 7.73 (2H, d), 7.46 (1H, t), 7.22 (2H, t), 6.75 (1H, dd), 6.71 (1H, d), 2.38 (3H, s).
Present compound 78: ¹H-NMR (CDCl₃) δ: 8.11 (1H, d), 7.87 (1H, dd), 7.73 (2H, dd), 7.70 (1H, dd), 7.21 (2H, t), 7.10 (1H, dd), 6.70 (1H, d), 2.37 (3H, s).
Present compound 79: ¹H-NMR (CDCl₃) δ: 8.19 (1H, d), 7.77 (2H, dt), 7.61 (1H, dd), 7.43 (1H, dd), 7.38 (2H, dt), 6.74 (1H, d), 6.58 (1H, dd).
Present compound 80: ¹H-NMR (CDCl₃) δ: 8.18 (1H, dd), 8.12 (1H, d), 7.78 (2H, dt), 7.48 (1H, t), 7.39 (2H, dt), 6.75 (1H, dd), 6.71 (1H, d).
Present compound 81: ¹H-NMR (CDCl₃) δ: 8.13 (1H, d), 7.89 (1H, dd), 7.77 (2H, dt), 7.73 (1H, dd), 7.38 (2H, dd), 7.12 (1H, dd), 6.69 (1H, d).
Present compound 82: ¹H-NMR (CDCl₃) δ: 8.19 (1H, d), 7.74 (2H, dt), 7.61 (1H, dd), 7.57 (2H, dt), 7.43 (1H, dd), 6.74 (1H, d), 6.58 (1H, dd).
Present compound 83: ¹H-NMR (CDCl₃) δ: 8.18 (1H, dd), 8.12 (1H, d), 7.76 (2H, dt), 7.58 (2H, dt), 7.48 (1H, t), 6.75 (1H, dd), 6.71 (1H, d).
Present compound 84: ¹H-NMR (CDCl₃) δ: 8.13 (1H, d), 7.88 (1H, dd), 7.76-7.73 (3H, m), 7.57 (2H, dt), 7.12 (1H, dd), 6.70 (1H, d).
Present compound 85: ¹H-NMR (CDCl₃) δ: 8.19 (1H, d), 7.85-7.82 (2H, m), 7.60 (1H, dd), 7.44-7.38 (4H, m), 6.77 (1H, d), 6.57 (1H, dd).
Present compound 86: ¹H-NMR (CDCl₃) δ: 8.17 (1H, dd), 8.12 (1H, d), 7.86-7.83 (2H, m), 7.47 (1H, t), 7.45-7.38 (3H, m), 6.76 (1H, dd), 6.75 (1H, d).
Present compound 87: ¹H-NMR (CDCl₃) δ: 8.13 (1H, d), 7.88 (1H, dd), 7.86-7.82 (2H, m), 7.72 (1H, dd), 7.44-7.36 (3H, m), 7.11 (1H, dd), 6.73 (1H, d).
Present compound 88: ¹H-NMR (CDCl₃) δ: 8.18 (1H, d), 7.84-7.79 (2H, m), 7.60 (1H, dd), 7.43 (1H, dd), 7.13-7.07 (2H, m), 6.72 (1H, d), 6.58 (1H, dd).
Present compound 89: ¹H-NMR (CDCl₃) δ: 8.17 (1H, dd), 8.12 (1H, d), 7.85-7.80 (2H, m), 7.48 (1H, t), 7.14-7.08 (2H, m), 6.75 (1H, dd), 6.70 (1H, d).
Present compound 90: ¹H-NMR (CDCl₃) δ: 8.13 (1H, d), 7.88 (1H, dd), 7.84-7.80 (2H, m), 7.73 (1H, dd), 7.13-7.07 (3H, m), 6.68 (1H, d).
Present compound 91: ¹H-NMR (CDCl₃) δ: 8.19 (1H, d), 7.70-7.65 (1H, m), 7.62-7.61 (1H, m), 7.56-7.52 (1H, m), 7.44 (1H, dd), 7.23-7.16 (1H, m), 6.70 (1H, d), 6.59 (1H, dd).
Present compound 92: ¹H-NMR (CDCl₃) δ: 8.19 (1H, dd), 8.13 (1H, d), 7.68 (1H, ddd), 7.57-7.52 (1H, m), 7.49 (1H, t), 7.24-7.17 (1H, m), 6.76 (1H, dd), 6.68 (1H, d).
Present compound 93: ¹H-NMR (CDCl₃) δ: 8.14 (1H, d), 7.89 (1H, dd), 7.75 (1H, dd), 7.71-7.66 (1H, m), 7.56-7.52 (1H, m), 7.19 (1H, dd), 7.14 (1H, dd), 6.66 (1H, d).
Present compound 94: ¹H-NMR (CDCl₃) δ: 8.20 (1H, d), 7.61-7.54 (3H, m), 7.44 (1H, dd), 7.40-7.35 (1H, m), 7.07 (1H, tdd), 6.75 (1H, d), 6.58 (1H, dd).
Present compound 95: ¹H-NMR (CDCl₃) δ: 8.19 (1H, dd), 8.13 (1H, d), 7.61-7.55 (2H, m), 7.49 (1H, t), 7.38 (1H, td), 7.08 (1H, tdd), 6.77 (1H, dd), 6.72 (1H, d).
Present compound 96: ¹H-NMR (CDCl₃) δ: 8.14 (1H, d), 7.90 (1H, dd), 7.74 (1H, dd), 7.61-7.54 (2H, m), 7.37 (1H, td), 7.13 (1H, dd), 7.07 (1H, tdd), 6.71 (1H, d).
Present compound 97: ¹H-NMR (CDCl₃) δ: 9.25 (1H, dd), 8.86 (1H, dd), 8.36 (1H, ddd), 8.15 (1H, d), 7.53-7.47 (2H, m), 7.45 (1H, t), 7.21 (1H, t), 6.70 (1H, d), 2.29 (3H, d).
Present compound 98: ¹H-NMR (CDCl₃) δ: 9.26 (1H, dd), 8.88 (1H, dd), 8.38 (1H, dq), 8.19 (1H, d), 7.53 (1H, ddd), 7.36-7.30 (2H, m), 6.85-6.80 (1H, m), 6.71 (1H, d). Present compound 99: ¹H-NMR (CDCl₃) δ: 9.24 (1H, dd), 8.84 (1H, dd), 8.35 (1H, ddd), 8.13 (1H, d), 7.71-7.68 (2H, m), 7.51-7.47 (1H, m), 7.21 (2H, d), 6.72 (1H, d), 2.37 (3H, s).
Present compound 100: ¹H-NMR (CDCl₃) δ: 9.08-9.08 (1H, m), 8.75 (1H, d), 8.19 (1H, d), 8.08 (1H, ddd), 7.43-7.39 (2H, m), 6.74 (1H, d).
Present compound 101: ¹H-NMR (CDCl₃) δ: 9.25 (1H, d), 8.88 (1H, dd), 8.36 (1H, dt), 8.17 (1H, d), 7.65 (1H, ddd), 7.54-7.50 (2H, m), 7.23-7.16 (1H, m), 6.69 (1H, d).
Present compound 102: ¹H-NMR (CDCl₃) δ: 9.26 (1H, dd), 8.87 (1H, dd), 8.37 (1H, ddd), 8.17 (1H, d), 7.58-7.50 (3H, m), 7.40-7.35 (1H, m), 7.08 (1H, tdd), 6.73 (1H, d).

### Preparation Example 3

A mixture of 5-(4,4,5,5-tetramethyl-1,3,2-dioxaborolan-2-yl)benzo[d]oxazole (0.29 g), the Intermediate 1 (0.40 g), {1,1'-bis(diphenylphosphino)ferrocene}dichloropalladium(II) (0.22 g), tripotassium phosphate (0.50 g), 1,2-dimethoxyethane (6 mL), and water (0.1 mL) was stirred under reflux for 13 hours. To the resulting mixture was added water, and the resulting mixture was subjected to extraction with ethyl acetate. The resulting organic layer was concentrated under reduced pressure, and the resulting residue was subjected to silica gel column chromatography (hexane : ethyl acetate = 7 : 3) to give the Present compound 66 represented by the following formula (0.07 g). 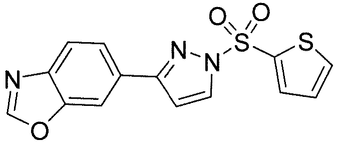 Present compound 66: ¹H-NMR (CDCl₃) δ: 8.21 (1H, d), 8.16 (1H, d), 8.13 (1H, s), 7.96 (1H, dd), 7.91 (1H, dd), 7.74 (1H, dd), 7.62 (1H, dd), 7.13 (1H, dd), 6.77 (1H, d).

### Preparation Example 3-1

The compounds prepared according to the Preparation Example 3 and physical properties thereof are shown below.

The Compound (IA-1), wherein the combination of R¹, R², R^{3A}, R^{3B}, R^{3 C}, and Z represents any one combination indicated in Table A-5.

**Table A-5**

| Present compound | 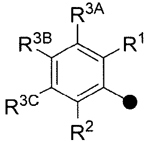 | Z | Present compound | 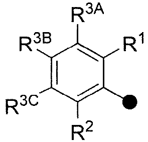 | Z |
|---|---|---|---|---|---|
| 67 | 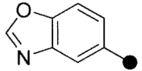 | 2-Thie | 68 | 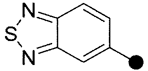 | 2-Thie |

Present compound 67: LCMS [M+H]⁺ = 332, RT = 1.75 min Present compound 68: ¹H-NMR (CDCl₃) δ: 8.37 (1H, dd), 8.25 (1H, dd), 8.21 (1H, d), 8.04 (1H, dd), 7.94 (1H, dd), 7.77 (1H, dd), 7.15 (1H, dd), 6.88 (1H, d).

### Reference Preparation Example 1

To a mixture of 3-iodo-1H-pyrazole (8.30 g), sodium hydride (oily, 60%) (2.05 g), and THF (83 mL) was added 2-thiophenesulfonylchloride (9.38 g), and the resulting mixture was stirred at room temperature for 5 hours. To the resulting mixture was added water, and the resulting mixture was subjected to extraction with ethyl acetate. The resulting organic layer was dried over anhydrous magnesium sulfate, and concentrated under reduced pressure. The resulting residue was subjected to silica gel column chromatography (hexane : ethyl acetate = 7 : 3) to give the Intermediate 1 represented by the following formula (13.5 g). 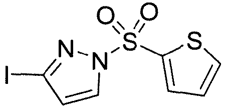 Intermediate 1: ¹H-NMR (CDCl₃) δ: 7.91 (1H, d), 7.89 (1H, dd), 7.78 (1H, dd), 7.15 (1H, dd), 6.56 (1H, d).

### Reference Preparation Example 1-1

The intermediate compounds prepared according to the Reference Preparation Example 1 and physical properties thereof are shown below.

A compound represented by formula (IIIA-1) 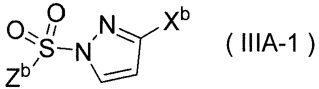 wherein the combination of X^{b} and Z^{b} represents any one combination indicated in Table A-6.

**Table A-6**

| Interme diate | X^{b} | Z^{b} |
|---|---|---|
| 2 | Br | 2-Thie |
| 3 | Cl | 2-Thie |
| 4 | I | 3-Py |

Intermediate 2: LCMS [M+H]⁺ = 293, RT = 1.75 min
Intermediate 3: LCMS [M+H]⁺ = 249, RT = 1.73 min
Intermediate 4: LCMS [M+H]⁺ = 336, RT = 1.55 min

Examples of the Present compound and the Compound of the present invention prepared according to the above Production methods and Preparation Examples are shown below.

A compound represented by formula (A-1) 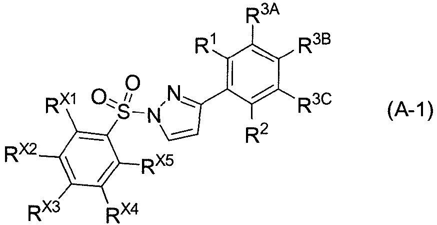 (hereinafter referred to as "Compound (A-1)"), wherein R^{X1}, R^{X 2}, R^{X 3}, R^{X 4}, and R^{X 5} each represent a hydrogen atom, and the combination of R¹, R², R^{3A}, R^{3B}, and R^{3 C} represents any one combination indicated in the Combination A (hereinafter referred to as "Compound group SX1").

Combination A consists of Substituent Numbers A1 to A300. Substituent Numbers A1 to A300 indicate the combinations of R¹, R², R^{3A}, R^{3B}, and R^{3C} in the compound represented by formula (A-1), the compound represented by formula (A-2), the compound represented by formula (A-3), the compound represented by formula (A-4), the compound represented by formula (A-5), the compound represented by formula (A-6), the compound represented by formula (A-7), the compound represented by formula (A-8), the compound represented by formula (A-9), the compound represented by formula (A-10), the compound represented by formula (A-11), and the compound represented by formula (A-12), and are hereinafter referred to as [Substituent Number; R¹, R², R^{3A}, R^{3B}, R^{3C}].

For example, Substituent Number A3 indicates a combination wherein R^{3A} represents an ethyl group, and R¹, R², R^{3B}, and R^{3C} each represent a hydrogen atom.

### Combination A

[A1;H,H,H,H,H], [A2;H,H,Me,H,H], [A3;H,H,Et,H,H], [A4;H,H,CF₃,H,H], [A5;H,H,c-Pr,H,H], [A6;H,H,F,H,H], [A7;H,H,Cl,H,H], [A8;H,H,Br,H,H], [A9;H,H, I,H,H], [A10;H,H,OMe,H,H], [A11;H,H,SMe,H,H), [A12;H,H,S(O)Me,H,H], [A13;H,H,S(O)₂Me,H,H], [A14;H,H,C(O)OMe,H,H], [A15;H,H,C(O)Me,H,H], [A16;H,H,C(O)NH₂,H,H], [A17;H,H,NMe₂,H,H], [A18;H,H,CN,H,H], [A19;H,H,NO₂,H,H], [A20;H,H,Ph,H,H], [A21;H,H,OPh,H,H], [A22;H,H,OCH₂Ph,H,H], [A23;H,H,H,Me,H], [A24;H,H,H,Et,H], [A25;H,H,H,CF₃,H], [A26;H,H,H,c-Pr,H], [A27;H,H,H,F,H], [A28;H,H,H,C1,H], [A29;H,H,H,Br,H], [A30;H,H,H,I,H], [A31;H,H,H,OMe,H], [A32;H,H,H,SMe,H], [A33;H,H,H,S(O)Me,H], [A34;H,H,H,S(O)₂Me,H], [A35;H,H,H,C(O)OMe,H], [A36;H,H,H,C(O)Me,H], [A37;H,H,H,C(O)NH₂,H], [A38;H,H,H,NMe₂,H], [A39;H,H,H,CN,H], [A40;H,H,H,NO₂,H], [A41;H,H,H,Ph,H], [A42;H,H,H,OPh,H], [A43;H,H,H,OCH,Ph,6H], [A44;Me,H,H,H,H], [A45;F,H,H,H,H], [A46;Cl,H,H,H,H], [A47;Br,H,H,H,H], [A48;OMe,H,H,H,H], [A49;H,H,Me,Me,H], [A50;H,H,Et,Me,H], [A51;H,H,CF₃,Me,H], [A52;H,H,c-Pr,Me, H], [A53;H,H,F,Me,H], [A54;H,H,Cl,Me,H], [A55;H,H,Br,Me,H], [A56;H,H,I,Me,H], [A57;H,H,OMe,Me,H], [A58;H,H,SMe,Me,H], [A59;H,H,S(O)Me,Me,H], [A60;H,H,S(O)₂Me,Me,H], [A61;H,H,C(O)OMe,Me,H], [A62;H,H,C(O)Me,Me,H], [A63;H,H,C(O)NH₂,Me,H], [A64;H,H,NMe₂,Me,H], [A65;H,H,CN,Me,H], [A66;H,H,NO₂,Me,H], [A67;H,H,Ph,Me,H], [A68;H,H,OPh,Me,H), [A69;H,H,OCH₂Ph,Me,H], [A70;H,H,Me,CF₃,H], [A71;H,H,Et,CF₃,H], [A72;H,H,CF₃,CF₃,H], [A73;H,H,c-Pr,CF₃,H], [A74;H,H,F,CF₃,H], [A75;H,H,Cl,CF₃,H], [A76;H,H,Br,CF₃,H], [A77;H,H,I,CF₃,H], [A78;H,H,OMe,CF₃,H], [A79;H,H,SMe,CF₃,H], [A80;H,H,S(O)Me,CF₃,H], [A81;H,H,S(O)₂Me,CF₃,H], [A82;H,H,C(O)OMe,CF₃,H], [A83;H,H,C(O)Me,CF₃,H], [A84;H,H,C(O)NH₂,CF₃,H], [A85;H,H,NMe₂,CF₃,H], [A86;H,H,CN,CF₃,H], [A87;H,H,NO₂,CF₃,H], [A88;H,H,Ph,CF₃,H], [A89;H,H,OPh,CF₃,H], [A90;H,H,OCH₂Ph,CF₃,H], [A91;H,H,Me,F,H], [A92;H,H,Et,F,H], [A93;H,H,CF₃,F,H], [A94;H,H,c-Pr,F,H], [A95;H,H,F,F,H], [A96;H,H,Cl,F,H], [A97;H,H,Br,F,H], [A98;H,H,I,F,H], [A99;H,H,OMe,F,H], [A100;H,H,SMe,F,H], [A101;H,H,S(O)Me,F,H], [A102;H,H,S(O)₂Me,F,H], [A103;H,H,C(O)OMe,F,H], [A104;H,H,C(O)Me,F,H], [A105;H,H,C(O)NH₂,F,H], [A106;H,H,NMe₂,F,H], [A107;H,H,CN,F,H], [A108;H,H,NO₂,F,H], [A109;H,H,Ph,F,H], [A110;H,H,OPh,F,H], [A111;H,H,OCH₂Ph,F,H], [A112;H,H,Me,Cl,H], [A113;H,H,Et,Cl,H], [A114;H,H,CF₃,Cl,H], [A115;H,H,c-Pr,Cl,H], [A116;H,H,F,Cl,H], [A117;H,H,Cl,Cl,H], [A118;H,H,Br,Cl,H], [A119;H,H,I,Cl,H], [A120;H,H,OMe,Cl,H], [A121;H,H,SMe,Cl,H], [A122;H,H,S(O)Me,Cl,H], [A123;H,H,S(O)₂Me,Cl,H), [A124;H,H,C(O)OMe,Cl,H], [A125;H,H,C(O)Me,Cl,H], [A126;H,H,C(O)NH₂,Cl,H], [A127;H,H,NMe₂,Cl,H], [A128;H,H,CN,Cl,H], [A129;H,H,NO₂,Cl,H], [A130;H,H,Ph,Cl,H], [A131;H,H,OPh,Cl,H], [A132;H,H,OCH₂Ph,Cl,H], [A133;H,H,Me,Br,H], [A134;H,H,Et,Br,H], [A135;H,H,CF₃,Br,H], [A136;H,H,c-Pr,Br,H], [A137;H,H,F,Br,H], [A138;H,H,Cl,Br,H], [A139;H,H,Br,Br,H], [A140;H,H,I,Br,H], [A141;H,H,OMe,Br,H], [A142;H,H,SMe,Br,H], [A143;H,H,S(O)Me,Br,H], [A144;H,H,S(O)₂Me,Br,H], [A145;H,H,C(O)OMe,Br,H], [A146;H,H,C(O)Me,Br,H], [A147;H,H,C(O)NH₂,Br,H], [A148;H,H,NMe₂,Br,H], [A149;H,H,CN,Br,H], [A150;H,H,NO₂,Br,H], [A151;H,H,Ph,Br,H], [A152;H,H,OPh,Br,H], [A153;H,H,OCH₂Ph,Br,H], [A154;H,H,Me,I,H], [A155;H,H,Et,I,H], [A156;H,H,CF₃,I,H], [A157;H,H,c-Pr,I,H], [A158;H,H,F,I,H], [A159;H,H,Cl,I,H], [A160;H,H,Br,I,H], [A161;H,H,I,I,H], [A162;H,H,OMe,I,H], [A163;H,H,SMe,I,H], [A164;H,H,S(O)Me,I,H], [A165;H,H,S(O)₂Me,I,H], [A166;H,H,C(O)OMe,I,H], [A167;H,H,C(O)Me,I,H], [A168;H,H,C(O)NH₂,I,H], [A169;H,H,NMe₂,I,H], [A170;H,H,CN,I,H], [A171;H,H,NO₂,I,H], [A172;H,H,Ph,I,H], [A173;H,H,OPh,I,H], [A174;H,H,OCH₂Ph,I,H], [A175;H,H,Me,OMe,H], [A176;H,H,Et,OMe,H], [A177;H,H,CF₃,OMe,H], [A178;H,H,c-Pr,OMe,H], [A179;H,H,F,OMe,H], [A180;H,H,Cl,OMe,H], [A181;H,H,Br,OMe,H], [A182;H,H,I,OMe,H], [A183;H,H,OMe,OMe,H], [A184;H,H,SMe,OMe,H], [A185;H,H,S(O)Me,OMe,H], [A186;H,H,S(O)₂Me,OMe,H], [A187;H,H,C(O)OMe,OMe,H], [A188;H,H,C(O)Me,OMe,H], [A189;H,H,C(O)NH₂,OMe,H], [A190;H,H,NMe₂,OMe,H], [A191;H,H,CN,OMe,H], [A192;H,H,NO₂,OMe,H], [A193;H,H,Ph,OMe,H], [A194;H,H,OPh,OMe,H], [A195;H,H,OCH₂Ph,OMe,H], [A196;H,H,Me,OPh,H], [A197;H,H,Et,OPh,H], [A198;H,H,CF₃,OPh,H], [A199;H,H,c-Pr,OPh,H], [A200;H,H,F,OPh,H], [A201;H,H,Cl,OPh,H], [A202;H,H,Br,OPh,H], [A203;H,H,I,OPh,H], [A204;H,H,OMe,OPh,H], [A205;H,H,SMe,OPh,H], [A206;H,H,S(O)Me,OPh,H], [A207;H,H,S(O)₂Me,OPh,H], [A208;H,H,C(O)OMe,OPh,H], [A209;H,H,C(O)Me,OPh,H], [A210;H,H,C(O)NH₂,OPh,H], [A211;H,H,NMe₂,OPh,H], [A212;H,H,CN,OPh,H], [A213;H,H,NO₂,OPh,H], [A214;H,H,Ph,OPh,H], [A215;H,H,OPh,OPh,H], [A216;H,H,OCH₂Ph,OPh,H], [A217;H,H,Me,Et,H], [A218;H,H,Et,Et,H], [A219;H,H,CF₃,Et,H], [A220;H,H,c-Pr,Et,H], [A221;H,H,F,Et,H], [A222;H,H,Cl,Et,H], [A223;H,H,Br,Et,H], [A224;H,H,I,Et,H], [A225;H,H,OMe,Et,H], [A226;H,H,SMe,Et,H], [A227;H,H,S(O)Me,Et,H], [A228;H,H,S(O)₂Me,Et,H], [A229;H,H,C(O)OMe,Et,H], [A230;H,H,C(O)Me,Et,H], [A231;H,H,C(O)NH₂,Et,H], [A232;H,H,NMe₂,Et,H], [A233;H,H,CN,Et,H], [A234;H,H,NO₂,Et,H], [A235;H,H,Ph,Et,H], [A236;H,H,OPh,Et,H], [A237;H,H,OCH₂Ph,Et,H], [A238;H,H,Me,Me,Me], [A239;H,H,Me,Et,Me], [A240;H,H,Me,CF₃,Me], [A241;H,H,Me,c-Pr, Me], [A242;H,H,Me,F,Me], [A243;H,H,Me,Cl,Me], [A244;H,H,Me,Br,Me], [A245;H,H,Me,I,Me], [A246;H,H,Me,OMe,Me], [A247;H,H,Me,SMe,Me], [A248;H,H,Me,S(O)Me,Me], [A249;H,H,Me,S(O)₂Me,Me], [A250;H,H,Me,C(O)OMe,Me], [A251;H,H,Me,C(O)Me,Me], [A252;H,H,Me, C(O)NH₂,Me], [A253;H,H,Me,NMe₂,Me], [A254;H,H,Me,CN,Me], [A255;H,H,Me,NO₂,Me], [A256;H,H,Me,Ph,Me], [A257;H,H,Me,OPh,Me], [A258;H,H,Me,OCH₂Ph,Me], [A259;H,H,F,Me,F], [A260;H,H,F,Et,F], [A261;H,H,F,CF₃,F], [A262;H,H,F,c-Pr,F] [A263;H,H,F,F,F], [A264;H,H,F,Cl,F], [A265;H,H,F,Br,F], [A266;H,H,F,I,F], [A267;H,H,F,OMe,F], [A268;H,H,F,SMe,F], [A269;H,H,F,S(O)Me,F], [A270;H,H,F,S(O)₂Me,F], [A271;H,H,F,C(O)OMe,F], [A272;H,H,F,C(O)Me,F], [A273;H,H,F,C(O)NH₂,F], [A274;H,H,F,NMe₂,F], [A275;H,H,F,CN,F], [A276;H,H,F,NO₂,F], [A277;H,H,F,Ph,F], [A278;H,H,F,OPh,F], [A279;H,H,F,OCH₂Ph,F], [A280;H,H,Cl,Me,Cl], [A281;H,H,Cl,Et,Cl], [A282;H,H,Cl,CF₃,Cl], [A283;H,H,Cl,c-Pr,Cl], [A284;H,H,Cl,F,Cl], [A285;H,H,Cl,Cl,Cl], [A286;H,H,Cl,Br,Cl], [A287;H,H,Cl,I,Cl], [A288;H,H,Cl,OMe,Cl], [A289;H,H,Cl,SMe,Cl], [A290;H,H,Cl,S(O)Me,Cl], [A291;H,H,Cl,S(O)₂Me,Cl], [A292;H,H,Cl,C(O)OMe,Cl], [A293;H,H,Cl,C(O)Me,Cl], [A294;H,H,Cl,C(O)NH₂,Cl], [A295;H,H,Cl,NMe₂,Cl], [A296;H,H,Cl,CN,Cl], [A297;H,H,Cl,NO₂,Cl], [A298;H,H,Cl,Ph,Cl], [A299;H,H,Cl,OPh,Cl], [A300;H,H,Cl,OCH₂Ph,Cl]

The Compound (A-1), wherein R^{X 1} represents a methyl group, R^{X 2}, R^{X 3}, R^{X 4}, and R^{X 5} each represent a hydrogen atom, and the combination of R¹, R², R^{3A}, R^{3B}, and R^{3 C} represents any one combination indicated in the Combination A (hereinafter referred to as "Compound group SX2").

The Compound (A-1), wherein R^{X 1} represents a fluorine atom, R^{X 2}, R^{X 3}, R^{X 4}, and R^{X 5} each represent a hydrogen atom, and the combination of R¹, R², R^{3A}, R^{3B}, and R^{3 C} represents any one combination indicated in the Combination A (hereinafter referred to as "Compound group SX3").

The Compound (A-1), wherein R^{X 1} represents a chlorine atom, R^{X 2}, R^{X 3}, R^{X 4}, and R^{X 5} each represent a hydrogen atom, and the combination of R¹, R², R^{3A}, R^{3B}, and R^{3 C} represents any one combination indicated in the Combination A (hereinafter referred to as "Compound group SX4").

The Compound (A-1), wherein R^{X 2} represents a methyl group, R^{X1}, R^{X 3}, R^{X 4}, and R^{X 5} each represent a hydrogen atom, and the combination of R¹, R², R^{3A}, R^{3B}, and R^{3 C} represents any one combination indicated in the Combination A (hereinafter referred to as "Compound group SX5").

The Compound (A-1), wherein R^{X 2} represents a fluorine atom, R^{X1}, R^{X 3}, R^{X 4}, and R^{X 5} each represent a hydrogen atom, and the combination of R¹, R², R^{3A}, R^{3B}, and R^{3 C} represents any one combination indicated in the Combination A (hereinafter referred to as "Compound group SX6").

The Compound (A-1), wherein R^{X 2} represents a chlorine atom, R^{X 1}, R^{X 3}, R^{X 4}, and R^{X 5} each represent a hydrogen atom, and the combination of R¹, R², R^{3A}, R^{3B}, and R^{3 C} represents any one combination indicated in the Combination A (hereinafter referred to as "Compound group SX7").

The Compound (A-1), wherein R^{X 3} represents a methyl group, R^{X 1}, R^{X 2}, R^{X 4}, and R^{X 5} each represent a hydrogen atom, and the combination of R¹, R², R^{3A}, R^{3B}, and R^{3 C} represents any one combination indicated in the Combination A (hereinafter referred to as "Compound group SX8").

The Compound (A-1), wherein R^{X 3} represents a fluorine atom, R^{X1}, R^{X 2}, R^{X 4}, and R^{X 5} each represent a hydrogen atom, and the combination of R¹, R², R^{3A}, R^{3B}, and R^{3 C} represents any one combination indicated in the Combination A (hereinafter referred to as "Compound group SX9").

The Compound (A-1), wherein R^{X 3} represents a chlorine atom, R^{X 1} , R^{X 2} , R^{X 4}, and R^{X 5} each represent a hydrogen atom, and the combination of R¹, R², R^{3A}, R^{3B}, and R^{3 C} represents any one combination indicated in the Combination A (hereinafter referred to as "Compound group SX10").

A compound represented by formula (A-2) 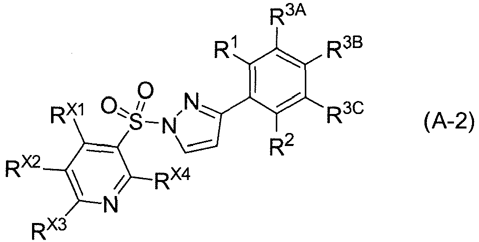 (hereinafter referred to as "Compound (A-2)"), wherein R^{X1}, R^{X2}, R^{X 3}, and R^{X4} each represent a hydrogen atom, and the combination of R¹, R², R^{3A}, R^{3B}, and R^{3 C} represents any one combination indicated in the Combination A (hereinafter referred to as "Compound group SX11").

The Compound (A-2), wherein R^{X 1} represents a methyl group, R^{X2}, R^{X 3}, and R^{X 4} each represent a hydrogen atom, and the combination of R¹, R², R^{3A}, R^{3B}, and R^{3 C} represents any one combination indicated in the Combination A (hereinafter referred to as "Compound group SX12").

The Compound (A-2), wherein R^{X 1} represents a fluorine atom, R^{X 2}, R^{X 3}, and R^{X 4} each represent a hydrogen atom, and the combination of R¹, R², R^{3A}, R^{3B}, and R^{3 C} represents any one combination indicated in the Combination A (hereinafter referred to as "Compound group SX13").

The Compound (A-2), wherein R^{X 1} represents a chlorine atom, R^{X 2}, R^{X 3}, and R^{X 4} each represent a hydrogen atom, and the combination of R¹, R², R^{3A}, R^{3B}, and R^{3 C} represents any one combination indicated in the Combination A (hereinafter referred to as "Compound group SX14").

The Compound (A-2), wherein R^{X 2} represents a methyl group, R^{X 1}, R^{X 3}, and R^{X 4} each represent a hydrogen atom, and the combination of R¹, R², R^{3A}, R^{3B}, and R^{3C} represents any one combination indicated in the Combination A (hereinafter referred to as "Compound group SX15").

The Compound (A-2), wherein R^{X 2} represents a fluorine atom, R^{X 1}, R^{X 3}, and R^{X 4} each represent a hydrogen atom, and the combination of R¹, R², R^{3A}, R^{3B}, and R^{3 C} represents any one combination indicated in the Combination A (hereinafter referred to as "Compound group SX16").

The Compound (A-2), wherein R^{X 2} represents a chlorine atom, R^{X 1}, R^{X 3}, and R^{X 4} each represent a hydrogen atom, and the combination of R¹, R², R^{3A}, R^{3B}, and R^{3 C} represents any one combination indicated in the Combination A (hereinafter referred to as "Compound group SX17").

The Compound (A-2), wherein R^{X 3} represents a methyl group, R^{X 1}, R^{X 2}, and R^{X 4} each represent a hydrogen atom, and the combination of R¹, R², R^{3A}, R^{3B}, and R^{3 C} represents any one combination indicated in the Combination A (hereinafter referred to as "Compound group SX18").

The Compound (A-2), wherein R^{X 3} represents a fluorine atom, R^{X 1}, R^{X 2}, and R^{X 4} each represent a hydrogen atom, and the combination of R¹, R², R^{3A}, R^{3B}, and R^{3 C} represents any one combination indicated in the Combination A (hereinafter referred to as "Compound group SX19").

The Compound (A-2), wherein R^{X 3} represents a chlorine atom, R^{X 1}, R^{X 2}, and R^{X 4} each represent a hydrogen atom, and the combination of R¹, R², R^{3A}, R^{3B}, and R^{3C} represents any one combination indicated in the Combination A (hereinafter referred to as "Compound group SX20").

The Compound (A-2), wherein R^{X 4} represents a methyl group, R^{X 1}, R^{X 2}, and R^{X 3} each represent a hydrogen atom, and the combination of R¹, R², R^{3A}, R^{3B}, and R^{3 C} represents any one combination indicated in the Combination A (hereinafter referred to as "Compound group SX21").

The Compound (A-2), wherein R^{X 4} represents a fluorine atom, R^{X 1}, R^{X 2}, and R^{X 3} each represent a hydrogen atom, and the combination of R¹, R², R^{3A}, R^{3B}, and R^{3 C} represents any one combination indicated in the Combination A (hereinafter referred to as "Compound group SX22").

The Compound (A-2), wherein R^{X 4} represents a chlorine atom, R^{X 1}, R^{X 2}, and R^{X 3} each represent a hydrogen atom, and the combination of R¹, R², R^{3A}, R^{3B}, and R^{3 C} represents any one combination indicated in the Combination A (hereinafter referred to as "Compound group SX23").

A compound represented by formula (A-3) 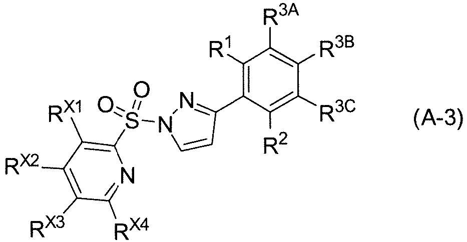 (hereinafter referred to as "Compound (A-3)"), wherein R^{X 1}, R^{X 2}, R^{X 3}, and R^{X 4} each represent a hydrogen atom, and the combination of R¹, R², R^{3A}, R^{3B}, and R^{3 C} represents any one combination indicated in the Combination A (hereinafter referred to as "Compound group SX24").

The Compound (A-3), wherein R^{X 1} represents a methyl group, R^{X 2}, R^{X 3}, and R^{X 4} each represent a hydrogen atom, and the combination of R¹, R², R^{3 A}, R^{3B}, and R^{3 C} represents any one combination indicated in the Combination A (hereinafter referred to as "Compound group SX25").

The Compound (A-3), wherein R^{X 1} represents a fluorine atom, R^{X 2}, R^{X 3}, and R^{X 4} each represent a hydrogen atom, and the combination of R¹, R², R^{3A}, R^{3B}, and R^{3 C} represents any one combination indicated in the Combination A (hereinafter referred to as "Compound group SX26").

The Compound (A-3), wherein R^{X 1} represents a chlorine atom, R^{X 2}, R^{X 3}, and R^{X 4} each represent a hydrogen atom, and the combination of R¹, R², R^{3A}, R^{3B}, and R^{3 C} represents any one combination indicated in the Combination A (hereinafter referred to as "Compound group SX27").

The Compound (A-3), wherein R^{X 2} represents a methyl group, R^{X1}, R^{X 3}, and R^{X 4} each represent a hydrogen atom, and the combination of R¹, R², R^{3A}, R^{3B}, and R^{3C} represents any one combination indicated in the Combination A (hereinafter referred to as "Compound group SX28").

The Compound (A-3), wherein R^{X 2} represents a fluorine atom, R^{X 1}, R^{X 3}, and R^{X 4} each represent a hydrogen atom, and the combination of R¹, R², R^{3A}, R^{3B}, and R^{3 C} represents any one combination indicated in the Combination A (hereinafter referred to as "Compound group SX29").

The Compound (A-3), wherein R^{X 2} represents a chlorine atom, R^{X 1}, R^{X 3}, and R^{X 4} each represent a hydrogen atom, and the combination of R¹, R², R^{3A}, R^{3B}, and R^{3 C} represents any one combination indicated in the Combination A (hereinafter referred to as "Compound group SX30").

The Compound (A-3), wherein R^{X 3} represents a methyl group, R^{X 1}, R^{X 2}, and R^{X 4} each represent a hydrogen atom, and the combination of R¹, R², R^{3A}, R^{3B}, and R^{3C} represents any one combination indicated in the Combination A (hereinafter referred to as "Compound group SX31").

The Compound (A-3), wherein R^{X3} represents a fluorine atom, R^{X1}, R^{X2}, and R^{X4} each represent a hydrogen atom, and the combination of R¹, R², R^{3A}, R^{3B}, and R^{3C} represents any one combination indicated in the Combination A (hereinafter referred to as "Compound group SX32").

The Compound (A-3), wherein R^{X3} represents a chlorine atom, R^{X1}, R^{X2}, and R^{X4} each represent a hydrogen atom, and the combination of R¹, R², R^{3A}, R^{3B}, and R^{3C} represents any one combination indicated in the Combination A (hereinafter referred to as "Compound group SX33").

The Compound (A-3), wherein R^{X4} represents a methyl group, R^{X1}, R^{X2}, and R^{X3} each represent a hydrogen atom, and the combination of R¹, R², R^{3A}, R^{3B}, and R^{3C} represents any one combination indicated in the Combination A (hereinafter referred to as "Compound group SX34").

The Compound (A-3), wherein R^{X4} represents a fluorine atom, R^{X1}, R^{X2}, and R^{X3} each represent a hydrogen atom, and the combination of R¹, R², R^{3A}, R^{3B}, and R^{3C} represents any one combination indicated in the Combination A (hereinafter referred to as "Compound group SX35").

The Compound (A-3), wherein R^{X4} represents a chlorine atom, R^{X1}, R^{X2}, and R^{X3} each represent a hydrogen atom, and the combination of R¹, R², R^{3A}, R^{3B}, and R^{3C} represents any one combination indicated in the Combination A (hereinafter referred to as "Compound group SX36").

A compound represented by formula (A-4) 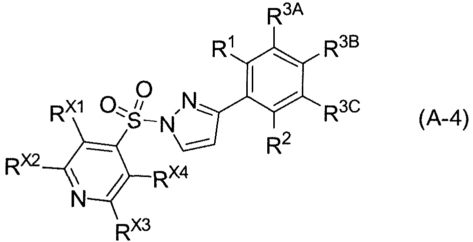 (hereinafter referred to as "Compound (A-4)"), wherein R^{X1}, R^{X2}, R^{X3}, and R^{X4} each represent a hydrogen atom, and the combination of R¹, R², R^{3A}, R^{3B}, and R^{3C} represents any one combination indicated in the Combination A (hereinafter referred to as "Compound group SX37").

The Compound (A-4), wherein R^{X1} represents a methyl group, R^{X2}, R^{X3}, and R^{X4} each represent a hydrogen atom, and the combination of R¹, R², R^{3A}, R^{3B}, and R^{3C} represents any one combination indicated in the Combination A (hereinafter referred to as "Compound group SX38").

The Compound (A-4), wherein R^{X1} represents a fluorine atom, R^{X2}, R^{X3}, and R^{X4} each represent a hydrogen atom, and the combination of R¹, R², R^{3A}, R^{3B}, and R^{3C} represents any one combination indicated in the Combination A (hereinafter referred to as "Compound group SX39").

The Compound (A-4), wherein R^{X1} represents a chlorine atom, R^{X2}, R^{X3}, and R^{X4} each represent a hydrogen atom, and the combination of R¹, R², R^{3A}, R^{3B}, and R^{3C} represents any one combination indicated in the Combination A (hereinafter referred to as "Compound group SX40").

The Compound (A-4), wherein R^{X2} represents a methyl group, R^{X1}, R^{X3}, and R^{X4} each represent a hydrogen atom, and the combination of R¹, R², R^{3A}, R^{3B}, and R^{3C} represents any one combination indicated in the Combination A (hereinafter referred to as "Compound group SX41").

The Compound (A-4), wherein R^{X2} represents a fluorine atom, R^{X1}, R^{X3}, and R^{X4} each represent a hydrogen atom, and the combination of R¹, R², R^{3A}, R^{3B}, and R^{3C} represents any one combination indicated in the Combination A (hereinafter referred to as "Compound group SX42").

The Compound (A-4), wherein R^{X2} represents a chlorine atom, R^{X1}, R^{X3}, and R^{X4} each represent a hydrogen atom, and the combination of R¹, R², R^{3A}, R^{3B}, and R^{3C} represents any one combination indicated in the Combination A (hereinafter referred to as "Compound group SX43").

The Compound (A-4), wherein R^{X3} represents a methyl group, R^{X1}, R^{X2}, and R^{X4} each represent a hydrogen atom, and the combination of R¹, R², R^{3A}, R^{3B}, and R^{3C} represents any one combination indicated in the Combination A (hereinafter referred to as "Compound group SX44").

The Compound (A-4), wherein R^{X3} represents a fluorine atom, R^{X1}, R^{X2}, and R^{X4} each represent a hydrogen atom, and the combination of R¹, R², R^{3A}, R^{3B}, and R^{3C} represents any one combination indicated in the Combination A (hereinafter referred to as "Compound group SX45").

The Compound (A-4), wherein R^{X3} represents a chlorine atom, R^{X1}, R^{X2}, and R^{X4} each represent a hydrogen atom, and the combination of R¹, R², R^{3A}, R^{3B}, and R^{3C} represents any one combination indicated in the Combination A (hereinafter referred to as "Compound group SX46").

The Compound (A-4), wherein R^{X4} represents a methyl group, R^{X1}, R^{X2}, and R^{X3} each represent a hydrogen atom, and the combination of R¹, R², R^{3A}, R^{3B}, and R^{3C} represents any one combination indicated in the Combination A (hereinafter referred to as "Compound group SX47").

The Compound (A-4), wherein R^{X4} represents a fluorine atom, R^{X1}, R^{X2}, and R^{X3} each represent a hydrogen atom, and the combination of R¹, R², R^{3A}, R^{3B}, and R^{3C} represents any one combination indicated in the Combination A (hereinafter referred to as "Compound group SX48").

The Compound (A-4), wherein R^{X4} represents a chlorine atom, R^{X1}, R^{X2}, and R^{X3} each represent a hydrogen atom, and the combination of R¹, R², R^{3A}, R^{3B}, and R^{3C} represents any one combination indicated in the Combination A (hereinafter referred to as "Compound group SX49").

A compound represented by formula (A-5) 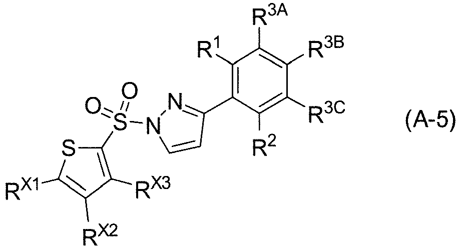 (hereinafter referred to as "Compound (A-5)"), wherein R^{X1}, R^{X2}, and R^{X3} each represent a hydrogen atom, and the combination of R¹, R², R^{3A}, R^{3B}, and R^{3C} represents any one combination indicated in the Combination A (hereinafter referred to as "Compound group SX50").

The Compound (A-5), wherein R^{X1} represents a methyl group, R^{X2} and R^{X3} each represent a hydrogen atom, and the combination of R¹, R², R^{3A}, R^{3B}, and R^{3C} represents any one combination indicated in the Combination A (hereinafter referred to as "Compound group SX51").

The Compound (A-5), wherein R^{X1} represents a fluorine atom, R^{X2} and R^{X3} each represent a hydrogen atom, and the combination of R¹, R², R^{3A}, R^{3B}, and R^{3C} represents any one combination indicated in the Combination A (hereinafter referred to as "Compound group SX52").

The Compound (A-5), wherein R^{X1} represents a chlorine atom, R^{X2} and R^{X3} each represent a hydrogen atom, and the combination of R¹, R², R^{3A}, R^{3B}, and R^{3C} represents any one combination indicated in the Combination A (hereinafter referred to as "Compound group SX53").

The Compound (A-5), wherein R^{X2} represents a methyl group, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX54").

The Compound (A-5), wherein R^{X2} represents a fluorine atom, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX55").

The Compound (A-5), wherein R^{X2} represents a chlorine atom, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX56").

The Compound (A-5), wherein R^{X3} represents a methyl group, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX57").

The Compound (A-5), wherein R^{X3} represents a fluorine atom, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX58").

The Compound (A-5), wherein R^{X3} represents a chlorine atom, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX59").

A compound represented by formula (A-6) 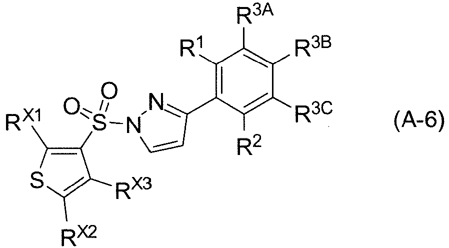 (hereinafter referred to as "Compound (A-6)"), wherein R^{X1}, R^{X2}, and R^{X3} each represent a hydrogen atom, and the combination of R¹, R², R^{3A}, R^{3B}, and R^{3C} represents any one combination indicated in the Combination A (hereinafter referred to as "Compound group SX60").

The Compound (A-6), wherein R^{X1} represents a methyl group, R^{X2} and R^{X3} each represent a hydrogen atom, and the combination of R¹, R², R^{3A}, R^{3B}, and R^{3C} represents any one combination indicated in the Combination A (hereinafter referred to as "Compound group SX61").

The Compound (A-6), wherein R^{X1} represents a fluorine atom, R^{X2} and R^{X3} each represent a hydrogen atom, and the combination of R¹, R², R^{3A}, R^{3B}, and R^{3C} represents any one combination indicated in the Combination A (hereinafter referred to as "Compound group SX62").

The Compound (A-6), wherein R^{X1} represents a chlorine atom, R^{X2} and R^{X3} each represent a hydrogen atom, and the combination of R¹, R², R^{3A}, R^{3B}, and R^{3C} represents any one combination indicated in the Combination A (hereinafter referred to as "Compound group SX63").

The Compound (A-6), wherein R^{X2} represents a methyl group, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX64").

The Compound (A-6), wherein R^{X2} represents a fluorine atom, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX65").

The Compound (A-6), wherein R^{X2} represents a chlorine atom, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX66").

The Compound (A-6), wherein R^{X3} represents a methyl group, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX67").

The Compound (A-6), wherein R^{X3} represents a fluorine atom, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX68").

The Compound (A-6), wherein R^{X3} represents a chlorine atom, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX69").

A compound represented by formula (A-7) 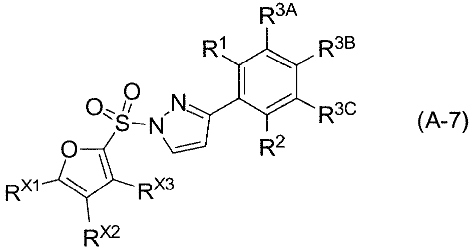 (hereinafter referred to as "Compound (A-7)"), wherein R^{X1}, R^{X2}, and R^{X3} each represent a hydrogen atom, and the combination of R¹, R², R^{3A}, R^{3B}, and R^{3C} represents any one combination indicated in the Combination A (hereinafter referred to as "Compound group SX70").

The Compound (A-7), wherein R^{X1} represents a methyl group, R^{X2} and R^{X3} each represent a hydrogen atom, and the combination of R¹, R², R^{3A}, R^{3B}, and R^{3C} represents any one combination indicated in the Combination A (hereinafter referred to as "Compound group SX71").

The Compound (A-7), wherein R^{X1} represents a fluorine atom, R^{X2} and R^{X3} each represent a hydrogen atom, and the combination of R¹, R², R^{3A}, R^{3B}, and R^{3C} represents any one combination indicated in the Combination A (hereinafter referred to as "Compound group SX72").

The Compound (A-7), wherein R^{X1} represents a chlorine atom, R^{X2} and R^{X3} each represent a hydrogen atom, and the combination of R¹, R², R^{3A}, R^{3B}, and R^{3C} represents any one combination indicated in the Combination A (hereinafter referred to as "Compound group SX73").

The Compound (A-7), wherein R^{X2} represents a methyl group, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX74").

The Compound (A-7), wherein R^{X2} represents a fluorine atom, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX75").

The Compound (A-7), wherein R^{X2} represents a chlorine atom, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX76").

The Compound (A-7), wherein R^{X3} represents a methyl group, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX77").

The Compound (A-7), wherein R^{X3} represents a fluorine atom, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX78").

The Compound (A-7), wherein R^{X3} represents a chlorine atom, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX79").

A compound represented by formula (A-8) 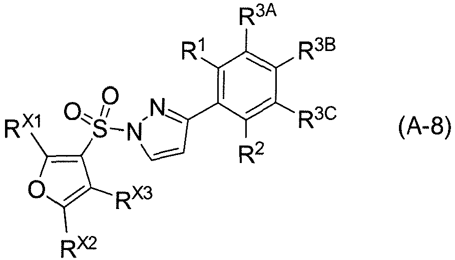 (hereinafter referred to as "Compound (A-8)"), wherein R^{X1}, R^{X2}, and R^{X3} each represent a hydrogen atom, and the combination of R¹, R², R^{3A}, R^{3B}, and R^{3C} represents any one combination indicated in the Combination A (hereinafter referred to as "Compound group SX80").

The Compound (A-8), wherein R^{X1} represents a methyl group, R^{X2} and R^{X3} each represent a hydrogen atom, and the combination of R¹, R², R^{3A}, R^{3B}, and R^{3C} represents any one combination indicated in the Combination A (hereinafter referred to as "Compound group SX81").

The Compound (A-8), wherein R^{X1} represents a fluorine atom, R^{X2} and R^{X3} each represent a hydrogen atom, and the combination of R¹, R², R^{3A}, R^{3B}, and R^{3C} represents any one combination indicated in the Combination A (hereinafter referred to as "Compound group SX82").

The Compound (A-8), wherein R^{X1} represents a chlorine atom, R^{X2} and R^{X3} each represent a hydrogen atom, and the combination of R¹ , R², R^{3A}, R^{3B}, and R^{3C} represents any one combination indicated in the Combination A (hereinafter referred to as "Compound group SX83").

The Compound (A-8), wherein R^{X2} represents a methyl group, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX84").

The Compound (A-8), wherein R^{X2} represents a fluorine atom, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX85").

The Compound (A-8), wherein R^{X2} represents a chlorine atom, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX86").

The Compound (A-8), wherein R^{X3} represents a methyl group, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX87").

The Compound (A-8), wherein R^{X3} represents a fluorine atom, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX88").

The Compound (A-8), wherein R^{X3} represents a chlorine atom, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX89").

A compound represented by formula (A-9) 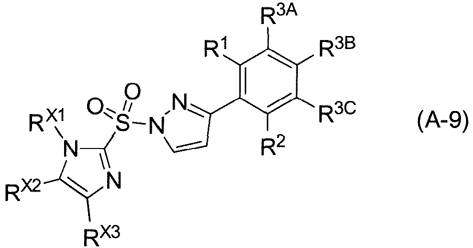 (hereinafter referred to as "Compound (A-9)"), wherein R^{X1}, R^{X2}, and R^{X3} each represent a hydrogen atom, and the combination of R¹, R², R^{3A}, R^{3B}, and R^{3C} represents any one combination indicated in the Combination A (hereinafter referred to as "Compound group SX90").

The Compound (A-9), wherein R^{X1} represents a methyl group, R^{X2} and R^{X3} each represent a hydrogen atom, and the combination of R¹, R², R^{3A}, R^{3B}, and R^{3C} represents any one combination indicated in the Combination A (hereinafter referred to as "Compound group SX91").

The Compound (A-9), wherein R^{X2} represents a methyl group, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX92").

The Compound (A-9), wherein R^{X2} represents a fluorine atom, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX93").

The Compound (A-9), wherein R^{X2} represents a chlorine atom, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX94").

The Compound (A-9), wherein R^{X3} represents a methyl group, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX95").

The Compound (A-9), wherein R^{X3} represents a fluorine atom, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX96").

The Compound (A-9), wherein R^{X3} represents a chlorine atom, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX97").

The Compound (A-9), wherein R^{X1} and R^{X2} each represent a methyl group, R^{X3} represents a hydrogen atom, and the combination of R¹, R², R^{3A}, R^{3B}, and R^{3C} represents any one combination indicated in the Combination A (hereinafter referred to as "Compound group SX98").

The Compound (A-9), wherein R^{X1} represents a methyl group, R^{X2} represents a fluorine atom, R^{X3} represents a hydrogen atom, and the combination of R¹, R², R^{3A}, R^{3B}, and R^{3C} represents any one combination indicated in the Combination A (hereinafter referred to as "Compound group SX99").

The Compound (A-9), wherein R^{X1} represents a methyl group, R^{X2} represents a chlorine atom, R^{X3} represents a hydrogen atom, and the combination of R¹, R², R^{3A}, R^{3B}, and R^{3C} represents any one combination indicated in the Combination A (hereinafter referred to as "Compound group SX100⁷) .

The Compound (A-9), wherein R^{X1} and R^{X3} each represent a methyl group, R^{X2} represents a hydrogen atom, and the combination of R¹, R², R^{3A}, R^{3B}, and R^{3C} represents any one combination indicated in the Combination A (hereinafter referred to as "Compound group SX101").

The Compound (A-9), wherein R^{X1} represents a methyl group, R^{X3} represents a fluorine atom, R^{X2} represents a hydrogen atom, and the combination of R¹, R², R^{3A}, R^{3B}, and R^{3C} represents any one combination indicated in the Combination A (hereinafter referred to as "Compound group SX102").

The Compound (A-9), wherein R^{X1} represents a methyl group, R^{X3} represents a chlorine atom, R^{X2} represents a hydrogen atom, and the combination ofR¹, R², R^{3A}, R^{3B}, and R^{3C} represents any one combination indicated in the Combination A (hereinafter referred to as "Compound group SX103").

A compound represented by formula (A-10) 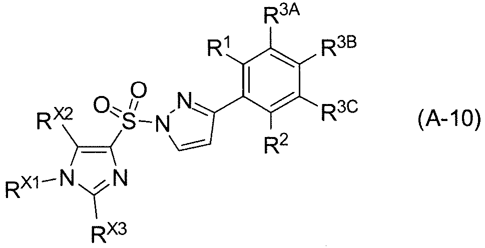 (hereinafter referred to as "Compound (A-10)"), wherein R^{X1}, R^{X2}, and R^{X3} each represent a hydrogen atom, and the combination of R¹, R², R^{3A}, R^{3B}, and R^{3C} represents any one combination indicated in the Combination A (hereinafter referred to as "Compound group SX104").

The Compound (A-10), wherein R^{X1} represents a methyl group, R^{X2} and R^{X3} each represent a hydrogen atom, and the combination of R¹, R², R^{3A}, R^{3B}, and R^{3C} represents any one combination indicated in the Combination A (hereinafter referred to as "Compound group SX105").

The Compound (A-10), wherein R^{X2} represents a methyl group, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX106").

The Compound (A-10), wherein R^{X2} represents a fluorine atom, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX107").

The Compound (A-10), wherein R^{X2} represents a chlorine atom, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX108").

The Compound (A-10), wherein R^{X3} represents a methyl group, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX109").

The Compound (A-10), wherein R^{X3} represents a fluorine atom, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX110").

The Compound (A-10), wherein R^{X3} represents a chlorine atom, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX111").

The Compound (A-10), wherein R^{X1} and R^{X2} each represent a methyl group, R^{X3} represents a hydrogen atom, and the combination of R¹, R², R^{3A}, R^{3B}, and R^{3C} represents any one combination indicated in the Combination A (hereinafter referred to as "Compound group SX112").

The Compound (A-10), wherein R^{X1} represents a methyl group, R^{X2} represents a fluorine atom, R^{X3} represents a hydrogen atom, and the combination of R¹, R², R^{3A}, R^{3B}, and R^{3C} represents any one combination indicated in the Combination A (hereinafter referred to as "Compound group SX113").

The Compound (A-10), wherein R^{X1} represents a methyl group, R^{X2} represents a chlorine atom, R^{X3} represents a hydrogen atom, and the combination of R¹, R², R^{3A}, R^{3B}, and R^{3C} represents any one combination indicated in the Combination A (hereinafter referred to as "Compound group SX114").

The Compound (A-10), wherein R^{X1} and R^{X3} each represent a methyl group, R^{X2} represents a hydrogen atom, and the combination of R¹, R², R^{3A}, R^{3B}, and R^{3C} represents any one combination indicated in the Combination A (hereinafter referred to as "Compound group SX115").

The Compound (A-10), wherein R^{X1} represents a methyl group, R^{X3} represents a fluorine atom, R^{X2} represents a hydrogen atom, and the combination of R¹, R², R^{3A}, R^{3B}, and R^{3C} represents any one combination indicated in the Combination A (hereinafter referred to as "Compound group SX116").

The Compound (A-10), wherein R^{X1} represents a methyl group, R^{X3} represents a chlorine atom, R^{X2} represents a hydrogen atom, and the combination of R¹, R², R^{3A}, R^{3B}, and R^{3C} represents any one combination indicated in the Combination A (hereinafter referred to as "Compound group SX117").

A compound represented by formula (A-11) 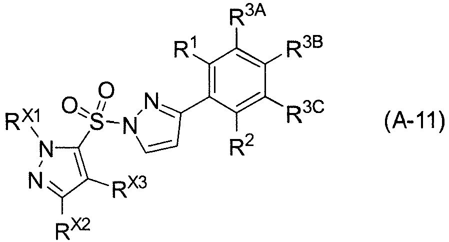 (hereinafter referred to as "Compound (A-11)"), wherein R^{X1}, R^{X2}, and R^{X3} each represent a hydrogen atom, and the combination of R¹, R², R^{3A}, R^{3B}, and R^{3C} represents any one combination indicated in the Combination A (hereinafter referred to as "Compound group SX118").

The Compound (A-11), wherein R^{X1} represents a methyl group, R^{X2} and R^{X3} each represent a hydrogen atom, and the combination of R¹, R², R^{3A}, R^{3B}, and R^{3C} represents any one combination indicated in the Combination A (hereinafter referred to as "Compound group SX119").

The Compound (A-11), wherein R^{X2} represents a methyl group, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX120").

The Compound (A-11), wherein R^{X2} represents a fluorine atom, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX121").

The Compound (A-11), wherein R^{X2} represents a chlorine atom, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX122").

The Compound (A-11), wherein R^{X3} represents a methyl group, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX123").

The Compound (A-11), wherein R^{X3} represents a fluorine atom, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX124").

The Compound (A-11), wherein R^{X3} represents a chlorine atom, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX125").

The Compound (A-11), wherein R^{X1} and R^{X2} each represent a methyl group, R^{X3} represents a hydrogen atom, and the combination of R¹, R², R^{3A}, R^{3B}, and R^{3C} represents any one combination indicated in the Combination A (hereinafter referred to as "Compound group SX126").

The Compound (A-11), wherein R^{X1} represents a methyl group, R^{X2} represents a fluorine atom, R^{X3} represents a hydrogen atom, and the combination of R¹, R², R^{3A}, R^{3B}, and R^{3C} represents any one combination indicated in the Combination A (hereinafter referred to as "Compound group SX127") .

The Compound (A-11), wherein R^{X1} represents a methyl group, R^{X2} represents a chlorine atom, R^{X3} represents a hydrogen atom, and the combination of R¹, R², R^{3A}, R^{3B}, and R^{3C} represents any one combination indicated in the Combination A (hereinafter referred to as "Compound group SX128") .

The Compound (A-11), wherein R^{X1} and R^{X3} each represent a methyl group, R^{X2} represents a hydrogen atom, and the combination of R¹, R², R^{3A}, R^{3B}, and R^{3C} represents any one combination indicated in the Combination A (hereinafter referred to as "Compound group SX129").

The Compound (A-11), wherein R^{X1} represents a methyl group, R^{X3} represents a fluorine atom, R^{X2} represents a hydrogen atom, and the combination of R¹, R², R^{3A}, R^{3B}, and R^{3C} represents any one combination indicated in the Combination A (hereinafter referred to as "Compound group SX130") .

The Compound (A-11), wherein R^{X1} represents a methyl group, R^{X3} represents a chlorine atom, R^{X2} represents a hydrogen atom, and the combination of R¹, R², R^{3A}, R^{3B}, and R^{3C} represents any one combination indicated in the Combination A (hereinafter referred to as "Compound group SX131") .

A compound represented by formula (A-12) 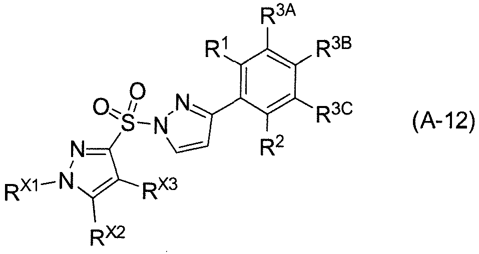 (hereinafter referred to as "Compound (A-12)"), wherein R^{X1}, R^{X2}, and R^{X3} each represent a hydrogen atom, and the combination of R¹, R², R^{3A}, R^{3B}, and R^{3C} represents any one combination indicated in the Combination A (hereinafter referred to as "Compound group SX132").

The Compound (A-12), wherein R^{X1} represents a methyl group, R^{X2} and R^{X3} each represent a hydrogen atom, and the combination of R¹, R², R^{3A}, R^{3B}, and R^{3C} represents any one combination indicated in the Combination A (hereinafter referred to as "Compound group SX133").

The Compound (A-12), wherein R^{X2} represents a methyl group, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX134").

The Compound (A-12), wherein R^{X2} represents a fluorine atom, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX135").

The Compound (A-12), wherein R^{X2} represents a chlorine atom, R^{X1} and R^{X3} each represent a hydrogen atom, and the combination of R¹, R², R^{3A}, R^{3B}, and R^{3C} represents any one combination indicated in the Combination A (hereinafter referred to as "Compound group SX136").

The Compound (A-12), wherein R^{X3} represents a methyl group, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX137").

The Compound (A-12), wherein R^{X3} represents a fluorine atom, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX138").

The Compound (A-12), wherein R^{X3} represents a chlorine atom, R^{X1} and R^{X2} each represent a hydrogen atom, and the combination of R¹, R², R^{3A}, R^{3B}, and R^{3C} represents any one combination indicated in the Combination A (hereinafter referred to as "Compound group SX139").

The Compound (A-12), wherein R^{X1} and R^{X2} each represent a methyl group, R^{X3} represents a hydrogen atom, and the combination of R¹, R², R^{3A}, R^{3B}, and R^{3C} represents any one combination indicated in the Combination A (hereinafter referred to as "Compound group SX140").

The Compound (A-12), wherein R^{X1} represents a methyl group, R^{X2} represents a fluorine atom, R^{X3} represents a hydrogen atom, and the combination of R¹, R², R^{3A}, R^{3B}, and R^{3C} represents any one combination indicated in the Combination A (hereinafter referred to as "Compound group SX141").

The Compound (A-12), wherein R^{X1} represents a methyl group, R^{X2} represents a chlorine atom, R^{X3} represents a hydrogen atom, and the combination of R¹, R², R^{3A}, R^{3B}, and R^{3C} represents any one combination indicated in the Combination A (hereinafter referred to as "Compound group SX142").

The Compound (A-12), wherein R^{X1} and R^{X3} each represent a methyl group, R^{X2} represents a hydrogen atom, and the combination of R¹, R², R^{3A}, R^{3B}, and R^{3C} represents any one combination indicated in the Combination A (hereinafter referred to as "Compound group SX143").

The Compound (A-12), wherein R^{X1} represents a methyl group, R^{X3} represents a fluorine atom, R^{X2} represents a hydrogen atom, and the combination of R¹, R², R^{3A}, R^{3B}, and R^{3C} represents any one combination indicated in the Combination A (hereinafter referred to as "Compound group SX144") .

The Compound (A-12), wherein R^{X1} represents a methyl group, R^{X3} represents a chlorine atom, R^{X2} represents a hydrogen atom, and the combination of R¹, R², R^{3A}, R^{3B}, and R^{3C} represents any one combination indicated in the Combination A (hereinafter referred to as "Compound group SX145").

Next, Formulation Examples are shown below. The "part(s)" represents "part(s) by weight". Also, the expression of "Present compound S" represents the compounds described in the Compound groups SX1 to SX145.

### Formulation Example 1

A mixture of polyoxyethylene alkyl ether sulfate ammonium salt and silica (weight ratio of 1 : 1) (35 parts), any one of the Present compound S (10 parts), and water (55 parts) are mixed, and the resulting mixture is subjected to fine grinding according to a wet grinding method to obtain each formulation.

### Formulation Example 2

Any one of the Present compound S (50 parts), calcium lignin sulfonate (3 parts), sodium lauryl sulfate (2 parts), and silica (45 parts) are ground and mixed to obtain each formulation.

### Formulation Example 3

Any one of the Present compound S (5 parts), polyoxyethylene styryl phenyl ether (9 parts), polyoxyethylene decyl ether (number of added ethyleneoxide: 5) (5 parts), calcium dodecylbenzene sulfonate (6 parts), and xylene (75 parts) are mixed to obtain each formulation.

### Formulation Example 4

Any one of the Present compound S (2 parts), silica (1 part), calcium lignin sulfonate (2 parts), bentonite (30 parts), and kaolin clay (65 parts) are ground and mixed, an appropriate amount of water is added thereto, the resulting mixture is kneaded, subjected to granulation with a granulator, and then dried to obtain each formulation.

### Formulation Example 5

Any one of the Present compound S (10 parts), and a mixture of benzyl alcohol (18 parts) and DMSO (9 parts) are mixed, GERONOL (registered trademark) TE250 (6.3 parts), Ethylan (registered trademark) NS-500LQ (2.7 parts), and solvent naphtha (54 parts) are added thereto, and the resulting mixture is mixed to obtain each formulation.

Next, Test Examples are shown below.

The term of "non-treated well" in Test Example 4, Test Example 5, and Test Example 6 represents a well wherein a test was carried out under the same conditions as those described in each Test Example except for dispensing DMSO instead of a diluted solution with DMSO comprising the Present compound. Also, the term of "non-treated" in Test Examples 15 to 17 represents a test which was carried out under the same conditions as those described in each Test Example except for dispensing DMSO instead of a diluted solution with DMSO comprising the Present compound. Also, the term of "non-treated leaf disk" in Test Example 7 represents a leaf disk wherein a test was carried out under the same conditions as those described in the Test Example except for dispensing DMSO instead of a diluted solution with DMSO comprising the Present compound. Also, the term of "non-treated" in Test Example 10 and Test Example 11 means that a diluted solution with water of a formulation comprising the Present compound was not sprayed.

### Test Example 1 Control test against wheat leaf blotch fungus (Septoria tritici)

The Present compound is diluted with DMSO to obtain a diluted solution containing 150 ppm of the compound, and 1 µL of the diluted solution is dispensed into a titer plate (96 well), and thereto is then dispensed 150 µL of a YBG medium to which spores of Septoria tritici are inoculated in advance. This plate is cultured at 18°C for 3 days, thereby allowing Septoria tritici to undergo proliferation, and the absorbance at 550 nm of each well of the titer plate is then measured to determine a degree of growth of Septoria tritici. As a result, each Present compound shows a control effect against the plant disease.

### Test Example 2 Control test against corn smut fungus (Ustilago maydis)

The Present compound is diluted with DMSO to obtain a diluted solution containing 150 ppm of the compound, and 1 µL of the diluted solution is dispensed into a titer plate (96 well), and thereto is then dispensed 150 µL of a potato dextrose broth medium (PDB medium) to which spores of Ustilago maydis are inoculated in advance. This plate is cultured at 18°C for 4 days, thereby allowing Ustilago maydis to undergo proliferation, and the absorbance at 550 nm of each well of the titer plate is then measured to determine a degree of growth of Ustilago maydis. As a result, each Present compound shows a control effect against the plant disease.

### Test Example 3 Control test against barley scald fungus (Rhynchosporium secalis)

The Present compound is diluted with DMSO to obtain a diluted solution containing 150 ppm of the compound, and 1 µL of the diluted solution is dispensed into a titer plate (96 well), and thereto is then dispensed 150 µL of a potato dextrose broth medium (PDB medium) to which spores of Rhynchosporium secalis are inoculated in advance. This plate is cultured at 18°C for 7 days, thereby allowing Rhynchosporium secalis to undergo proliferation, and the absorbance at 550 nm of each well of the titer plate is . then measured to determine a degree of growth of Rhynchosporium secalis. As a result, each Present compound shows a control effect against the plant disease.

### Test Example 4 Control test against cucumber Botrytis rot fungus (Botrytis cinerea)

The Present compound 24 was diluted with DMSO to obtain a diluted solution containing 150 ppm of the compound, and 1 µL of the diluted solution was dispensed into a titer plate (96 well), and thereto was then dispensed 150 µL of a complete medium to which spores of Botrytis cinerea were inoculated in advance. This plate was cultured at 18°C for 4 days, thereby allowing Botrytis cinerea to undergo proliferation, and the absorbance at 550 nm of each well of the titer plate was then measured to determine a degree of growth of Botrytis cinerea. As a result, the degree of growth in the well treated with the above Present compound was 50% or less relative to the degree of growth in the non-treated well.

### Test Example 5 Control test against peach scab fungus (Cladosporium carpophilum)

Each of the Present compound 79, 80, 81, 89, 92, 93, 96, or 99 was diluted with DMSO to obtain a diluted solution containing 150 ppm of each compound, and 1 µL of the diluted solution was dispensed into a titer plate (96 well), and thereto was then dispensed 150 µL of a potato dextrose broth medium (PDB medium) to which spores of Cladosporium carpophilum were inoculated in advance. This plate was cultured at 18°C for 5 days, thereby allowing Cladosporium carpophilum to undergo proliferation, and the absorbance at 550 nm of each well of the titer plate was then measured to determine a degree of growth of Cladosporium carpophilum. As a result, the degree of growth in the well treated with each of the above Present compound was 50% or less relative to the degree of growth in the non-treated well.

### Test Example 6 Control test against rice brown spot fungus (Cochliobolus miyabeanus)

Each of the Present compound 17, 33, 36, 37, 39, 40, 41, 42, 43, 44, 48, 49, 50, 51, 52, 53, 62, 68, 69, 70, 71, 76, 77, 79, 80, 81, 82, 83, 84, or 97 was diluted with DMSO to obtain a diluted solution containing 150 ppm of each compound, and 1 µL of the diluted solution was dispensed into a titer plate (96 well), and thereto was then dispensed 150 µL of a YB liquid medium to which spores of Cochliobolus miyabeanus were inoculated in advance. This plate was cultured at 23°C for 3 days, thereby allowing Cochliobolus miyabeanus to undergo proliferation, and the absorbance at 550 nm of each well of the titer plate was then measured to determine a degree of growth of Cochliobolus miyabeanus. As a result, the degree of growth in the well treated with each of the above Present compounds was 50% or less relative to the degree of growth in the non-treated well.

### Test Example 7 Control test against soybean rust (Phakopsora pachyrhizi)

A true leaf of a soybean (cultivar: Kurosengoku) was cut into a diameter of 1 cm to prepare each leaf disk. To each well of a 24 well microplate was dispensed 1 mL of an agar medium (agar concentration 1.2%), and then one of said leaf disk was placed on the agar medium in each well. To a mixture of Sorpol (registered trademark) 1200KX (0.5 µL), DMSO (4.5 µL), and xylene (5 µL) was added a DMSO solution (20 µL) comprising a test compound (10000 ppm), and the resulting mixture was mixed. The resulting mixture was diluted with ion exchange water to prepare a mixture comprising a prescribed concentration of the test compound. The resulting mixture was sprayed into each leaf disk at a ratio of 10 µL per leaf disk. After 1 day, an aqueous suspension of spores of soybean rust fungus (Phakopsora pachyrhizi) having an amino acid substitution of F129L in a mitochondrial cytochrome b protein (1.0 × 10⁵/mL) was inoculated by spraying on each leaf disk. After the inoculation, the microplate was placed into an artificial climate chamber (Lighting: 6 hours, Lights-out: 18 hours, Temperature: 23°C, Humidity: 60%). After 1 day, the leaf disks were air-dried until water droplets on the surfaces of the leaf disks disappeared, and the microplate was placed into the artificial climate chamber again for 12 days. Then, lesion area of soybean rust was investigated. As a result, when the prescribed concentration was 200 ppm, each lesion area of leaf disk treated with any one of the Present compound 9, 10, 11, 13, 14, 15, 16, 17, 18, 20, 32, 33, 34, 35, 36, 37, 38, 39, 40, 42, 43, 44, 47, 48, 49, 50, 51, 52, 53, 55, 56, 57, 58, 62, 63, 64, 65, 66, 67, 68, 69, 70, 71, 73, 74, 75, 76, 77, 78, 79, 80, 81, 82, 83, 84, 85, 86, 87, 88, 89, 90, 91, 92, 93, 94, 95, 96, 97, 98, 99, 100, 101, or 102 as the test compound was 30% or less relative to the lesion area of non-treated leaf disk.

### Test Example 8 Control test against barley net blotch (Pyrenophora teres)

A plastic pot is filled with soil, barley (cultivar: Nishinohoshi) is seeded thereto, and grown in a greenhouse for 7 days. The Present compound formulated according to the method described in the Formulation Example 1 is mixed with water in such a way that the concentration thereof is 200 ppm, and the resulting mixture is sprayed to foliage of the above barley so as to adequately adhere onto the surfaces of leaves of the above barley. After spraying the mixture, the barley is air-dried. After 1 day, an aqueous suspension of spores of Pyrenophora teres is inoculated by spraying to the barley. After the inoculation, the barley is placed in a greenhouse at 23°C in daytime and at 20°C in nighttime under a humid condition for 3 days, then cultured in a greenhouse for 7 days, and then a lesion area is investigated. As a result, each Present compound shows a control effect against the plant disease.

### Test Example 9 Control test against wheat leaf rust (Puccinia recondita)

A plastic pot is filled with soil, wheat (cultivar: Shirogane) is seeded thereto, and grown in a greenhouse for 9 days. The Present compound formulated according to the method described in the Formulation Example 1 is mixed with water in such a way that the concentration thereof is 200 ppm, and the resulting mixture is sprayed to foliage of the above wheat so as to adequately adhere onto the surfaces of leaves of the above wheat. After spraying the mixture, the wheat is air-dried, cultured at 20°C under lighting for 5 to 7 days, and then spores of Puccinia recondita are inoculated by dusting thereto. After the inoculation, the wheat is placed at 23°C under a dark humid condition for 1 day, then cultured at 20°C under lighting for 8 days, and a lesion area is investigated. As a result, each Present compound shows a control effect against the plant disease.

### Test Example 10 Control test against wheat leaf blotch (Septoria tritici)

A plastic pot was filled with soil, wheat (cultivar: Apogee) was seeded thereto, and cultured in a greenhouse for 10 days. The Present compound 4 or 31 formulated according to the method described in the Formulation Example 1 was mixed with water in such a way that the concentration thereof was 200 ppm, and the resulting mixture was sprayed to foliage of the above wheat so as to adequately adhere onto the surfaces of leaves of the above wheat. After spraying the mixture, the wheat was air-dried. After 4 days, an aqueous suspension comprising spores of Septoria tritici was inoculated by spraying to the wheat. After the inoculation, the wheat was placed at 18°C under a humid condition for 3 days, and then cultured under lighting for 14 to 18 days, and then a lesion area was investigated. As a result, the lesion area in the wheat treated with each of the above Present compound was 30% or less relative to the lesion area in the non-treated wheat.

### Test Example 11 Control test against soybean rust (Phakopsora pachyrhizi)

A plastic pot was filled with soil, soybeans (cultivar: Kurosengoku) were seeded thereto, and cultured in a greenhouse for 10 to 14 days. The Present compound 9, 10, 11, 13, 14, 15, 17, 18, 20, 21, 22, 23, 26, 27, 33, 34, 35, 36, 37, 38, 39, 40, 41, 42, 43, 44, 46, 47, 53, 54, 55, 56, 57, 58, 66, 67, 68, 69, 70, 71, 73, 74, 75, 76, 77, 78, 79, 80, 81, 82, 83, 84, 85, 86, 87, 88, 89, 90, 91, 92, 93, 94, 95, 96, 97, 98, 99, 100, 101, or 102 formulated according to the method described in the Formulation Example 1 was mixed with water in such a way that the concentration thereof was 200 ppm, and the resulting mixture was sprayed to foliage of the above soybeans so as to adequately adhere onto the surfaces of leaves of the above soybeans. After spraying the mixture, the soybeans were air-dried. After 2 to 5 days, an aqueous suspension of spores of Phakopsora pachyrhizi was inoculated by spraying to the soybeans. After the inoculation, the soybeans were placed in a greenhouse at 23°C in daytime and at 20°C in nighttime under a humid condition for 1 to 2 day(s), then cultured in a greenhouse for 12 days, and then a lesion area was investigated. As a result, the lesion area in the soybeans treated with each of the above Present compound was 30% or less relative to the lesion area in the non-treated soybeans.

### Test Example 12 Control test against soybean rust (Phakopsora pachyrhizi)

A plastic pot is filled with soil, soybeans (cultivar: Kurosengoku) are seeded thereto, and cultured in a greenhouse for 10 days. An aqueous suspension comprising spores of Phakopsora pachyrhizi is inoculated by spraying to the soybeans. After the inoculation, the soybeans are placed in a greenhouse at 23°C in daytime and at 20°C in nighttime under a humid condition for 1 day, and then cultured in a greenhouse for 2 days. Then, the Present compound formulated according to the method described in the Formulation Example 1 is mixed with water in such a way that the concentration thereof is 200 ppm, and the resulting mixture is sprayed to foliage of the above soybeans so as to adequately adhere onto the surfaces of leaves of the above soybeans. After spraying the mixture, the soybeans are air-dried, then cultured in a greenhouse for 8 days, and then a lesion area is investigated. As a result, each Present compound shows a control effect against the plant disease.

### Test Example 13 Control test against soybean Cercospora leaf spot (Cercospora sojina)

A plastic pot is filled with soil, soybeans (cultivar: Tachinagaha) are seeded thereto, and cultured in a greenhouse for 13 days. The Present compound formulated according to the method described in the Formulation Example 1 is mixed with water in such a way that the concentration thereof is 200 ppm, and the resulting mixture is sprayed to foliage of the above soybeans so as to adequately adhere onto the surfaces of leaves of the above soybeans. After spraying the mixture, the soybeans are air-dried. After 1 day, an aqueous suspension of spores of Cercospora sojina is inoculated by spraying to the soybeans. After the inoculation, the soybeans are placed in a greenhouse at 23°C in daytime and at 20°C in nighttime under a humid condition for 3 days, then cultured in a greenhouse for 16 days, and then a lesion area is investigated. As a result, each Present compound shows a control effect against the plant disease.

### Test Example 14 Control test against tomato early blight (Alternaria solani)

A plastic pot is filled with soil, tomatoes (cultivar: Patio) are seeded thereto, and cultured in a greenhouse for 20 days. The Present compound formulated according to the method described in the Formulation Example 1 is mixed with water in such a way that the concentration thereof is 200 ppm, and the resulting mixture is sprayed to foliage of the above tomatoes so as to adequately adhere onto the surfaces of leaves of the above tomatoes. After spraying the mixture, the tomatoes are air-dried. After 1 day, an aqueous suspension of spores of Alternaria solani is inoculated by spraying to the tomatoes. After the inoculation, the tomatoes are placed at 18°C under a humid condition for 6 days, and then a lesion area is investigated. As a result, each Present compound shows a control effect against the plant disease.

### Test Example 15 Control test against soybean rust fungus (Phakopsora pachyrhizi)

The Present compound was diluted with DMSO to obtain a diluted solution containing 150 ppm of the compound, and 1 µL of the diluted solution was dispensed into a titer plate (96 well), and thereto was then dispensed 149 µL of an aqueous suspension (1.0 × 10⁴ / mL) of spores of Phakopsora pachyrhizi to which spores of Phakopsora pachyrhizi were suspended in advance. This plate was cultured at 23°C for several hours, and then germinated spores of Phakopsora pachyrhizi were counted. As a result, when the treatment concentration was 1 ppm, the number of germinated spores in each well comprising any one of the Present compound 1, 11, 17, 18, 20, 23, 30, 33, 34, 35, 36, 37, 38, 39, 40, 41, 42, 43, 44, 46, 47, 48, 49, 50, 51, 52, 53, 54, 55, 56, 57, 58, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, or 102 as the test compound was 50% or less relative to the number of germinated spores in the non-treated well.

### Test Example 16 Control test against soybean rust fungus (Phakopsora pachyrhizi)

An agar medium (agar concentration: 1.0%) (3 mL) was placed in a plastic petri dish having a diameter of 3.5 cm, and solidified. Each Present compound was diluted with DMSO in such a way that the resulting diluted solution containing 10000 ppm of the compound, and said solution (1 µL) and an aqueous suspension (1.0 × 10⁴ / mL) (999 µL) of spores of Phakopsora pachyrhizi to which spores of Phakopsora pachyrhizi were suspended were mixed, and then the resulting mixture was dispensed into the plastic petri dish. This plastic petri dish was cultured at 23°C for 1 day, and then germinated spores of Phakopsora pachyrhizi were counted. As a result, when the treatment concentration was 10 ppm, and any one of the Present compound 9, 10, 28, 29, or 32 was used as the test compound to carry out the test, the number of germinated spores in each plastic petri dish using the above each Present compound as the test compound was 50% or less relative to the number of germinated spores in the non-treated plastic petri dish.

### Test Example 17 Control test against soybean rust fungus (Phakopsora pachyrhizi)

A test was carried out by the method according to the Test Example 16 and using the Present compound 61 as the test compound with the treatment concentration of 30 ppm. As a result, the number of germinated spores in the plastic petri dish using the above Present compound as the test compound was 50% or less relative to the number of germinated spores in the non-treated plastic petri dish.

### INDUSTRIAL APPLICABILITY

The Present compounds have control effects against plant diseases, and can be used in controlling plant diseases.

## Claims

1. A method for controlling a plant disease which comprises applying a compound represented by formula (I) 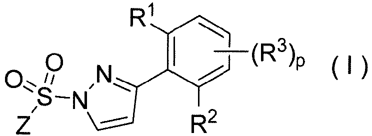 [wherein:
Z represents a C6-C10 aryl group or a 5-10 membered aromatic heterocyclic group {wherein said C6-C10 aryl group and said 5-10 membered aromatic heterocyclic group may be optionally substituted with one or more substituent(s) selected from Group D};
R¹ and R² are identical to or different from each other, and each represent a C1-C6 chain hydrocarbon group optionally substituted with one or more substituent(s) selected from Group A, a C3-C8 alicyclic hydrocarbon group, a 3-8 membered nonaromatic heterocyclic group, a C6-C10 aryl group, a 5-10 membered aromatic heterocyclic group {wherein said C3-C8 alicyclic hydrocarbon group, said 3-8 membered nonaromatic heterocyclic group, said C6-C10 aryl group, and said 5-10 membered aromatic heterocyclic group may be optionally substituted with one or more substituent(s) selected from Group C}, a hydrogen atom, a halogen atom, a nitro group, a cyano group, NR⁸R⁹, OR¹⁰, S (O)ₖR¹¹, C(O)R¹², C (O)OR¹³, C(O)NR⁴R⁵, or CR⁶=N-O-R⁷;
R³ represents a C1-C6 chain hydrocarbon group optionally substituted with one or more substituent(s) selected from Group A, a C3-C8 alicyclic hydrocarbon group, a 3-8 membered nonaromatic heterocyclic group, a C6-C10 aryl group, a 5-10 membered aromatic heterocyclic group {wherein said C3-C8 alicyclic hydrocarbon group, said 3-8 membered nonaromatic heterocyclic group, said C6-C10 aryl group, and said 5-10 membered aromatic heterocyclic group may be optionally substituted with one or more substituent(s) selected from Group C}, a halogen atom, a nitro group, a cyano group, NR⁸R⁹, OR¹⁰, S(O)ₖR¹¹, C(O)R¹², C(O)OR¹³, C(O)NR⁴R⁵, or CR⁶=N-O-R⁷;
adjacent R¹ and R³ may be optionally combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B}, a benzene ring, or a 5-7 membered aromatic heterocyclic ring {wherein said benzene ring and said 5-7 membered aromatic heterocyclic ring may be optionally substituted with one or more substituent(s) selected from Group E};
adjacent two R³ and R³ may be optionally combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B}, a benzene ring, or a 5-7 membered aromatic heterocyclic ring {wherein said benzene ring and said 5-7 membered aromatic heterocyclic ring may be optionally substituted with one or more substituent(s) selected from Group E};
R⁴, R⁵, R⁶, R⁷, R⁸, R⁹, R¹⁰, R¹¹, R¹², and R¹³ are identical to or different from each other, and each represent a C1-C6 chain hydrocarbon group optionally substituted with one or more substituent(s) selected from Group A, a C3-C8 alicyclic hydrocarbon group, a 3-8 membered nonaromatic heterocyclic group, a C6-C10 aryl group, a 5-10 membered aromatic heterocyclic group {wherein said C3-C8 alicyclic hydrocarbon group, said 3-8 membered nonaromatic heterocyclic group, said C6-C10 aryl group, and said 5-10 membered aromatic heterocyclic group may be optionally substituted with one or more substituent(s) selected from Group C}, or a hydrogen atom;
p represents 0, 1, 2, or 3;
when p represents 2 or 3, then two or three R³ are identical to or different from each other; and
k represents 0, 1, or 2;
Group A: a group consisting of a C1-C6 alkoxy group, a C1-C6 alkylsulfanyl group, a C1-C6 alkylsulfinyl group, a C1-C6 alkylsulfonyl group {wherein said C1-C6 alkoxy group, said C1-C6 alkylsulfanyl group, said C1-C6 alkylsulfinyl group, and said C1-C6 alkylsulfonyl group may be optionally substituted with one or more halogen atom(s)}, a C3-C8 alicyclic hydrocarbon group, a 3-8 membered nonaromatic heterocyclic group, a C6-C10 aryl group, a 5-10 membered aromatic heterocyclic group {wherein said C3-C8 alicyclic hydrocarbon group, said C6-C10 aryl group, and said 5-10 membered aromatic heterocyclic group may be optionally substituted with one or more substituent(s) selected from Group C}, a halogen atom, and a cyano group;
Group B: a group consisting of an oxo group, a thioxo group, a C1-C6 chain hydrocarbon group, a C3-C8 alicyclic hydrocarbon group, a C1-C6 alkoxy group {wherein said C1-C6 chain hydrocarbon group, said C3-C8 alicyclic hydrocarbon group, and said C1-C6 alkoxy group may be optionally substituted with one or more halogen atom(s)}, a halogen atom, a nitro group, and a cyano group;
Group C: a group consisting of a C1-C6 chain hydrocarbon group, a C3-C8 alicyclic hydrocarbon group, a C1-C6 alkoxy group, a C1-C6 alkylsulfanyl group, a C1-C6 alkylsulfinyl group, a C1-C6 alkylsulfonyl group {wherein said C1-C6 chain hydrocarbon group, said C3-C8 alicyclic hydrocarbon group, said C1-C6 alkoxy group, said C1-C6 alkylsulfanyl group, said C1-C6 alkylsulfinyl group, and said C1-C6 alkylsulfonyl group may be optionally substituted with one or more halogen atom(s)}, a halogen atom, a nitro group, and a cyano group;
Group D: a groupconsisting of a C1-C6 chain hydrocarbon group, a C3-C8 alicyclic hydrocarbon group, a C1-C6 alkoxy group {wherein said C1-C6 chain hydrocarbon group, said C3-C8 alicyclic hydrocarbon group, and said C1-C6 alkoxy group may be optionally substituted with one or more halogen atom(s)}, a halogen atom, a nitro group, and a cyano group;
Group E: a group consisting of a C1-C6 chain hydrocarbon group, a C3-C8 alicyclic hydrocarbon group, a C1-C6 alkoxy group {wherein said C1-C6 chain hydrocarbon group, said C3-C8 alicyclic hydrocarbon group, and said C1-C6 alkoxy group may be optionally substituted with one or more halogen atom(s)}, a halogen atom, a nitro group, and a cyano group]
or an N-oxide thereof, or a salt thereof, to a plant or soil for cultivating a plant.

2. The method for controlling a plant disease according to claim 1, wherein the compound represented by formula (I) or an N-oxide thereof, or a salt thereof in claim 1 is a compound or an N-oxide thereof, or a salt thereof wherein
Z represents a phenyl group or a 5-6 membered aromatic heterocyclic group {wherein said phenyl group and said 5-6 membered aromatic heterocyclic group may be optionally substituted with one or more substituent(s) selected from Group D};
R¹ and R² each represent a hydrogen atom;
R³ represents a C1-C6 chain hydrocarbon group optionally substituted with one or more halogen atom(s), a C3-C8 alicyclic hydrocarbon group, a halogen atom, a nitro group, NR⁸R⁹, OR¹⁰, SR¹¹, C(O)OR¹³, or C(O)NR⁴R⁵;
adjacent two R³ and R³ may be optionally combined with the carbon atoms to which they are attached to form a C4-C7 alicyclic hydrocarbon, a 5-7 membered nonaromatic heterocyclic ring, or a 5-7 membered aromatic heterocyclic ring {wherein said C4-C7 alicyclic hydrocarbon, said 5-7 membered nonaromatic heterocyclic ring, and said 5-7 membered aromatic heterocyclic ring may be optionally substituted with one or more halogen atom(s)}; and
R⁴, R⁵, R⁸, R⁹, R¹⁰, R¹¹, and R¹³ are identical to or different from each other, and each represent a C1-C6 chain hydrocarbon group, a C3-C8 alicyclic hydrocarbon group, a phenyl group, or a hydrogen atom.

3. The method for controlling a plant disease according to claim 1 or 2, wherein the compound represented by formula (I) or an N-oxide thereof, or a salt thereof in claim 1 or 2 is a compound or an N-oxide thereof, or a salt thereof wherein
Z represents a phenyl group or a 5-6 membered aromatic heterocyclic group {wherein said phenyl group and said 5-6 membered aromatic heterocyclic group may be optionally substituted with one or more substituent(s) selected from Group F}; and
R¹ and R² each represent a hydrogen atom;
Group F: a group consisting of a C1-C6 chain hydrocarbon group and a halogen atom.

4. A compound represented by formula (II) 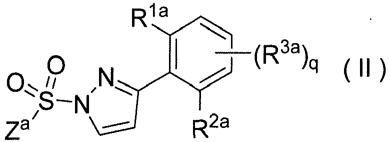 [wherein:
Z^{a} represents a 5-10 membered aromatic heterocyclic group optionally substituted with one or more substituent(s) selected from Group D^{a};
R^{1a} and R^{2a} are identical to or different from each other, and each represent a C1-C6 chain hydrocarbon group optionally substituted with one or more substituent(s) selected from Group A^{a}, a C3-C8 alicyclic hydrocarbon group, a 3-8 membered nonaromatic heterocyclic group, a C6-C10 aryl group, a 5-10 membered aromatic heterocyclic group {wherein said C3-C8 alicyclic hydrocarbon group, said 3-8 membered nonaromatic heterocyclic group, said C6-C10 aryl group, and said 5-10 membered aromatic heterocyclic group may be optionally substituted with one or more substituent(s) selected from Group C^{a}}, a hydrogen atom, a halogen atom, a nitro group, a cyano group, NR^{8a}R^{9a}, OR^{10a}, S(O)ₘR^{11a}, C(O)R^{12a}, C(O)OR^{13a}, C(O)NR^{4a}R^{5a}, or CR^{6a}=N-O-R^{7a};
R^{3a} represents a C1-C6 chain hydrocarbon group optionally substituted with one or more substituent(s) selected from Group A^{a}, a C3-C8 alicyclic hydrocarbon group, a 3-8 membered nonaromatic heterocyclic group, a C6-C10 aryl group, a 5-10 membered aromatic heterocyclic group {wherein said C3-C8 alicyclic hydrocarbon group, said 3-8 membered nonaromatic heterocyclic group, said C6-C10 aryl group, and said 5-10 membered aromatic heterocyclic group may be optionally substituted with one or more substituent(s) selected from Group C^{a}}, a halogen atom, a nitro group, a cyano group, NR^{8a}R^{9a}, OR^{10a}, S(O)ₘR^{11a}, C(O)R^{12a}, C(O)OR^{13a}, C(O)NR^{4a}R^{5a}, or CR^{6a}=N-O-R^{7a};
adjacent R^{1a} and R^{3a} may be optionally combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B^{a}}, a benzene ring, or a 5-7 membered aromatic heterocyclic ring {wherein said benzene ring and said 5-7 membered aromatic heterocyclic ring may be optionally substituted with one or more substituent(s) selected from Group E^{a}};
adjacent two R^{3a} and R^{3a} may be optionally combined with the carbon atoms to which they are attached to form a C4-C7 alicyclic hydrocarbon, a 5-7 membered nonaromatic heterocyclic ring {wherein said C4-C7 alicyclic hydrocarbon and said 5-7 membered nonaromatic heterocyclic ring may be optionally substituted with one or more substituent(s) selected from Group B^{a}}, a benzene ring, or a 5-7 membered aromatic heterocyclic ring {wherein said benzene ring and said 5-7 membered aromatic heterocyclic ring may be optionally substituted with one or more substituent(s) selected from Group E^{a}};
R^{4a}, R^{5a}, R^{6a}, R^{7a}, R^{8a}, R9a, R^{10a}, R^{11a}, R^{12a}, and R^{13a} are identical to or different from each other, and each represent a C1-C6 chain hydrocarbon group optionally substituted with one or more substituent(s) selected from Group A^{a}, a C3-C8 alicyclic hydrocarbon group, a 3-8 membered nonaromatic heterocyclic group, a C6-C10 aryl group, a 5-10 membered aromatic heterocyclic group {wherein said C3-C8 alicyclic hydrocarbon group, said 3-8 membered nonaromatic heterocyclic group, said C6-C10 aryl group, and said 5-10 membered aromatic heterocyclic group may be optionally substituted with one or more substituent(s) selected from Group C^{a}}, or a hydrogen atom;
q represents 1, 2, or 3;
when q represents 2 or 3, then two or three R^{3a} are identical to or different from each other; and
m represents 0, 1, or 2;
Group A^{a}: a group consisting of a C1-C6 alkoxy group, a C1-C6 alkylsulfanyl group, a C1-C6 alkylsulfinyl group, a C1-C6 alkylsulfonyl group {wherein said C1-C6 alkoxy group, said C1-C6 alkylsulfanyl group, said C1-C6 alkylsulfinyl group, and said C1-C6 alkylsulfonyl group may be optionally substituted with one or more halogen atom(s)}, a C3-C8 alicyclic hydrocarbon group, a 3-8 membered nonaromatic heterocyclic group, a C6-C10 aryl group, a 5-10 membered aromatic heterocyclic group {wherein said C3-C8 alicyclic hydrocarbon group, said 3-8 membered nonaromatic heterocyclic group, said C6-C10 aryl group, and said 5-10 membered aromatic heterocyclic group may be optionally substituted with one or more substituent(s) selected from Group C^{a}}, a halogen atom, and a cyano group;
Group B^{a}: a group consisting of an oxo group, a thioxo group, a C1-C6 chain hydrocarbon group, a C3-C8 alicyclic hydrocarbon group, a C1-C6 alkoxy group {wherein said C1-C6 chain hydrocarbon group, said C3-C8 alicyclic hydrocarbon group, and said C1-C6 alkoxy group may be optionally substituted with one or more halogen atom(s)}, a halogen atom, a nitro group, and a cyano group;
Group C^{a}: a group consisting of a C1-C6 chain hydrocarbon group, a C3-C8 alicyclic hydrocarbon group, a C1-C6 alkoxy group, a C1-C6 alkylsulfanyl group, a C1-C6 alkylsulfinyl group, a C1-C6 alkylsulfonyl group {wherein said C1-C6 chain hydrocarbon group, said C3-C8 alicyclic hydrocarbon group, said C1-C6 alkoxy group, said C1-C6 alkylsulfanyl group, said C1-C6 alkylsulfinyl group, and said C1-C6 alkylsulfonyl group may be optionally substituted with one or more halogen atom(s)}, a halogen atom, a nitro group, and a cyano group;
Group D^{a}: a group consisting of a C1-C6 chain hydrocarbon group, a C3-C8 alicyclic hydrocarbon group, a C1-C6 alkoxy group {wherein said C1-C6 chain hydrocarbon group, said C3-C8 alicyclic hydrocarbon group, and said C1-C6 alkoxy group may be optionally substituted with one or more halogen atom(s)}, a halogen atom, a nitro group, and a cyano group;
Group E^{a}: a group consisting of a C1-C6 chain hydrocarbon group, a C3-C8 alicyclic hydrocarbon group, a C1-C6 alkoxy group {wherein said C1-C6 chain hydrocarbon group, said C3-C8 alicyclic hydrocarbon group, and said C1-C6 alkoxy group may be optionally substituted with one or more halogen atom(s)}, a halogen atom, a nitro group, and a cyano group]
(provided that 4-{[3-(4-fluorophenyl)-1H-pyrazol-1-yl]sulfony1}-1,3-dimethyl-1H-pyrazole and 3-(2,3-dihydrobenzofuran-5-yl)-1-(thiophen-2-ylsulfonyl)-1H-pyrazole are excluded)
or an N-oxide thereof, or a salt thereof.

5. The compound or an N-oxide thereof, or a salt thereof according to claim 4, wherein
Z^{a} represents a 5-6 membered aromatic heterocyclic group optionally substituted with one or more substituent(s) selected from Group F^{a}; and
R^{1a} and R^{2a} each represent a hydrogen atom;
Group F^{a}: a group consisting of a C1-C6 chain hydrocarbon group and a halogen atom.

6. The compound or an N-oxide thereof, or a salt thereof according to claim 4 or 5, wherein
R^{3a} represents a C1-C6 chain hydrocarbon group optionally substituted with one or more halogen atom(s), a C3-C8 alicyclic hydrocarbon group, a halogen atom, a nitro group, NR^{8a}R^{9a}, OR^{10a}, or SR^{11a};
adjacent two R^{3a} and R^{3a} may be optionally combined with the carbon atoms to which they are attached to form a C4-C7 alicyclic hydrocarbon, a 5-7 membered nonaromatic heterocyclic ring, or a 5-7 membered aromatic heterocyclic ring {wherein said C4-C7 alicyclic hydrocarbon, said 5-7 membered nonaromatic heterocyclic ring, and said 5-7 membered aromatic heterocyclic ring may be optionally substituted with one or more halogen atom(s)}; and
R^{8a}, R^{9a}, R^{10a}, and R^{11a} are identical to or different from each other, and each represent a C1-C6 chain hydrocarbon group.

7. A composition comprising the compound or an N-oxide thereof, or a salt thereof according to any one of claims 4 to 6, and an inert carrier.

8. A composition comprising one or more ingredient(s) selected from the group consisting of Group (a), Group (b), Group (c), and Group (d), and the compound or an N-oxide thereof, or a salt thereof according to any one of claims 4 to 6:
Group (a): a group consisting of insecticidal active ingredients, miticidal active ingredients, and nematicidal active ingredients;
Group (b): fungicidal active ingredients;
Group (c): plant growth regulatory ingredients;
Group (d): repellent ingredients.

9. A method for controlling a plant disease which comprises applying an effective amount of the compound or an N-oxide thereof, or a salt thereof according to any one of claims 4 to 6, or an effective amount of the composition according to claim 8, to a plant or soil for cultivating a plant.

10. Use of the compound or an N-oxide thereof, or a salt thereof according to any one of claims 4 to 6, or the composition according to claim 8, for controlling a plant disease.

11. A seed or a vegetative reproductive organ holding an effective amount of the compound or an N-oxide thereof, or a salt thereof according to any one of claims 4 to 6, or an effective amount of the composition according to claim 8.

12. A compound represented by formula (III) 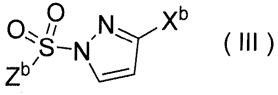 [wherein:
Z^{b} represents a 5-10 membered aromatic heterocyclic group optionally substituted with one or more substituent(s) selected from Group D^{b}; and
X^{b} represents a chlorine atom, a bromine atom, an iodine atom, B(OH)₂, or a 4,4,5,5-tetramethyl-1,3,2-dioxaborolan-2-yl group;
Group D^{b}: a group consisting of a C1-C6 chain hydrocarbon group, a C3-C8 alicyclic hydrocarbon group, a C1-C6 alkoxy group {wherein said C1-C6 chain hydrocarbon group, said C3-C8 alicyclic hydrocarbon group, and said C1-C6 alkoxy group may be optionally substituted with one or more halogen atom(s)}, a halogen atom, a nitro group, and a cyano group].
